# EUROPEAN PATENT SPECIFICATION

(11) **EP 3 212 776 B1**
(45) Date of publication and mention of the grant of the patent: **22.04.2020**
(21) Application number: 15791843.4
(22) Date of filing: 27.10.2015
(51) Int. Cl.: C12N 9/42

(54) **COMPOSITIONS AND METHODS RELATED TO BETA-GLUCOSIDASE**
ZUSAMMENSETZUNGEN UND VERFAHREN IN ZUSAMMENHANG MIT BETA-GLUCOSIDASE
COMPOSITIONS ET PROCÉDÉS RELATIFS À UNE BÊTA-GLUCOSIDASE

(30) Priority: 27.10.2014 US 201462069120 P
(43) Date of publication of application: 06.09.2017
(73) Proprietor: Danisco US Inc., Palo Alto, California 94304 (US)
(72) Inventor: BOWER, Benjamin S., Palo Alto, California 94304 (US); CHAN, Jimmy, Palo Alto, California 94304 (US); FUJDALA, Meredith K., Palo Alto, California 94304 (US); KAPER, Thijs, Palo Alto, California 94304 (US); LANTZ, Suzanne E., Palo Alto, California 94304 (US); NOSÉ CROTTY, Kirstin Y., Palo Alto, California 94304 (US); TOPPOZADA, Amr R., Palo Alto, California 94304 (US)
(74) Representative: Mewburn Ellis LLP
(86) International application number: PCT/US2015/057487
(87) International publication number: WO 2016/069541

(56) References cited:
- WO-A1-2011/069106
- WO-A1-2013/177714
- DATABASE UniProt [Online] 11 January 2011 (2011-01-11), "SubName: Full=Glycosyl hydrolase family 3 N terminal domain-containing protein {ECO:0000313|EMBL:EFQ32803.1};", XP002752198, retrieved from EBI accession no. UNIPROT:E3QPM6 Database accession no. E3QPM6
- DATABASE EMBL [Online] 20 March 2008 (2008-03-20), "fcg3x.226150j06 C. graminicola genomic sequence Glomerella graminicola genomic, genomic survey sequence.", XP002752199, retrieved from EBI accession no. EM_GSS:ET519535 Database accession no. ET519535
- DATABASE UniProt [Online] 6 March 2013 (2013-03-06), "RecName: Full=Beta-glucosidase {ECO:0000256|RuleBase:RU361161}; EC=3.2.1.21 {ECO:0000256|RuleBase:RU361161};", retrieved from EBI accession no. UNIPROT:L2G5M9 Database accession no. L2G5M9
- DATABASE UniProt [Online] 26 June 2013 (2013-06-26), "RecName: Full=Beta-glucosidase {ECO:0000256|RuleBase:RU361161}; EC=3.2.1.21 {ECO:0000256|RuleBase:RU361161};", retrieved from EBI accession no. UNIPROT:N4VJC4 Database accession no. N4VJC4

### SEQUENCE LISTING

<110> Danisco USA Inc.
   Bower, Benjamin S.
   Chan, Jimmy
   Fujdala, Meredith K.
   Kaper, Thijs
   Lantz, Suzanne E.
   Nose Krotty, Kristin Y
   Toppozada, Amir R.
<120> Compositions and Methods Related to Beta Glucosidase
<130> NB40751
<150> US 62/069,120
   <151> 2014-10-27
<160> 45
<170> PatentIn version 3.5
<210> 1
   <211> 3239
   <212> DNA
   <213> Glomerella graminicola
<400> 1
   atgaggtcac agactctggc tgttgccctc ttggcggcag ctgaccaggt tgcagctgct 60
   gtaaccacag aacgacagct acacaaggta cgttactcgg tctgtctgtt gttctctgtg 120
   gcgccgtcgt tggcgactcg ccagccttgc ccttcttgga aatccaagac atccccattc 180
   ccttcgggcc tgtgtgtggc gagcaggccc gccgcggtgt tatatcccga ttttgacgtc 240
   ttccctctct atttcgtatc gcattggcgt ctcgtctcgt ctctctcctt tagcaacaca 300
   gagaacccgg ttccgcagtc atgcgtttcc tttcactcgc aggctcggcc gtcggcctgt 360
   cacaactctc gagccgctca gatgacttac aatgtttaca gcgcgacctc gcatactcgc 420
   cgcccgtcta tccgtcacca tggatggatc ccaatgccga cggctggact gatgcttacg 480
   ccaaggccaa ggactttgtc tcccagctca cgcttctcga gaaggttaat ttgaccaccg 540
   gagtcgggtg agtgaattcg catcaactac gatgggaaca catgctgata ggaatataca 600
   gttggcaagg agacctgtgc gtcggcaacg ttggttccgt cccccgtttg ggactccgcg 660
   gcctttgcat gcaggacggc cctgtcggca tccgcttctc cgactacaac tcggtcttcc 720
   cctccggcca gaccgccgcc gcgacttggg accgagagtt gatctaccgc agagccgaag 780
   ctatcgggtt cgagcaccgc gccaagggcg tcgacgttgt cctggccccg gttgccggac 840
   ctattggtcg tgcaccagcc ggcggccgca actgggaagg cttttcgtcc gatccttacc 900
   tgactggcgt tgccatggcc gagtcagtca agggaatcca gcagcacgcc atcgcctgcg 960
   ccaagcattt catcggcaac gaacaggagc acttcagaca agctccagag gcaattggct 1020
   ataactacac catagacgag tccatcagct cgaatattga tgacaaaact ctccacgaat 1080
   tatatttgtg gccattccag gacgcagtgg ctgctggggt aggttccttc atgtgctcat 1140
   acaatcaggt gaacaactcg tatggctgcc agaactccaa gctcatgaac ggcatcttga 1200
   aagacgagct tggtttccag ggattcatca tgtctgattg gtcagtactt ccttccattc 1260
   tctttagccc ctcctacaag cacttcttca acctccgttc catatctact cagctccatc 1320
   ccctctttca tactctcttt ccttccatca gatgccgact tcagtagcta actttcttac 1380
   agggcagcgc agcatgtacg tatccccggt tttgttccat ttcatatgat gatgcccacc 1440
   cgccttcctt tcccatcaat gacttatgac tgacaatcga acaggctgga gttgctactg 1500
   cggttgccgg tctcgatatg gccatgcctg gtgacacagc gttcaactct ggcatgacgt 1560
   tctggggaac caatctaacc gtggccgtcc tgaacggcac gctccccgag taccggctcg 1620
   atgacatggc gatgcgcatc atggccgcat tctttaaggt cggcttcgaa ctgaatgagg 1680
   tacccgagat caacttttcc tcgtggacga cggacaccgt tggtccgcta cagtactacg 1740
   ccaaggagaa cgttcaagtc atcaaccagc atgtcgacgt tcgaagaggt caggaacacg 1800
   gtaaactcat tcgtgagatc gccgccaagg ccactgtgct gctgaagaat gagggcgctc 1860
   tcccgctgaa gaagcccaag ttcctggctg ttatcggaga ggatgccggc cccaacctca 1920
   gtgggcccaa cgggtgctcc gaccatggct gtaacgaagg aacgctcggc gctggctggg 1980
   gatccggaac ctcgaactac ccctacctca tcacaccgga ccaggcactg caagctcggg 2040
   ctgttgcgga gggttctcgg tatgagagca ttctcagcaa ctatgacttc gccgccacca 2100
   cggccctggt tacgcaacca gacgccacgg ccatcgtctt cgtcaatgcg gatagcggtg 2160
   aaggctacat cgatgtgggt gggaacgaag gcgaccgtca gaacttgacg ctttggaatg 2220
   ggggagacga gctggtcaag aacgtggctg caggcaacaa caacactatt gtcgtgatcc 2280
   actccgtcgg ccctgtgttg ctggctgaca tgaagaacaa ccccaatatc acggcgattg 2340
   tctgggccgg tcttccgggt caggagtctg gtaattcgat cacggacgtg ctctacggag 2400
   acgttaaccc cggaggcaaa tccccgttca cttggggtcc cacgcgcgag agttatggca 2460
   ccgatgttct gtacgagccc aacaatggtg agggtgctcc tcaagacgac tttagcgagg 2520
   gtgtcttcat cgactaccgc tacttcgacc gggcgacctc gggttccaac gagacctcca 2580
   ctggcgcagc ccccgtctac ccattcggat ttggtctctc gtacacgacg tttgaatact 2640
   ccaacttggt cgtaaccccc aaggaggcag gcgagtacac gcccaccact ggcgtgacgg 2700
   agaaggcgcc gacctttggc aactacagca ccgacccggc ggcctacgtt ttcccgagcg 2760
   gagagttccg ttacatctac aacttcatct acccctacct caacactacc gacattagca 2820
   agtcggccaa cgaccccgcg tacggacaga cggcggacga gtttctgcct cccaaggctc 2880
   tggagagcgg tccccagccc aagcacccag catcgggcgc ccctggcggc aaccctcaac 2940
   tttgggacgt cctgtacact gtgactgcca cgatcactaa caagggcgac gttgccggcg    3000
   acgaggttgc ccagttgtac gtctcgctcg gcggtccgaa tgatccggtc aaggtgctgc    3060
   gtgggttcga ccgcattggt atcgcgcctg gcgagtcggc cactttcacg gcggacatca    3120
   ccagacgcga cctcagcaac tgggacacgg tgagccaaaa ctgggtcatc agcaagtacc    3180
   cgaagaaggt gtgggtggga ggctcgtcga gggagttgcc tctgagcgcg tcgctctaa     3239
<210> 2
   <211> 876
   <212> PRT
   <213> Glomerella graminicola
<400> 2
   Met Arg Ser Gln Thr Leu Ala Val Ala Leu Leu Ala Ala Ala Asp Gln
   1               5                   10                  15
   Val Ala Ala Ala Val Thr Thr Glu Arg Gln Leu His Lys Arg Asp Leu
               20                  25                  30
   Ala Tyr Ser Pro Pro Val Tyr Pro Ser Pro Trp Met Asp Pro Asn Ala
           35                  40                  45
   Asp Gly Trp Thr Asp Ala Tyr Ala Lys Ala Lys Asp Phe Val Ser Gln
       50                  55                  60
   Leu Thr Leu Leu Glu Lys Val Asn Leu Thr Thr Gly Val Gly Trp Gln
   65                  70                  75                  80
   Gly Asp Leu Cys Val Gly Asn Val Gly Ser Val Pro Arg Leu Gly Leu
                   85                  90                  95
   Arg Gly Leu Cys Met Gln Asp Gly Pro Val Gly Ile Arg Phe Ser Asp
               100                 105                 110
   Tyr Asn Ser Val Phe Pro Ser Gly Gln Thr Ala Ala Ala Thr Trp Asp
           115                 120                 125
   Arg Glu Leu Ile Tyr Arg Arg Ala Glu Ala Ile Gly Phe Glu His Arg
       130                 135                 140
   Ala Lys Gly Val Asp Val Val Leu Ala Pro Val Ala Gly Pro Ile Gly
   145                 150                 155                 160
   Arg Ala Pro Ala Gly Gly Arg Asn Trp Glu Gly Phe Ser Ser Asp Pro
                   165                 170                 175
   Tyr Leu Thr Gly Val Ala Met Ala Glu Ser Val Lys Gly Ile Gln Gln
   180 185 190
   His Ala Ile Ala Cys Ala Lys His Phe Ile Gly Asn Glu Gln Glu His
   195 200 205
   Phe Arg Gln Ala Pro Glu Ala Ile Gly Tyr Asn Tyr Thr Ile Asp Glu
   210 215 220
   Ser Ile Ser Ser Asn Ile Asp Asp Lys Thr Leu His Glu Leu Tyr Leu
   225 230 235 240
   Trp Pro Phe Gln Asp Ala Val Ala Ala Gly Val Gly Ser Phe Met Cys
   245 250 255
   Ser Tyr Asn Gln Val Asn Asn Ser Tyr Gly Cys Gln Asn Ser Lys Leu
   260 265 270
   Met Asn Gly Ile Leu Lys Asp Glu Leu Gly Phe Gln Gly Phe Ile Met
   275 280 285
   Ser Asp Trp Ser Ala Gly Val Ala Thr Ala Val Ala Gly Leu Asp Met
   290 295 300
   Ala Met Pro Gly Asp Thr Ala Phe Asn Ser Gly Met Thr Phe Trp Gly
   305 310 315 320
   Thr Asn Leu Thr Val Ala Val Leu Asn Gly Thr Leu Pro Glu Tyr Arg
   325 330 335
   Leu Asp Asp Met Ala Met Arg Ile Met Ala Ala Phe Phe Lys Val Gly
   340 345 350
   Phe Glu Leu Asn Glu Val Pro Glu Ile Asn Phe Ser Ser Trp Thr Thr
   355 360 365
   Asp Thr Val Gly Pro Leu Gln Tyr Tyr Ala Lys Glu Asn Val Gln Val
   370 375 380
   Ile Asn Gln His Val Asp Val Arg Arg Gly Gln Glu His Gly Lys Leu
   385 390 395 400
   Ile Arg Glu Ile Ala Ala Lys Ala Thr Val Leu Leu Lys Asn Glu Gly
   405 410 415
   Ala Leu Pro Leu Lys Lys Pro Lys Phe Leu Ala Val Ile Gly Glu Asp
   420 425 430
   Ala Gly Pro Asn Leu Ser Gly Pro Asn Gly Cys Ser Asp His Gly Cys
   435 440 445
   Asn Glu Gly Thr Leu Gly Ala Gly Trp Gly Ser Gly Thr Ser Asn Tyr
   450 455 460
   Pro Tyr Leu Ile Thr Pro Asp Gln Ala Leu Gln Ala Arg Ala Val Ala
   465 470 475 480
   Glu Gly Ser Arg Tyr Glu Ser Ile Leu Ser Asn Tyr Asp Phe Ala Ala
   485 490 495
   Thr Thr Ala Leu Val Thr Gln Pro Asp Ala Thr Ala Ile Val Phe Val
   500 505 510
   Asn Ala Asp Ser Gly Glu Gly Tyr Ile Asp Val Gly Gly Asn Glu Gly
   515 520 525
   Asp Arg Gln Asn Leu Thr Leu Trp Asn Gly Gly Asp Glu Leu Val Lys
   530 535 540
   Asn Val Ala Ala Gly Asn Asn Asn Thr Ile Val Val Ile His Ser Val
   545 550 555 560
   Gly Pro Val Leu Leu Ala Asp Met Lys Asn Asn Pro Asn Ile Thr Ala
   565 570 575
   Ile Val Trp Ala Gly Leu Pro Gly Gln Glu Ser Gly Asn Ser Ile Thr
   580 585 590
   Asp Val Leu Tyr Gly Asp Val Asn Pro Gly Gly Lys Ser Pro Phe Thr
   595 600 605
   Trp Gly Pro Thr Arg Glu Ser Tyr Gly Thr Asp Val Leu Tyr Glu Pro
   610 615 620
   Asn Asn Gly Glu Gly Ala Pro Gln Asp Asp Phe Ser Glu Gly Val Phe
   625 630 635 640
   Ile Asp Tyr Arg Tyr Phe Asp Arg Ala Thr Ser Gly Ser Asn Glu Thr
   645 650 655
   Ser Thr Gly Ala Ala Pro Val Tyr Pro Phe Gly Phe Gly Leu Ser Tyr
   660 665 670
   Thr Thr Phe Glu Tyr Ser Asn Leu Val Val Thr Pro Lys Glu Ala Gly
   675 680 685
   Glu Tyr Thr Pro Thr Thr Gly Val Thr Glu Lys Ala Pro Thr Phe Gly
       690                 695                 700
   Asn Tyr Ser Thr Asp Pro Ala Ala Tyr Val Phe Pro Ser Gly Glu Phe
   705                 710                 715                 720
   Arg Tyr Ile Tyr Asn Phe Ile Tyr Pro Tyr Leu Asn Thr Thr Asp Ile
                   725                 730                 735
   Ser Lys Ser Ala Asn Asp Pro Ala Tyr Gly Gln Thr Ala Asp Glu Phe
               740                 745                 750
   Leu Pro Pro Lys Ala Leu Glu Ser Gly Pro Gln Pro Lys His Pro Ala
           755                 760                 765
   Ser Gly Ala Pro Gly Gly Asn Pro Gln Leu Trp Asp Val Leu Tyr Thr
       770                 775                 780
   Val Thr Ala Thr Ile Thr Asn Lys Gly Asp Val Ala Gly Asp Glu Val
   785                 790                 795                 800
   Ala Gln Leu Tyr Val Ser Leu Gly Gly Pro Asn Asp Pro Val Lys Val
                   805                 810                 815
   Leu Arg Gly Phe Asp Arg Ile Gly Ile Ala Pro Gly Glu Ser Ala Thr
               820                 825                 830
   Phe Thr Ala Asp Ile Thr Arg Arg Asp Leu Ser Asn Trp Asp Thr Val
           835                 840                 845
   Ser Gln Asn Trp Val Ile Ser Lys Tyr Pro Lys Lys Val Trp Val Gly
       850                 855                 860
   Gly Ser Ser Arg Glu Leu Pro Leu Ser Ala Ser Leu
   865                 870                 875
<210> 3
   <211> 857
   <212> PRT
   <213> Glomerella graminicola
<400> 3
   Ala Val Thr Thr Glu Arg Gln Leu His Lys Arg Asp Leu Ala Tyr Ser
   1               5                   10                  15
   Pro Pro Val Tyr Pro Ser Pro Trp Met Asp Pro Asn Ala Asp Gly Trp
               20                  25                  30
   Thr Asp Ala Tyr Ala Lys Ala Lys Asp Phe Val Ser Gln Leu Thr Leu
           35                  40                  45
   Leu Glu Lys Val Asn Leu Thr Thr Gly Val Gly Trp Gln Gly Asp Leu
       50                  55                  60
   Cys Val Gly Asn Val Gly Ser Val Pro Arg Leu Gly Leu Arg Gly Leu
   65                  70                  75                  80
   Cys Met Gln Asp Gly Pro Val Gly Ile Arg Phe Ser Asp Tyr Asn Ser
                   85                  90                  95
   Val Phe Pro Ser Gly Gln Thr Ala Ala Ala Thr Trp Asp Arg Glu Leu
               100                 105                 110
   Ile Tyr Arg Arg Ala Glu Ala Ile Gly Phe Glu His Arg Ala Lys Gly
           115                 120                 125
   Val Asp Val Val Leu Ala Pro Val Ala Gly Pro Ile Gly Arg Ala Pro
       130                 135                 140
   Ala Gly Gly Arg Asn Trp Glu Gly Phe Ser Ser Asp Pro Tyr Leu Thr
   145                 150                 155                 160
   Gly Val Ala Met Ala Glu Ser Val Lys Gly Ile Gln Gln His Ala Ile
                   165                 170                 175
   Ala Cys Ala Lys His Phe Ile Gly Asn Glu Gln Glu His Phe Arg Gln
               180                 185                 190
   Ala Pro Glu Ala Ile Gly Tyr Asn Tyr Thr Ile Asp Glu Ser Ile Ser
           195                 200                 205
   Ser Asn Ile Asp Asp Lys Thr Leu His Glu Leu Tyr Leu Trp Pro Phe
       210                 215                 220
   Gln Asp Ala Val Ala Ala Gly Val Gly Ser Phe Met Cys Ser Tyr Asn
   225                 230                 235                 240
   Gln Val Asn Asn Ser Tyr Gly Cys Gln Asn Ser Lys Leu Met Asn Gly
                   245                 250                 255
   Ile Leu Lys Asp Glu Leu Gly Phe Gln Gly Phe Ile Met Ser Asp Trp
               260                 265                 270
   Ser Ala Gly Val Ala Thr Ala Val Ala Gly Leu Asp Met Ala Met Pro
           275                 280                 285
   Gly Asp Thr Ala Phe Asn Ser Gly Met Thr Phe Trp Gly Thr Asn Leu
       290                 295                 300
   Thr Val Ala Val Leu Asn Gly Thr Leu Pro Glu Tyr Arg Leu Asp Asp
   305                 310                 315                 320
   Met Ala Met Arg Ile Met Ala Ala Phe Phe Lys Val Gly Phe Glu Leu
                   325                 330                 335
   Asn Glu Val Pro Glu Ile Asn Phe Ser Ser Trp Thr Thr Asp Thr Val
               340                 345                 350
   Gly Pro Leu Gln Tyr Tyr Ala Lys Glu Asn Val Gln Val Ile Asn Gln
           355                 360                 365
   His Val Asp Val Arg Arg Gly Gln Glu His Gly Lys Leu Ile Arg Glu
       370                 375                 380
   Ile Ala Ala Lys Ala Thr Val Leu Leu Lys Asn Glu Gly Ala Leu Pro
   385                 390                 395                 400
   Leu Lys Lys Pro Lys Phe Leu Ala Val Ile Gly Glu Asp Ala Gly Pro
                   405                 410                 415
   Asn Leu Ser Gly Pro Asn Gly Cys Ser Asp His Gly Cys Asn Glu Gly
               420                 425                 430
   Thr Leu Gly Ala Gly Trp Gly Ser Gly Thr Ser Asn Tyr Pro Tyr Leu
           435                 440                 445
   Ile Thr Pro Asp Gln Ala Leu Gln Ala Arg Ala Val Ala Glu Gly Ser
       450                 455                 460
   Arg Tyr Glu Ser Ile Leu Ser Asn Tyr Asp Phe Ala Ala Thr Thr Ala
   465                 470                 475                 480
   Leu Val Thr Gln Pro Asp Ala Thr Ala Ile Val Phe Val Asn Ala Asp
                   485                 490                 495
   Ser Gly Glu Gly Tyr Ile Asp Val Gly Gly Asn Glu Gly Asp Arg Gln
               500                 505                 510
   Asn Leu Thr Leu Trp Asn Gly Gly Asp Glu Leu Val Lys Asn Val Ala
           515                 520                 525
   Ala Gly Asn Asn Asn Thr Ile Val Val Ile His Ser Val Gly Pro Val
   530 535 540
   Leu Leu Ala Asp Met Lys Asn Asn Pro Asn Ile Thr Ala Ile Val Trp
   545 550 555 560
   Ala Gly Leu Pro Gly Gln Glu Ser Gly Asn Ser Ile Thr Asp Val Leu
   565 570 575
   Tyr Gly Asp Val Asn Pro Gly Gly Lys Ser Pro Phe Thr Trp Gly Pro
   580 585 590
   Thr Arg Glu Ser Tyr Gly Thr Asp Val Leu Tyr Glu Pro Asn Asn Gly
   595 600 605
   Glu Gly Ala Pro Gln Asp Asp Phe Ser Glu Gly Val Phe Ile Asp Tyr
   610 615 620
   Arg Tyr Phe Asp Arg Ala Thr Ser Gly Ser Asn Glu Thr Ser Thr Gly
   625 630 635 640
   Ala Ala Pro Val Tyr Pro Phe Gly Phe Gly Leu Ser Tyr Thr Thr Phe
   645 650 655
   Glu Tyr Ser Asn Leu Val Val Thr Pro Lys Glu Ala Gly Glu Tyr Thr
   660 665 670
   Pro Thr Thr Gly Val Thr Glu Lys Ala Pro Thr Phe Gly Asn Tyr Ser
   675 680 685
   Thr Asp Pro Ala Ala Tyr Val Phe Pro Ser Gly Glu Phe Arg Tyr Ile
   690 695 700
   Tyr Asn Phe Ile Tyr Pro Tyr Leu Asn Thr Thr Asp Ile Ser Lys Ser
   705 710 715 720
   Ala Asn Asp Pro Ala Tyr Gly Gln Thr Ala Asp Glu Phe Leu Pro Pro
   725 730 735
   Lys Ala Leu Glu Ser Gly Pro Gln Pro Lys His Pro Ala Ser Gly Ala
   740 745 750
   Pro Gly Gly Asn Pro Gln Leu Trp Asp Val Leu Tyr Thr Val Thr Ala
   755 760 765
   Thr Ile Thr Asn Lys Gly Asp Val Ala Gly Asp Glu Val Ala Gln Leu
   770 775 780
   Tyr Val Ser Leu Gly Gly Pro Asn Asp Pro Val Lys Val Leu Arg Gly
   785 790 795 800
   Phe Asp Arg Ile Gly Ile Ala Pro Gly Glu Ser Ala Thr Phe Thr Ala
   805 810 815
   Asp Ile Thr Arg Arg Asp Leu Ser Asn Trp Asp Thr Val Ser Gln Asn
   820 825 830
   Trp Val Ile Ser Lys Tyr Pro Lys Lys Val Trp Val Gly Gly Ser Ser
   835 840 845
   Arg Glu Leu Pro Leu Ser Ala Ser Leu
   850 855
<210> 4
   <211> 744
   <212> PRT
   <213> Trichoderma reesei
<400> 4
   Met Arg Tyr Arg Thr Ala Ala Ala Leu Ala Leu Ala Thr Gly Pro Phe
   1 5 10 15
   Ala Arg Ala Asp Ser His Ser Thr Ser Gly Ala Ser Ala Glu Ala Val
   20 25 30
   Val Pro Pro Ala Gly Thr Pro Trp Gly Thr Ala Tyr Asp Lys Ala Lys
   35 40 45
   Ala Ala Leu Ala Lys Leu Asn Leu Gln Asp Lys Val Gly Ile Val Ser
   50 55 60
   Gly Val Gly Trp Asn Gly Gly Pro Cys Val Gly Asn Thr Ser Pro Ala
   65 70 75 80
   Ser Lys Ile Ser Tyr Pro Ser Leu Cys Leu Gln Asp Gly Pro Leu Gly
   85 90 95
   Val Arg Tyr Ser Thr Gly Ser Thr Ala Phe Thr Pro Gly Val Gln Ala
   100 105 110
   Ala Ser Thr Trp Asp Val Asn Leu Ile Arg Glu Arg Gly Gln Phe Ile
   115 120 125
   Gly Glu Glu Val Lys Ala Ser Gly Ile His Val Ile Leu Gly Pro Val
   130 135 140
   Ala Gly Pro Leu Gly Lys Thr Pro Gln Gly Gly Arg Asn Trp Glu Gly
   145                 150                 155                 160
   Phe Gly Val Asp Pro Tyr Leu Thr Gly Ile Ala Met Gly Gln Thr Ile
                   165                 170                 175
   Asn Gly Ile Gln Ser Val Gly Val Gln Ala Thr Ala Lys His Tyr Ile
               180                 185                 190
   Leu Asn Glu Gln Glu Leu Asn Arg Glu Thr Ile Ser Ser Asn Pro Asp
           195                 200                 205
   Asp Arg Thr Leu His Glu Leu Tyr Thr Trp Pro Phe Ala Asp Ala Val
       210                 215                 220
   Gln Ala Asn Val Ala Ser Val Met Cys Ser Tyr Asn Lys Val Asn Thr
   225                 230                 235                 240
   Thr Trp Ala Cys Glu Asp Gln Tyr Thr Leu Gln Thr Val Leu Lys Asp
                   245                 250                 255
   Gln Leu Gly Phe Pro Gly Tyr Val Met Thr Asp Trp Asn Ala Gln His
               260                 265                 270
   Thr Thr Val Gln Ser Ala Asn Ser Gly Leu Asp Met Ser Met Pro Gly
           275                 280                 285
   Thr Asp Phe Asn Gly Asn Asn Arg Leu Trp Gly Pro Ala Leu Thr Asn
       290                 295                 300
   Ala Val Asn Ser Asn Gln Val Pro Thr Ser Arg Val Asp Asp Met Val
   305                 310                 315                 320
   Thr Arg Ile Leu Ala Ala Trp Tyr Leu Thr Gly Gln Asp Gln Ala Gly
                   325                 330                 335
   Tyr Pro Ser Phe Asn Ile Ser Arg Asn Val Gln Gly Asn His Lys Thr
               340                 345                 350
   Asn Val Arg Ala Ile Ala Arg Asp Gly Ile Val Leu Leu Lys Asn Asp
           355                 360                 365
   Ala Asn Ile Leu Pro Leu Lys Lys Pro Ala Ser Ile Ala Val Val Gly
       370                 375                 380
   Ser Ala Ala Ile Ile Gly Asn His Ala Arg Asn Ser Pro Ser Cys Asn
   385                 390                 395                 400
   Asp Lys Gly Cys Asp Asp Gly Ala Leu Gly Met Gly Trp Gly Ser Gly
                   405                 410                 415
   Ala Val Asn Tyr Pro Tyr Phe Val Ala Pro Tyr Asp Ala Ile Asn Thr
               420                 425                 430
   Arg Ala Ser Ser Gln Gly Thr Gln Val Thr Leu Ser Asn Thr Asp Asn
           435                 440                 445
   Thr Ser Ser Gly Ala Ser Ala Ala Arg Gly Lys Asp Val Ala Ile Val
       450                 455                 460
   Phe Ile Thr Ala Asp Ser Gly Glu Gly Tyr Ile Thr Val Glu Gly Asn
   465                 470                 475                 480
   Ala Gly Asp Arg Asn Asn Leu Asp Pro Trp His Asn Gly Asn Ala Leu
                   485                 490                 495
   Val Gln Ala Val Ala Gly Ala Asn Ser Asn Val Ile Val Val Val His
               500                 505                 510
   Ser Val Gly Ala Ile Ile Leu Glu Gln Ile Leu Ala Leu Pro Gln Val
           515                 520                 525
   Lys Ala Val Val Trp Ala Gly Leu Pro Ser Gln Glu Ser Gly Asn Ala
       530                 535                 540
   Leu Val Asp Val Leu Trp Gly Asp Val Ser Pro Ser Gly Lys Leu Val
   545                 550                 555                 560
   Tyr Thr Ile Ala Lys Ser Pro Asn Asp Tyr Asn Thr Arg Ile Val Ser
                   565                 570                 575
   Gly Gly Ser Asp Ser Phe Ser Glu Gly Leu Phe Ile Asp Tyr Lys His
               580                 585                 590
   Phe Asp Asp Ala Asn Ile Thr Pro Arg Tyr Glu Phe Gly Tyr Gly Leu
           595                 600                 605
   Ser Tyr Thr Lys Phe Asn Tyr Ser Arg Leu Ser Val Leu Ser Thr Ala
       610                 615                 620
   Lys Ser Gly Pro Ala Thr Gly Ala Val Val Pro Gly Gly Pro Ser Asp
   625                 630                 635                 640
   Leu Phe Gln Asn Val Ala Thr Val Thr Val Asp Ile Ala Asn Ser Gly
                   645                 650                 655
   Gln Val Thr Gly Ala Glu Val Ala Gln Leu Tyr Ile Thr Tyr Pro Ser
               660                 665                 670
   Ser Ala Pro Arg Thr Pro Pro Lys Gln Leu Arg Gly Phe Ala Lys Leu
           675                 680                 685
   Asn Leu Thr Pro Gly Gln Ser Gly Thr Ala Thr Phe Asn Ile Arg Arg
       690                 695                 700
   Arg Asp Leu Ser Tyr Trp Asp Thr Ala Ser Gln Lys Trp Val Val Pro
   705                 710                 715                 720
   Ser Gly Ser Phe Gly Ile Ser Val Gly Ala Ser Ser Arg Asp Ile Arg
                   725                 730                 735
   Leu Thr Ser Thr Leu Ser Val Ala
               740
<210> 5
   <211> 28
   <212> DNA
   <213> artificial sequence
<220>
   <223> primer
<400> 5
   caccatgaga tatagaacaf ctgccgct 28
<210> 6
   <211> 40
   <212> DNA
   <213> artificial sequence
<220>
   <223> primer
<400> 6
   cgaccgccct gcggagtctt gcccagtggt cccgcgacag 40
<210> 7
   <211> 40
   <212> DNA
   <213> artificial sequence
<220>
   <223> primer
<400> 7
   ctgtcgcggg accactgggc aagactccgc agggcggtcg 40
<210> 8
   <211> 20
   <212> DNA
   <213> artificial sequence
<220>
   <223> primer
<400> 8
   cctacgctac cgacagagtg 20
<210> 9
   <211> 20
   <212> DNA
   <213> artificial sequence
<220>
   <223> primer
<400> 9
   gtctagactg gaaacgcaac 20
<210> 10
   <211> 21
   <212> DNA
   <213> artificial sequence
<220>
   <223> primer
<400> 10
   gagttgtgaa gtcggtaatc c 21
<210> 11
   <211> 19
   <212> PRT
   <213> Trichoderma reesei
<400> 11
   Met Arg Tyr Arg Thr Ala Ala Ala Leu Ala Leu Ala Thr Gly Pro Phe
   1               5                   10                  15
   Ala Arg Ala
<210> 12
   <211> 32
   <212> PRT
   <213> Trichoderma reesei
<400> 12
   Met Val Ser Phe Thr Ser Leu Leu Ala Ala Ser Pro Pro Ser Arg Ala
   1               5                   10                  15
   Ser Cys Arg Pro Ala Ala Glu Val Glu Ser Val Ala Val Glu Lys Arg
               20                  25                  30
<210> 13
   <211> 16
   <212> PRT
   <213> Trichoderma reesei
<400> 13
   Met Lys Ala Asn Val Ile Leu Cys Leu Leu Ala Pro Leu Val Ala Ala
   1               5                   10                  15
<210> 14
   <211> 18
   <212> PRT
   <213> Trichoderma reesei
<400> 14
   Met Ile Val Gly Ile Leu Thr Thr Leu Ala Thr Leu Ala Thr Leu Ala
   1               5                   10                  15
   Ala Ser
<210> 15
   <211> 17
   <212> PRT
   <213> Trichoderma reesei
<400> 15
   Met Tyr Arg Lys Leu Ala Val Ile Ser Ala Phe Leu Ala Thr Ala Arg
   1               5                   10                  15
   Ala
<210> 16
   <211> 23
   <212> PRT
   <213> Fusarium verticillioides
<400> 16
   Met Leu Leu Asn Leu Gln Val Ala Ala Ser Ala Leu Ser Leu Ser Leu
   1               5                   10                  15
   Leu Gly Gly Leu Ala Glu Ala
               20
<210> 17
   <211> 19
   <212> PRT
   <213> Fusarium verticillioides
<400> 17
   Met Lys Leu Asn Trp Val Ala Ala Ala Leu Ser Ile Gly Ala Ala Gly
   1 5 10 15
   Thr Asp Ser
<210> 18
   <211> 19
   <212> PRT
   <213> Fusarium verticillioides
<400> 18
   Met Ala Ser Ile Arg Ser Val Leu Val Ser Gly Leu Leu Ala Ala Gly
   1 5 10 15
   Val Asn Ala
<210> 19
   <211> 22
   <212> PRT
   <213> Fusarium verticillioides
<400> 19
   Met Trp Leu Thr Ser Pro Leu Leu Phe Ala Ser Thr Leu Leu Gly Leu
   1 5 10 15
   Thr Gly Val Ala Leu Ala
   20
<210> 20
   <211> 16
   <212> PRT
   <213> Fusarium verticillioides
<400> 20
   Met Arg Phe Ser Trp Leu Leu Cys Pro Leu Leu Ala Met Gly Ser Ala
   1 5 10 15
<210> 21
   <211> 22
   <212> PRT
   <213> Fusarium verticillioides
<400> 21
   Met Arg Leu Leu Ser Phe Pro Ser His Leu Leu Val Ala Phe Leu Thr
   1 5 10 15
   Leu Lys Glu Ala Ser Ser
   20
<210> 22
   <211> 20
   <212> PRT
   <213> Fusarium verticillioides
<400> 22
   Met Gln Leu Lys Phe Leu Ser Ser Ala Leu Leu Leu Ser Leu Thr Gly
   1               5                   10                  15
   Asn Cys Ala Ala
               20
<210> 23
   <211> 18
   <212> PRT
   <213> Fusarium verticillioides
<400> 23
   Met Lys Val Tyr Trp Leu Val Ala Trp Ala Thr Ser Leu Thr Pro Ala
   1               5                   10                  15
   Leu Ala
<210> 24
   <211> 19
   <212> PRT
   <213> Fusarium verticillioides
<400> 24
   Met Val Arg Phe Ser Ser Ile Leu Ala Ala Ala Ala Cys Phe Val Ala
   1               5                   10                  15
   Val Glu Ser
<210> 25
   <211> 20
   <212> PRT
   <213> Podospora anserine
<400> 25
   Met Ile His Leu Lys Pro Ala Leu Ala Ala Leu Leu Ala Leu Ser Thr
   1               5                   10                  15
   Gln Cys Val Ala
               20
<210> 26
   <211> 17
   <212> PRT
   <213> Podospora anserine
<400> 26
   Met Ala Leu Gln Thr Phe Phe Leu Leu Ala Ala Ala Met Leu Ala Asn
   1               5                   10                  15
   Ala
<210> 27
   <211> 19
   <212> PRT
   <213> Podospora anserine
<400> 27
   Met Lys Leu Asn Lys Pro Phe Leu Ala Ile Tyr Leu Ala Phe Asn Leu
   1               5                   10                  15
   Ala Glu Ala
<210> 28
   <211> 20
   <212> PRT
   <213> Chaetomium globosum
<400> 28
   Met Ala Pro Leu Ser Leu Arg Ala Leu Ser Leu Leu Ala Leu Thr Gly
   1               5                   10                  15
   Ala Ala Ala Ala
               20
<210> 29
   <211> 19
   <212> PRT
   <213> Thermoascus aurantiacus
<400> 29
   Met Val Arg Pro Thr Ile Leu Leu Thr Ser Leu Leu Leu Ala Pro Phe
   1               5                   10                  15
   Ala Ala Ala
<210> 30
   <211> 21
   <212> PRT
   <213> Aspergillus terreus
<400> 30
   Met His Met His Ser Leu Val Ala Ala Leu Ala Ala Gly Thr Leu Pro
   1 5 10 15
   Leu Leu Ala Ser Ala
   20
<210> 31
   <211> 19
   <212> PRT
   <213> Aspergillus fumigatus
<400> 31
   Met Val His Leu Ser Ser Leu Ala Ala Ala Leu Ala Ala Leu Pro Leu
   1 5 10 15
   Val Tyr Gly
<210> 32
   <211> 17
   <212> PRT
   <213> Aspergillus fumigatus
<400> 32
   Met Arg Phe Ser Leu Ala Ala Thr Thr Leu Leu Ala Gly Leu Ala Thr
   1 5 10 15
   Ala
<210> 33
   <211> 19
   <212> PRT
   <213> Aspergillus fumigatus
<400> 33
   Met Val Val Leu Ser Lys Leu Val Ser Ser Ile Leu Phe Ala Ser Leu
   1 5 10 15
   Val Ser Ala
<210> 34
   <211> 19
   <212> PRT
   <213> Aspergillus kawachii
<400> 34
   Met Val Gln Ile Lys Ala Ala Ala Leu Ala Met Leu Phe Ala Ser His
   1 5 10 15
   Val Leu Ser
<210> 35
   <211> 17
   <212> PRT
   <213> Magnaporthe grisea
<400> 35
   Met Lys Ala Ser Ser Val Leu Leu Gly Leu Ala Pro Leu Ala Ala Leu
   1               5                   10                  15
   Ala
<210> 36
   <211> 19
   <212> PRT
   <213> Saccharomyces cerevisiae
<400> 36
   Met Arg Phe Pro Ser Ile Phe Thr Ala Val Leu Phe Ala Ala Ser Ser
   1               5                   10                  15
   Ala Leu Ala
<210> 37
   <211> 85
   <212> PRT
   <213> Saccharomyces cerevisiae
<400> 37
   Met Arg Phe Pro Ser Ile Phe Thr Ala Val Leu Phe Ala Ala Ser Ser
   1               5                   10                  15
   Ala Leu Ala Ala Pro Val Asn Thr Thr Thr Glu Asp Glu Thr Ala Gln
               20                  25                  30
   Ile Pro Ala Glu Ala Val Ile Gly Tyr Leu Asp Leu Glu Gly Asp Phe
           35                  40                  45
   Asp Val Ala Val Leu Pro Phe Ser Asn Ser Thr Asn Asn Gly Leu Leu
       50                  55                  60
   Phe Ile Asn Thr Thr Ile Ala Ser Ile Ala Ala Lys Glu Glu Gly Val
    65                  70                  75                  80
   Ser Leu Asp Lys Arg
                   85
<210> 38
   <211> 20
   <212> PRT
   <213> Saccharomyces cerevisiae
<400> 38
   Met Leu Leu Gln Ala Phe Leu Phe Leu Leu Ala Gly Phe Ala Ala Lys
   1               5                   10                  15
   Ile Ser Ala Arg
               20
<210> 39
   <211> 29
   <212> PRT
   <213> Zymomonas mobilis
<400> 39
   Met Ile Lys Val Pro Arg Phe Ile Cys Met Ile Ala Leu Thr Ser Ser
   1               5                   10                  15
   Val Leu Ala Ser Gly Leu Ser Gln Ser Val Ser Ala His
               20                  25
<210> 40
   <211> 30
   <212> PRT
   <213> Zymomonas mobilis
<400> 40
   Met Lys Arg Lys Leu Gly Arg Arg Gln Leu Leu Thr Gly Phe Val Ala
   1               5                   10                  15
   Leu Gly Gly Met Ala Ile Thr Ala Gly Lys Ala Gln Ala Ser
               20                  25                  30
<210> 41
   <211> 15441
   <212> DNA
   <213> artificial sequence
<220>
   <223> synthetic construct
<400> 41
   tcaggaaata gctttaagta gcttattaag tattaaaatt atatatattt ttaatataac        60
   tatatttctt taataaatag gtattttaag ctttatatat aaatataata ataaaataat 120
   atattatata gctttttatt aataaataaa atagctaaaa atataaaaaa aatagcttta 180
   aaatacttat ttttaattag aattttatat atttttaata tataagatct tttacttttt 240
   tataagcttc ctaccttaaa ttaaattttt actttttttt actattttac tatatcttaa 300
   ataaaggctt taaaaatata aaaaaaatct tcttatatat tataagctat aaggattata 360
   tatatatttt tttttaattt ttaaagtaag tattaaagct agaattaaag ttttaatttt 420
   ttaaggcttt atttaaaaaa aggcagtaat agcttataaa agaaatttct ttttctttta 480
   tactaaaagt actttttttt taataaggtt agggttaggg tttactcaca ccgaccatcc 540
   caaccacatc ttagggttag ggttagggtt agggttaggg ttagggttag ggttagggta 600
   agggtttaaa caaagccacg ttgtgtctca aaatctctga tgttacattg cacaagataa 660
   aaatatatca tcatgaacaa taaaactgtc tgcttacata aacagtaata caaggggtgt 720
   tatgagccat attcaacggg aaacgtcttg ctcgaggccg cgattaaatt ccaacatgga 780
   tgctgattta tatgggtata aatgggctcg cgataatgtc gggcaatcag gtgcgacaat 840
   ctatcgattg tatgggaagc ccgatgcgcc agagttgttt ctgaaacatg gcaaaggtag 900
   cgttgccaat gatgttacag atgagatggt cagactaaac tggctgacgg aatttatgcc 960
   tcttccgacc atcaagcatt ttatccgtac tcctgatgat gcatggttac tcaccactgc 1020
   gatccccggg aaaacagcat tccaggtatt agaagaatat cctgattcag gtgaaaatat 1080
   tgttgatgcg ctggcagtgt tcctgcgccg gttgcattcg attcctgttt gtaattgtcc 1140
   ttttaacagc gatcgcgtat ttcgtctcgc tcaggcgcaa tcacgaatga ataacggttt 1200
   ggttgatgcg agtgattttg atgacgagcg taatggctgg cctgttgaac aagtctggaa 1260
   agaaatgcat aagcttttgc cattctcacc ggattcagtc gtcactcatg gtgatttctc 1320
   acttgataac cttatttttg acgaggggaa attaataggt tgtattgatg ttggacgagt 1380
   cggaatcgca gaccgatacc aggatcttgc catcctatgg aactgcctcg gtgagttttc 1440
   tccttcatta cagaaacggc tttttcaaaa atatggtatt gataatcctg atatgaataa 1500
   attgcagttt catttgatgc tcgatgagtt tttctaatca gaattggtta attggttgta 1560
   acactggcag agcattacgc tgacttgacg ggacggcggc tttgttgaat aaatcgaact 1620
   tttgctgagt tgaaggatca gatcacgcat cttcccgaca acgcagaccg ttccgtggca 1680
   aagcaaaagt tcaaaatcac caactggtcc acctacaaca aagctctcat caaccgtggc 1740
   tccctcactt tctggctgga tgatggggcg attcaggcct ggtatgagtc agcaacacct 1800
   tcttcacgag gcagacctca gcggtttaaa cctaacccta accctaaccc taaccctaac 1860
   cctaacccta accctaaccc taaccctaac cctaacccta accctaaccc taacctaacc 1920
   ctaatggggt cgatctgaac cgaggatgag ggttctatag actaatctac aggccgtaca 1980
   tggtgtgatt gcagatgcga cgggcaaggt gtacagtgtc cagaaggagg agagcggcat 2040
   aggtattgta atagaccagc tttacataat aatcgcctgt tgctactgac tgatgacctt 2100
   cttccctaac cagtttccta attaccactg cagtgaggat aaccctaact cgctctgggg 2160
   ttattattat actgattagc aggtggctta tatagtgctg aagtactata agagtttctg 2220
   cgggaggagg tggaaggact ataaactgga cacagttagg gatagagtga tgacaagacc 2280
   tgaatgttat cctccggtgt ggtatagcga attggctgac cttgcagatg gtaatggttt 2340
   aggcagggtt tttgcagagg gggacgagaa cgcgttctgc gatttaacgg ctgctgccgc 2400
   caagctttac ggttctctaa tgggcggccg cctcaggtcg acgtcccatg gccattcgaa 2460
   ttcgtaatca tgtcatagct gtttcctgtg tgaaattgtt atccgctcac aattccacac 2520
   aacatacgag ccggaagcat aaagtgtaaa gcctggggtg cctaatgagt gagctaactc 2580
   acattaattg cgttgcgctc actgcccgct ttccagtcgg gaaacctgtc gtgccagctg 2640
   cattaatgaa tcggccaacg cgtggggaga ggcggtttgc gtattgggcg ctcttccgct 2700
   tcctcgctca ctgactcgct gcgctcggtc gttcggctgc ggcgagcggt atcagctcac 2760
   tcaaaggcgg taatacggtt atccacagaa tcaggggata acgcaggaaa gaacatgtga 2820
   gcaaaaggcc agcaaaaggc caggaaccgt aaaaaggccg cgttgctggc gtttttccat 2880
   aggctccgcc cccctgacga gcatcacaaa aatcgacgct caagtcagag gtggcgaaac 2940
   ccgacaggac tataaagata ccaggcgttt ccccctggaa gctccctcgt gcgctctcct 3000
   gttccgaccc tgccgcttac cggatacctg tccgcctttc tcccttcggg aagcgtggcg 3060
   ctttctcata gctcacgctg taggtatctc agttcggtgt aggtcgttcg ctccaagctg 3120
   ggctgtgtgc acgaaccccc cgttcagccc gaccgctgcg ccttatccgg taactatcgt 3180
   cttgagtcca acccggtaag acacgactta tcgccactgg cagcagccac tggtaacagg 3240
   attagcagag cgaggtatgt aggcggtgct acagagttct tgaagtggtg gcctaactac 3300
   ggctacacta gaagaacagt atttggtatc tgcgctctgc tgaagccagt taccttcgga 3360
   aaaagagttg gtagctcttg atccggcaaa caaaccaccg ctggtagcgg tggttttttt 3420
   gtttgcaagc agcagattac gcgcagaaaa aaaggatctc aagaagatcc tttgatcttt 3480
   tctacggggt ctgacgctca gtggaacgaa aactcacgtt aagggatttt ggtcatgaga 3540
   ttatcaaaaa ggatcttcac ctagatcctt ttaaattaaa aatgaagttt taaatcaatc 3600
   taaagtatat atgagtaaac ttggtctgac agttaccaat gcttaatcag tgaggcacct 3660
   atctcagcga tctgtctatt tcgttcatcc atagttgcct gactccccgt cgtgtagata 3720
   actacgatac gggagggctt accatctggc cccagtgctg caatgatacc gcgagaccca 3780
   cgctcaccgg ctccagattt atcagcaata aaccagccag ccggaagggc cgagcgcaga 3840
   agtggtcctg caactttatc cgcctccatc cagtctatta attgttgccg ggaagctaga 3900
   gtaagtagtt cgccagttaa tagtttgcgc aacgttgttg ccattgctac aggcatcgtg 3960
   gtgtcacgct cgtcgtttgg tatggcttca ttcagctccg gttcccaacg atcaaggcga 4020
   gttacatgat cccccatgtt gtgcaaaaaa gcggttagct ccttcggtcc tccgatcgtt 4080
   gtcagaagta agttggccgc agtgttatca ctcatggtta tggcagcact gcataattct 4140
   cttactgtca tgccatccgt aagatgcttt tctgtgactg gtgagtactc aaccaagtca 4200
   ttctgagaat agtgtatgcg gcgaccgagt tgctcttgcc cggcgtcaat acgggataat 4260
   accgcgccac atagcagaac tttaaaagtg ctcatcattg gaaaacgttc ttcggggcga 4320
   aaactctcaa ggatcttacc gctgttgaga tccagttcga tgtaacccac tcgtgcaccc 4380
   aactgatctt cagcatcttt tactttcacc agcgtttctg ggtgagcaaa aacaggaagg 4440
   caaaatgccg caaaaaaggg aataagggcg acacggaaat gttgaatact catactcttc 4500
   ctttttcaat attattgaag catttatcag ggttattgtc tcatgagcgg atacatattt 4560
   gaatgtattt agaaaaataa acaaataggg gttccgcgca catttccccg aaaagtgcca 4620
   cctgacgtct aagaaaccat tattatcatg acattaacct ataaaaatag gcgtatcacg 4680
   aggccctttc gtctcgcgcg tttcggtgat gacggtgaaa acctctgaca catgcagctc 4740
   ccggagacgg tcacagcttg tctgtaagcg gatgccggga gcagacaagc ccgtcagggc 4800
   gcgtcagcgg gtgttggcgg gtgtcggggc tggcttaact atgcggcatc agagcagatt 4860
   gtactgagag tgcaccataa aattgtaaac gttaatattt tgttaaaatt cgcgttaaat 4920
   ttttgttaaa tcagctcatt ttttaaccaa taggccgaaa tcggcaaaat cccttataaa 4980
   tcaaaagaat agcccgagat agggttgagt gttgttccag tttggaacaa gagtccacta 5040
   ttaaagaacg tggactccaa cgtcaaaggg cgaaaaaccg tctatcaggg cgatggccca 5100
   ctacgtgaac catcacccaa atcaagtttt ttggggtcga ggtgccgtaa agcactaaat 5160
   cggaacccta aagggagccc ccgatttaga gcttgacggg gaaagccggc gaacgtggcg 5220
   agaaaggaag ggaagaaagc gaaaggagcg ggcgctaggg cgctggcaag tgtagcggtc 5280
   acgctgcgcg taaccaccac acccgccgcg cttaatgcgc cgctacaggg cgcgtactat 5340
   ggttgctttg acgtatgcgg tgtgaaatac cgcacagatg cgtaaggaga aaataccgca 5400
   tcaggcgcca ttcgccattc aggctgcgca actgttggga agggcgatcg gtgcgggcct 5460
   cttcgctatt acgccagctg gcgaaagggg gatgtgctgc aaggcgatta agttgggtaa 5520
   cgccagggtt ttcccagtca cgacgttgta aaacgacggc cagtgccaag cttactagat 5580
   gcatgctcga gcggccgcca gtgtgatgga tatctgcaga attcgccctt attcgccctt 5640
   gactagtgat ctatgctctt caccgttcag agacgaaact tttacttcat tagggggagt 5700
   ccaacttttc attctcactc tgtgagaagc tgtgtcacaa tacttcagag gcattaaagc 5760
   tacctcagct ctttaaacat gtcaattcta tcaaaattag gtatgtgatt caagtagtga 5820
   acaatatgtg gccgttactc gagtttataa gtgacaacat gctctcaaag cgctcatggc 5880
   tggcacaagc ctggaaagaa ccaacacaaa gcatactgca gcaaatcagc tgaattcgtc 5940
   accaattaag tgaacatcaa cctgaaggca gagtatgagg ccagaagcac atctggatcg 6000
   cagatcatgg attgcccctc ttgttgaaga tgagaatcta gaaagatggc ggggtatgag 6060
   ataagagcga tgggggggca catcatcttc caagacaaac aacctttgca gagtcaggca 6120
   atttttcgta taagagcagg aggagggagt ccagtcattt catcagcggt aaaatcactc 6180
   tagacaatct tcaagatgag ttctgccttg ggtgacttat agccatcatc atacctagac 6240
   agaagcttgt gggatactaa gaccaacgta caagctcgca ctgtacgctt tgacttccat 6300
   gtgaaaactc gatacggcgc gcctctaaat tttatagctc aaccactcca atccaacctc 6360
   tgcatccctc tcactcgtcc tgatctactg ttcaaatcag agaataagga cactatccaa 6420
   atccaacaga atggctacca cctcccagct gcctgcctac aagcaggact tcctcaaatc 6480
   cgccatcgac ggcggcgtcc tcaagtttgg cagcttcgag ctcaagtcca agcggatatc 6540
   cccctacttc ttcaacgcgg gcgaattcca cacggcgcgc ctcgccggcg ccatcgcctc 6600
   cgcctttgca aagaccatca tcgaggccca ggagaaggcc ggcctagagt tcgacatcgt 6660
   cttcggcccg gcctacaagg gcatcccgct gtgctccgcc atcaccatca agctcggcga 6720
   gctggcgccc cagaacctgg accgcgtctc ctactcgttt gaccgcaagg aggccaagga 6780
   ccacggcgag ggcggcaaca tcgtcggcgc ttcgctcaag ggcaagaggg tcctgattgt 6840
   cgacgacgtc atcaccgccg gcaccgccaa gagggacgcc attgagaaga tcaccaagga 6900
   gggcggcatc gtcgccggca tcgtcgtggc cctggaccgc atggagaagc tccccgctgc 6960
   ggatggcgac gactccaagc ctggaccgag tgccattggc gagctgagga aggagtacgg 7020
   catccccatc tttgccatcc tcactctgga tgacattatc gatggcatga agggctttgc     7080
   tacccctgag gatatcaaga acacggagga ttaccgtgcc aagtacaagg cgactgactg     7140
   attgaggcgt tcaatgtcag aagggagaga aagactgaaa aggtggaaag aagaggcaaa 7200
   ttgttgttat tattattatt ctatctcgaa tcttctagat cttgtcgtaa ataaacaagc 7260
   gtaactagct agcctccgta caactgcttg aatttgatac ccgtatggag ggcagttatt 7320
   ttattttgtt tttcaagatt ttccattcgc cgttgaactc gtctcacatc gcgtgtattg 7380
   cccggttgcc catgtgttct cctactaccc caagtccctc acgggttgtc tcactttctt 7440
   tctcctttat cctccctatt ttttttcaag tcagcgacag agcagtcata tggggatacg 7500
   tgcaactggg actcacaaca ggccatctta tggcctaata gccggcgttg gcaattgctt 7560
   gctttcgcca gtctgttgat actctcgtga gccactccgc tcacgatatc ccttcattgt 7620
   gcagcttcct tttcccttcc aattcctgct gcttccagac tatgccccgg gctgctttgt 7680
   tgatcgccct ctcgcccttc cataccattt ttctagttga tcattaatac cattacgctc 7740
   catgtttaat gatcatattc ctgatgcaaa cagcgccgtg aacgttttct tcacatagac 7800
   atgcgtctgc cgtgtcccaa ggtatacaaa gagccgccaa agaaacaaac aaagaaggtc 7860
   gacatgcgta gcacacatcg tatcagtcaa atggtaccgg gaggaaggct ggaaagctta 7920
   cgagaaaaga gttggacttt gagtgtgagt ggaaatgtgt aacggtattg actaaaaggg 7980
   atccatatgt ttattgcagc cagcatagta ttaccagaaa gagcctcact gacggctcta 8040
   gtagtattcg aacagatatt attgtgacca gctctgaacg atatgctccc taatctggta 8100
   gacaagcact gatctacccc ttggaacgca gcatctaggc tctggctgtg ctctaaccct 8160
   aactagacga ttgatcgcag accatccaat actgaaaagt ctctatcaga ggaaatcccc 8220
   aacattgtag tagtcaggtt cctttgtggc tgggagagaa ttggttcgct ccactgattc 8280
   cagttgagaa agtgggctag aaaaaagtct tgaagattgg agttgggctg tggttatcta 8340
   gtacttctcg agctctgtac atgtccggtc gcgacgtacg cgtatcgatg gcgccagctg 8400
   caggcggccg cctgcagcca cttgcagtcc cgtggaattc tcacggtgaa tgtaggcctt 8460
   ttgtagggta ggaattgtca ctcaagcacc cccaacctcc attacgcctc ccccatagag 8520
   ttcccaatca gtgagtcatg gcactgttct caaatagatt ggggagaagt tgacttccgc 8580
   ccagagctga aggtcgcaca accgcatgat atagggtcgg caacggcaaa aaagcacgtg 8640
   gctcaccgaa aagcaagatg tttgcgatct aacatccagg aacctggata catccatcat 8700
   cacgcacgac cactttgatc tgctggtaaa ctcgtattcg ccctaaaccg aagtgcgtgg 8760
   taaatctaca cgtgggcccc tttcggtata ctgcgtgtgt cttctctagg tgccattctt 8820
   ttcccttcct ctagtgttga attgtttgtg ttggagtccg agctgtaact acctctgaat 8880
   ctctggagaa tggtggacta acgactaccg tgcacctgca tcatgtatat aatagtgatc 8940
   ctgagaaggg gggtttggag caatgtggga ctttgatggt catcaaacaa agaacgaaga 9000
   cgcctctttt gcaaagtttt gtttcggcta cggtgaagaa ctggatactt gttgtgtctt 9060
   ctgtgtattt ttgtggcaac aagaggccag agacaatcta ttcaaacacc aagcttgctc 9120
   ttttgagcta caagaacctg tggggtatat atctagagtt gtgaagtcgg taatcccgct 9180
   gtatagtaat acgagtcgca tctaaatact ccgaagctgc tgcgaacccg gagaatcgag 9240
   atgtgctgga aagcttctag cgagcggcta aattagcatg aaaggctatg agaaattctg 9300
   gagacggctt gttgaatcat ggcgttccat tcttcgacaa gcaaagcgtt ccgtcgcagt 9360
   agcaggcact cattcccgaa aaaactcgga gattcctaag tagcgatgga accggaataa 9420
   tataataggc aatacattga gttgcctcga cggttgcaat gcaggggtac tgagcttgga 9480
   cataactgtt ccgtacccca cctcttctca acctttggcg tttccctgat tcagcgtacc 9540
   cgtacaagtc gtaatcacta ttaacccaga ctgaccggac gtgttttgcc cttcatttgg 9600
   agaaataatg tcattgcgat gtgtaatttg cctgcttgac cgactggggc tgttcgaagc 9660
   ccgaatgtag gattgttatc cgaactctgc tcgtagaggc atgttgtgaa tctgtgtcgg 9720
   gcaggacacg cctcgaaggt tcacggcaag ggaaaccacc gatagcagtg tctagtagca 9780
   acctgtaaag ccgcaatgca gcatcactgg aaaatacaaa ccaatggcta aaagtacata 9840
   agttaatgcc taaagaagtc atataccagc ggctaataat tgtacaatca agtggctaaa 9900
   cgtaccgtaa tttgccaacg gcttgtgggg ttgcagaagc aacggcaaag ccccacttcc 9960
   ccacgtttgt ttcttcactc agtccaatct cagctggtga tcccccaatt gggtcgcttg 10020
   tttgttccgg tgaagtgaaa gaagacagag gtaagaatgt ctgactcgga gcgttttgca 10080
   tacaaccaag ggcagtgatg gaagacagtg aaatgttgac attcaaggag tatttagcca 10140
   gggatgcttg agtgtatcgt gtaaggaggt ttgtctgccg atacgacgaa tactgtatag 10200
   tcacttctga tgaagtggtc catattgaaa tgtaagtcgg cactgaacag gcaaaagatt 10260
   gagttgaaac tgcctaagat ctcgggccct cgggccttcg gcctttgggt gtacatgttt 10320
   gtgctccggg caaatgcaaa gtgtggtagg atcgaacaca ctgctgcctt taccaagcag 10380
   ctgagggtat gtgataggca aatgttcagg ggccactgca tggtttcgaa tagaaagaga 10440
   agcttagcca agaacaatag ccgataaaga tagcctcatt aaacggaatg agctagtagg 10500
   caaagtcagc gaatgtgtat atataaaggt tcgaggtccg tgcctccctc atgctctccc 10560
   catctactca tcaactcaga tcctccagga gacttgtaca ccatcttttg aggcacagaa 10620
   acccaatagt caaccatcac aagtttgtac aaaaaagctg aacgagaaac gtaaaatgat 10680
   ataaatatca atatattaaa ttagattttg cataaaaaac agactacata atactgtaaa 10740
   acacaacata tccagtcact atggcggccg cattaggcac cccaggcttt acactttatg 10800
   cttccggctc gtataatgtg tggattttga gttaggatcc gtcgagattt tcaggagcta 10860
   aggaagctaa aatggagaaa aaaatcactg gatataccac cgttgatata tcccaatggc 10920
   atcgtaaaga acattttgag gcatttcagt cagttgctca atgtacctat aaccagaccg 10980
   ttcagctgga tattacggcc tttttaaaga ccgtaaagaa aaataagcac aagttttatc 11040
   cggcctttat tcacattctt gcccgcctga tgaatgctca tccggaattc cgtatggcaa 11100
   tgaaagacgg tgagctggtg atatgggata gtgttcaccc ttgttacacc gttttccatg 11160
   agcaaactga aacgttttca tcgctctgga gtgaatacca cgacgatttc cggcagtttc 11220
   tacacatata ttcgcaagat gtggcgtgtt acggtgaaaa cctggcctat ttccctaaag 11280
   ggtttattga gaatatgttt ttcgtctcag ccaatccctg ggtgagtttc accagttttg 11340
   atttaaacgt ggccaatatg gacaacttct tcgcccccgt tttcaccatg ggcaaatatt 11400
   atacgcaagg cgacaaggtg ctgatgccgc tggcgattca ggttcatcat gccgtttgtg 11460
   atggcttcca tgtcggcaga atgcttaatg aattacaaca gtactgcgat gagtggcagg 11520
   gcggggcgta aacgcgtgga tccggcttac taaaagccag ataacagtat gcgtatttgc 11580
   gcgctgattt ttgcggtata agaatatata ctgatatgta tacccgaagt atgtcaaaaa 11640
   gaggtatgct atgaagcagc gtattacagt gacagttgac agcgacagct atcagttgct 11700
   caaggcatat atgatgtcaa tatctccggt ctggtaagca caaccatgca gaatgaagcc 11760
   cgtcgtctgc gtgccgaacg ctggaaagcg gaaaatcagg aagggatggc tgaggtcgcc 11820
   cggtttattg aaatgaacgg ctcttttgct gacgagaaca ggggctggtg aaatgcagtt 11880
   taaggtttac acctataaaa gagagagccg ttatcgtctg tttgtggatg tacagagtga 11940
   tattattgac acgcccgggc gacggatggt gatccccctg gccagtgcac gtctgctgtc 12000
   agataaagtc tcccgtgaac tttacccggt ggtgcatatc ggggatgaaa gctggcgcat 12060
   gatgaccacc gatatggcca gtgtgccggt ctccgttatc ggggaagaag tggctgatct 12120
   cagccaccgc gaaaatgaca tcaaaaacgc cattaacctg atgttctggg gaatataaat 12180
   gtcaggctcc cttatacaca gccagtctgc aggtcgacca tagtgactgg atatgttgtg 12240
   ttttacagta ttatgtagtc tgttttttat gcaaaatcta atttaatata ttgatattta 12300
   tatcatttta cgtttctcgt tcagctttct tgtacaaagt ggtgatcgcg ccagctccgt 12360
   gcgaaagcct gacgcaccgg tagattcttg gtgagcccgt atcatgacgg cggcgggagc 12420
   tacatggccc cgggtgattt attttttttg tatctacttc tgaccctttt caaatatacg 12480
   gtcaactcat ctttcactgg agatgcggcc tgcttggtat tgcgatgttg tcagcttggc 12540
   aaattgtggc tttcgaaaac acaaaacgat tccttagtag ccatgcattt taagataacg 12600
   gaatagaaga aagaggaaat taaaaaaaaa aaaaaaacaa acatcccgtt cataacccgt 12660
   agaatcgccg ctcttcgtgt atcccagtac cagtttattt tgaatagctc gcccgctgga 12720
   gagcatcctg aatgcaagta acaaccgtag aggctgacac ggcaggtgtt gctagggagc 12780
   gtcgtgttct acaaggccag acgtcttcgc ggttgatata tatgtatgtt tgactgcagg 12840
   ctgctcagcg acgacagtca agttcgccct cgctgcttgt gcaataatcg cagtggggaa 12900
   gccacaccgt gactcccatc tttcagtaaa gctctgttgg tgtttatcag caatacacgt 12960
   aatttaaact cgttagcatg gggctgatag cttaattacc gtttaccagt gccatggttc 13020
   tgcagctttc cttggcccgt aaaattcggc gaagccagcc aatcaccagc taggcaccag 13080
   ctaaacccta taattagtct cttatcaaca ccatccgctc cccgggatca aatgaggaga 13140
   atgaggggga tgcggggcta aagaagccta cataaccctc atgccaactc ccagtttaca 13200
   ctcgtcgagc caacatcctg actataagct aacacagaat gcctcaatcc tgggaagaac 13260
   tggccgctga taagcgcgcc cgcctcgcaa aaaccatccc tgatgaatgg aaagtccaga 13320
   cgctgcctgc ggaagacagc gttattgatt tcccaaagaa atcggggatc ctttcagagg 13380
   ccgaactgaa gatcacagag gcctccgctg cagatcttgt gtccaagctg gcggccggag 13440
   agttgacctc ggtggaagtt acgctagcat tctgtaaacg ggcagcaatc gcccagcagt 13500
   tagtagggtc ccctctacct ctcagggaga tgtaacaacg ccaccttatg ggactatcaa 13560
   gctgacgctg gcttctgtgc agacaaactg cgcccacgag ttcttccctg acgccgctct 13620
   cgcgcaggca agggaactcg atgaatacta cgcaaagcac aagagacccg ttggtccact 13680
   ccatggcctc cccatctctc tcaaagacca gcttcgagtc aaggtacacc gttgccccta 13740
   agtcgttaga tgtccctttt tgtcagctaa catatgccac cagggctacg aaacatcaat 13800
   gggctacatc tcatggctaa acaagtacga cgaaggggac tcggttctga caaccatgct 13860
   ccgcaaagcc ggtgccgtct tctacgtcaa gacctctgtc ccgcagaccc tgatggtctg 13920
   cgagacagtc aacaacatca tcgggcgcac cgtcaaccca cgcaacaaga actggtcgtg 13980
   cggcggcagt tctggtggtg agggtgcgat cgttgggatt cgtggtggcg tcatcggtgt 14040
   aggaacggat atcggtggct cgattcgagt gccggccgcg ttcaacttcc tgtacggtct 14100
   aaggccgagt catgggcggc tgccgtatgc aaagatggcg aacagcatgg agggtcagga 14160
   gacggtgcac agcgttgtcg ggccgattac gcactctgtt gagggtgagt ccttcgcctc 14220
   ttccttcttt tcctgctcta taccaggcct ccactgtcct cctttcttgc tttttatact 14280
   atatacgaga ccggcagtca ctgatgaagt atgttagacc tccgcctctt caccaaatcc 14340
   gtcctcggtc aggagccatg gaaatacgac tccaaggtca tccccatgcc ctggcgccag 14400
   tccgagtcgg acattattgc ctccaagatc aagaacggcg ggctcaatat cggctactac 14460
   aacttcgacg gcaatgtcct tccacaccct cctatcctgc gcggcgtgga aaccaccgtc 14520
   gccgcactcg ccaaagccgg tcacaccgtg accccgtgga cgccatacaa gcacgatttc 14580
   ggccacgatc tcatctccca tatctacgcg gctgacggca gcgccgacgt aatgcgcgat 14640
   atcagtgcat ccggcgagcc ggcgattcca aatatcaaag acctactgaa cccgaacatc 14700
   aaagctgtta acatgaacga gctctgggac acgcatctcc agaagtggaa ttaccagatg 14760
   gagtaccttg agaaatggcg ggaggctgaa gaaaaggccg ggaaggaact ggacgccatc 14820
   atcgcgccga ttacgcctac cgctgcggta cggcatgacc agttccggta ctatgggtat 14880
   gcctctgtga tcaacctgct ggatttcacg agcgtggttg ttccggttac ctttgcggat 14940
   aagaacatcg ataagaagaa tgagagtttc aaggcggtta gtgagcttga tgccctcgtg 15000
   caggaagagt atgatccgga ggcgtaccat ggggcaccgg ttgcagtgca ggttatcgga 15060
   cggagactca gtgaagagag gacgttggcg attgcagagg aagtggggaa gttgctggga 15120
   aatgtggtga ctccatagct aataagtgtc agatagcaat ttgcacaaga aatcaatacc 15180
   agcaactgta aataagcgct gaagtgacca tgccatgcta cgaaagagca gaaaaaaacc    15240
   tgccgtagaa ccgaagagat atgacacgct tccatctctc aaaggaagaa tcccttcagg    15300
   gttgcgtttc cagtctagac acgtataacg gcacaagtgt ctctcaccaa atgggttata    15360
   tctcaaatgt gatctaagga tggaaagccc agaatatcga tcgcgcgcag atccatatat    15420
   agggcccggg ttataattac c                                              15441
<210> 42
   <211> 17043
   <212> DNA
   <213> artificial sequence
<220>
   <223> synthetic construct
<400> 42
   ttgtacaaag tggtgatcgc gccagctccg tgcgaaagcc tgacgcaccg gtagattctt 60
   ggtgagcccg tatcatgacg gcggcgggag ctacatggcc ccgggtgatt tatttttttt 120
   gtatctactt ctgacccttt tcaaatatac ggtcaactca tctttcactg gagatgcggc 180
   ctgcttggta ttgcgatgtt gtcagcttgg caaattgtgg ctttcgaaaa cacaaaacga 240
   ttccttagta gccatgcatt ttaagataac ggaatagaag aaagaggaaa ttaaaaaaaa 300
   aaaaaaaaca aacatcccgt tcataacccg tagaatcgcc gctcttcgtg tatcccagta 360
   ccagtttatt ttgaatagct cgcccgctgg agagcatcct gaatgcaagt aacaaccgta 420
   gaggctgaca cggcaggtgt tgctagggag cgtcgtgttc tacaaggcca gacgtcttcg 480
   cggttgatat atatgtatgt ttgactgcag gctgctcagc gacgacagtc aagttcgccc 540
   tcgctgcttg tgcaataatc gcagtgggga agccacaccg tgactcccat ctttcagtaa 600
   agctctgttg gtgtttatca gcaatacacg taatttaaac tcgttagcat ggggctgata 660
   gcttaattac cgtttaccag tgccatggtt ctgcagcttt ccttggcccg taaaattcgg 720
   cgaagccagc caatcaccag ctaggcacca gctaaaccct ataattagtc tcttatcaac 780
   accatccgct cccccgggat caatgaggag aatgaggggg atgcggggct aaagaagcct 840
   acataaccct catgccaact cccagtttac actcgtcgag ccaacatcct gactataagc 900
   taacacagaa tgcctcaatc ctgggaagaa ctggccgctg ataagcgcgc ccgcctcgca 960
   aaaaccatcc ctgatgaatg gaaagtccag acgctgcctg cggaagacag cgttattgat 1020
   ttcccaaaga aatcggggat cctttcagag gccgaactga agatcacaga ggcctccgct 1080
   gcagatcttg tgtccaagct ggcggccgga gagttgacct cggtggaagt tacgctagca 1140
   ttctgtaaac gggcagcaat cgcccagcag ttagtagggt cccctctacc tctcagggag 1200
   atgtaacaac gccaccttat gggactatca agctgacgct ggcttctgtg cagacaaact 1260
   gcgcccacga gttcttccct gacgccgctc tcgcgcaggc aagggaactc gatgaatact 1320
   acgcaaagca caagagaccc gttggtccac tccatggcct ccccatctct ctcaaagacc 1380
   agcttcgagt caaggtacac cgttgcccct aagtcgttag atgtcccttt ttgtcagcta 1440
   acatatgcca ccagggctac gaaacatcaa tgggctacat ctcatggcta aacaagtacg 1500
   acgaagggga ctcggttctg acaaccatgc tccgcaaagc cggtgccgtc ttctacgtca 1560
   agacctctgt cccgcagacc ctgatggtct gcgagacagt caacaacatc atcgggcgca 1620
   ccgtcaaccc acgcaacaag aactggtcgt gcggcggcag ttctggtggt gagggtgcga 1680
   tcgttgggat tcgtggtggc gtcatcggtg taggaacgga tatcggtggc tcgattcgag 1740
   tgccggccgc gttcaacttc ctgtacggtc taaggccgag tcatgggcgg ctgccgtatg 1800
   caaagatggc gaacagcatg gagggtcagg agacggtgca cagcgttgtc gggccgatta 1860
   cgcactctgt tgagggtgag tccttcgcct cttccttctt ttcctgctct ataccaggcc 1920
   tccactgtcc tcctttcttg ctttttatac tatatacgag accggcagtc actgatgaag 1980
   tatgttagac ctccgcctct tcaccaaatc cgtcctcggt caggagccat ggaaatacga 2040
   ctccaaggtc atccccatgc cctggcgcca gtccgagtcg gacattattg cctccaagat 2100
   caagaacggc gggctcaata tcggctacta caacttcgac ggcaatgtcc ttccacaccc 2160
   tcctatcctg cgcggcgtgg aaaccaccgt cgccgcactc gccaaagccg gtcacaccgt 2220
   gaccccgtgg acgccataca agcacgattt cggccacgat ctcatctccc atatctacgc 2280
   ggctgacggc agcgccgacg taatgcgcga tatcagtgca tccggcgagc cggcgattcc 2340
   aaatatcaaa gacctactga acccgaacat caaagctgtt aacatgaacg agctctggga 2400
   cacgcatctc cagaagtgga attaccagat ggagtacctt gagaaatggc gggaggctga 2460
   agaaaaggcc gggaaggaac tggacgccat catcgcgccg attacgccta ccgctgcggt 2520
   acggcatgac cagttccggt actatgggta tgcctctgtg atcaacctgc tggatttcac 2580
   gagcgtggtt gttccggtta cctttgcgga taagaacatc gataagaaga atgagagttt 2640
   caaggcggtt agtgagcttg atgccctcgt gcaggaagag tatgatccgg aggcgtacca 2700
   tggggcaccg gttgcagtgc aggttatcgg acggagactc agtgaagaga ggacgttggc 2760
   gattgcagag gaagtgggga agttgctggg aaatgtggtg actccatagc taataagtgt 2820
   cagatagcaa tttgcacaag aaatcaatac cagcaactgt aaataagcgc tgaagtgacc 2880
   atgccatgct acgaaagagc agaaaaaaac ctgccgtaga accgaagaga tatgacacgc 2940
   ttccatctct caaaggaaga atcccttcag ggttgcgttt ccagtctaga cacgtataac 3000
   ggcacaagtg tctctcacca aatgggttat atctcaaatg tgatctaagg atggaaagcc 3060
   cagaatatcg atcgcgcgca gatccatata tagggcccgg gttataatta cctcaggaaa 3120
   tagctttaag tagcttatta agtattaaaa ttatatatat ttttaatata actatatttc 3180
   tttaataaat aggtatttta agctttatat ataaatataa taataaaata atatattata 3240
   tagcttttta ttaataaata aaatagctaa aaatataaaa aaaatagctt taaaatactt 3300
   atttttaatt agaattttat atatttttaa tatataagat cttttacttt tttataagct 3360
   tcctacctta aattaaattt ttactttttt ttactatttt actatatctt aaataaaggc 3420
   tttaaaaata taaaaaaaat cttcttatat attataagct ataaggatta tatatatatt 3480
   tttttttaat ttttaaagta agtattaaag ctagaattaa agttttaatt ttttaaggct 3540
   ttatttaaaa aaaggcagta atagcttata aaagaaattt ctttttcttt tatactaaaa 3600
   gtactttttt tttaataagg ttagggttag ggtttactca caccgaccat cccaaccaca 3660
   tcttagggtt agggttaggg ttagggttag ggttagggtt agggttaggg taagggttta 3720
   aacaaagcca cgttgtgtct caaaatctct gatgttacat tgcacaagat aaaaatatat 3780
   catcatgaac aataaaactg tctgcttaca taaacagtaa tacaaggggt gttatgagcc 3840
   atattcaacg ggaaacgtct tgctcgaggc cgcgattaaa ttccaacatg gatgctgatt 3900
   tatatgggta taaatgggct cgcgataatg tcgggcaatc aggtgcgaca atctatcgat 3960
   tgtatgggaa gcccgatgcg ccagagttgt ttctgaaaca tggcaaaggt agcgttgcca 4020
   atgatgttac agatgagatg gtcagactaa actggctgac ggaatttatg cctcttccga 4080
   ccatcaagca ttttatccgt actcctgatg atgcatggtt actcaccact gcgatccccg 4140
   ggaaaacagc attccaggta ttagaagaat atcctgattc aggtgaaaat attgttgatg 4200
   cgctggcagt gttcctgcgc cggttgcatt cgattcctgt ttgtaattgt ccttttaaca 4260
   gcgatcgcgt atttcgtctc gctcaggcgc aatcacgaat gaataacggt ttggttgatg 4320
   cgagtgattt tgatgacgag cgtaatggct ggcctgttga acaagtctgg aaagaaatgc 4380
   ataagctttt gccattctca ccggattcag tcgtcactca tggtgatttc tcacttgata 4440
   accttatttt tgacgagggg aaattaatag gttgtattga tgttggacga gtcggaatcg 4500
   cagaccgata ccaggatctt gccatcctat ggaactgcct cggtgagttt tctccttcat 4560
   tacagaaacg gctttttcaa aaatatggta ttgataatcc tgatatgaat aaattgcagt 4620
   ttcatttgat gctcgatgag tttttctaat cagaattggt taattggttg taacactggc 4680
   agagcattac gctgacttga cgggacggcg gctttgttga ataaatcgaa cttttgctga 4740
   gttgaaggat cagatcacgc atcttcccga caacgcagac cgttccgtgg caaagcaaaa 4800
   gttcaaaatc accaactggt ccacctacaa caaagctctc atcaaccgtg gctccctcac 4860
   tttctggctg gatgatgggg cgattcaggc ctggtatgag tcagcaacac cttcttcacg 4920
   aggcagacct cagcggttta aacctaaccc taaccctaac cctaacccta accctaaccc 4980
   taaccctaac cctaacccta accctaaccc taaccctaac cctaacctaa ccctaatggg 5040
   gtcgatctga accgaggatg agggttctat agactaatct acaggccgta catggtgtga 5100
   ttgcagatgc gacgggcaag gtgtacagtg tccagaagga ggagagcggc ataggtattg 5160
   taatagacca gctttacata ataatcgcct gttgctactg actgatgacc ttcttcccta 5220
   accagtttcc taattaccac tgcagtgagg ataaccctaa ctcgctctgg ggttattatt 5280
   atactgatta gcaggtggct tatatagtgc tgaagtacta taagagtttc tgcgggagga 5340
   ggtggaagga ctataaactg gacacagtta gggatagagt gatgacaaga cctgaatgtt 5400
   atcctccggt gtggtatagc gaattggctg accttgcaga tggtaatggt ttaggcaggg 5460
   tttttgcaga gggggacgag aacgcgttct gcgatttaac ggctgctgcc gccaagcttt 5520
   acggttctct aatgggcggc cgcctcaggt cgacgtccca tggccattcg aattcgtaat 5580
   catgtcatag ctgtttcctg tgtgaaattg ttatccgctc acaattccac acaacatacg 5640
   agccggaagc ataaagtgta aagcctgggg tgcctaatga gtgagctaac tcacattaat 5700
   tgcgttgcgc tcactgcccg ctttccagtc gggaaacctg tcgtgccagc tgcattaatg 5760
   aatcggccaa cgcgtgggga gaggcggttt gcgtattggg cgctcttccg cttcctcgct 5820
   cactgactcg ctgcgctcgg tcgttcggct gcggcgagcg gtatcagctc actcaaaggc 5880
   ggtaatacgg ttatccacag aatcagggga taacgcagga aagaacatgt gagcaaaagg 5940
   ccagcaaaag gccaggaacc gtaaaaaggc cgcgttgctg gcgtttttcc ataggctccg 6000
   cccccctgac gagcatcaca aaaatcgacg ctcaagtcag aggtggcgaa acccgacagg 6060
   actataaaga taccaggcgt ttccccctgg aagctccctc gtgcgctctc ctgttccgac 6120
   cctgccgctt accggatacc tgtccgcctt tctcccttcg ggaagcgtgg cgctttctca 6180
   tagctcacgc tgtaggtatc tcagttcggt gtaggtcgtt cgctccaagc tgggctgtgt 6240
   gcacgaaccc cccgttcagc ccgaccgctg cgccttatcc ggtaactatc gtcttgagtc 6300
   caacccggta agacacgact tatcgccact ggcagcagcc actggtaaca ggattagcag 6360
   agcgaggtat gtaggcggtg ctacagagtt cttgaagtgg tggcctaact acggctacac 6420
   tagaagaaca gtatttggta tctgcgctct gctgaagcca gttaccttcg gaaaaagagt 6480
   tggtagctct tgatccggca aacaaaccac cgctggtagc ggtggttttt ttgtttgcaa 6540
   gcagcagatt acgcgcagaa aaaaaggatc tcaagaagat cctttgatct tttctacggg 6600
   gtctgacgct cagtggaacg aaaactcacg ttaagggatt ttggtcatga gattatcaaa 6660
   aaggatcttc acctagatcc ttttaaatta aaaatgaagt tttaaatcaa tctaaagtat 6720
   atatgagtaa acttggtctg acagttacca atgcttaatc agtgaggcac ctatctcagc 6780
   gatctgtcta tttcgttcat ccatagttgc ctgactcccc gtcgtgtaga taactacgat 6840
   acgggagggc ttaccatctg gccccagtgc tgcaatgata ccgcgagacc cacgctcacc 6900
   ggctccagat ttatcagcaa taaaccagcc agccggaagg gccgagcgca gaagtggtcc 6960
   tgcaacttta tccgcctcca tccagtctat taattgttgc cgggaagcta gagtaagtag     7020
   ttcgccagtt aatagtttgc gcaacgttgt tgccattgct acaggcatcg tggtgtcacg     7080
   ctcgtcgttt ggtatggctt cattcagctc cggttcccaa cgatcaaggc gagttacatg     7140
   atcccccatg ttgtgcaaaa aagcggttag ctccttcggt cctccgatcg ttgtcagaag     7200
   taagttggcc gcagtgttat cactcatggt tatggcagca ctgcataatt ctcttactgt     7260
   catgccatcc gtaagatgct tttctgtgac tggtgagtac tcaaccaagt cattctgaga     7320
   atagtgtatg cggcgaccga gttgctcttg cccggcgtca atacgggata ataccgcgcc     7380
   acatagcaga actttaaaag tgctcatcat tggaaaacgt tcttcggggc gaaaactctc     7440
   aaggatctta ccgctgttga gatccagttc gatgtaaccc actcgtgcac ccaactgatc     7500
   ttcagcatct tttactttca ccagcgtttc tgggtgagca aaaacaggaa ggcaaaatgc     7560
   cgcaaaaaag ggaataaggg cgacacggaa atgttgaata ctcatactct tcctttttca     7620
   atattattga agcatttatc agggttattg tctcatgagc ggatacatat ttgaatgtat     7680
   ttagaaaaat aaacaaatag gggttccgcg cacatttccc cgaaaagtgc cacctgacgt     7740
   ctaagaaacc attattatca tgacattaac ctataaaaat aggcgtatca cgaggccctt     7800
   tcgtctcgcg cgtttcggtg atgacggtga aaacctctga cacatgcagc tcccggagac     7860
   ggtcacagct tgtctgtaag cggatgccgg gagcagacaa gcccgtcagg gcgcgtcagc     7920
   gggtgttggc gggtgtcggg gctggcttaa ctatgcggca tcagagcaga ttgtactgag     7980
   agtgcaccat aaaattgtaa acgttaatat tttgttaaaa ttcgcgttaa atttttgtta     8040
   aatcagctca ttttttaacc aataggccga aatcggcaaa atcccttata aatcaaaaga     8100
   atagcccgag atagggttga gtgttgttcc agtttggaac aagagtccac tattaaagaa     8160
   cgtggactcc aacgtcaaag ggcgaaaaac cgtctatcag ggcgatggcc cactacgtga     8220
   accatcaccc aaatcaagtt ttttggggtc gaggtgccgt aaagcactaa atcggaaccc     8280
   taaagggagc ccccgattta gagcttgacg gggaaagccg gcgaacgtgg cgagaaagga     8340
   agggaagaaa gcgaaaggag cgggcgctag ggcgctggca agtgtagcgg tcacgctgcg     8400
   cgtaaccacc acacccgccg cgcttaatgc gccgctacag ggcgcgtact atggttgctt     8460
   tgacgtatgc ggtgtgaaat accgcacaga tgcgtaagga gaaaataccg catcaggcgc     8520
   cattcgccat tcaggctgcg caactgttgg gaagggcgat cggtgcgggc ctcttcgcta     8580
   ttacgccagc tggcgaaagg gggatgtgct gcaaggcgat taagttgggt aacgccaggg     8640
   ttttcccagt cacgacgttg taaaacgacg gccagtgcca agcttactag atgcatgctc     8700
   gagcggccgc cagtgtgatg gatatctgca gaattcgccc ttattcgccc ttgactagtg     8760
   atctatgctc ttcaccgttc agagacgaaa cttttacttc attaggggga gtccaacttt     8820
   tcattctcac tctgtgagaa gctgtgtcac aatacttcag aggcattaaa gctacctcag     8880
   ctctttaaac atgtcaattc tatcaaaatt aggtatgtga ttcaagtagt gaacaatatg 8940
   tggccgttac tcgagtttat aagtgacaac atgctctcaa agcgctcatg gctggcacaa 9000
   gcctggaaag aaccaacaca aagcatactg cagcaaatca gctgaattcg tcaccaatta 9060
   agtgaacatc aacctgaagg cagagtatga ggccagaagc acatctggat cgcagatcat 9120
   ggattgcccc tcttgttgaa gatgagaatc tagaaagatg gcggggtatg agataagagc 9180
   gatggggggg cacatcatct tccaagacaa acaacctttg cagagtcagg caatttttcg 9240
   tataagagca ggaggaggga gtccagtcat ttcatcagcg gtaaaatcac tctagacaat 9300
   cttcaagatg agttctgcct tgggtgactt atagccatca tcatacctag acagaagctt 9360
   gtgggatact aagaccaacg tacaagctcg cactgtacgc tttgacttcc atgtgaaaac 9420
   tcgatacggc gcgcctctaa attttatagc tcaaccactc caatccaacc tctgcatccc 9480
   tctcactcgt cctgatctac tgttcaaatc agagaataag gacactatcc aaatccaaca 9540
   gaatggctac cacctcccag ctgcctgcct acaagcagga cttcctcaaa tccgccatcg 9600
   acggcggcgt cctcaagttt ggcagcttcg agctcaagtc caagcggata tccccctact 9660
   tcttcaacgc gggcgaattc cacacggcgc gcctcgccgg cgccatcgcc tccgcctttg 9720
   caaagaccat catcgaggcc caggagaagg ccggcctaga gttcgacatc gtcttcggcc 9780
   cggcctacaa gggcatcccg ctgtgctccg ccatcaccat caagctcggc gagctggcgc 9840
   cccagaacct ggaccgcgtc tcctactcgt ttgaccgcaa ggaggccaag gaccacggcg 9900
   agggcggcaa catcgtcggc gcttcgctca agggcaagag ggtcctgatt gtcgacgacg 9960
   tcatcaccgc cggcaccgcc aagagggacg ccattgagaa gatcaccaag gagggcggca 10020
   tcgtcgccgg catcgtcgtg gccctggacc gcatggagaa gctccccgct gcggatggcg 10080
   acgactccaa gcctggaccg agtgccattg gcgagctgag gaaggagtac ggcatcccca 10140
   tctttgccat cctcactctg gatgacatta tcgatggcat gaagggcttt gctacccctg 10200
   aggatatcaa gaacacggag gattaccgtg ccaagtacaa ggcgactgac tgattgaggc 10260
   gttcaatgtc agaagggaga gaaagactga aaaggtggaa agaagaggca aattgttgtt 10320
   attattatta ttctatctcg aatcttctag atcttgtcgt aaataaacaa gcgtaactag 10380
   ctagcctccg tacaactgct tgaatttgat acccgtatgg agggcagtta ttttattttg 10440
   tttttcaaga ttttccattc gccgttgaac tcgtctcaca tcgcgtgtat tgcccggttg 10500
   cccatgtgtt ctcctactac cccaagtccc tcacgggttg tctcactttc tttctccttt 10560
   atcctcccta ttttttttca agtcagcgac agagcagtca tatggggata cgtgcaactg 10620
   ggactcacaa caggccatct tatggcctaa tagccggcgt tggcaattgc ttgctttcgc 10680
   cagtctgttg atactctcgt gagccactcc gctcacgata tcccttcatt gtgcagcttc 10740
   cttttccctt ccaattcctg ctgcttccag actatgcccc gggctgcttt gttgatcgcc 10800
   ctctcgccct tccataccat ttttctagtt gatcattaat accattacgc tccatgttta 10860
   atgatcatat tcctgatgca aacagcgccg tgaacgtttt cttcacatag acatgcgtct 10920
   gccgtgtccc aaggtataca aagagccgcc aaagaaacaa acaaagaagg tcgacatgcg 10980
   tagcacacat cgtatcagtc aaatggtacc gggaggaagg ctggaaagct tacgagaaaa 11040
   gagttggact ttgagtgtga gtggaaatgt gtaacggtat tgactaaaag ggatccatat 11100
   gtttattgca gccagcatag tattaccaga aagagcctca ctgacggctc tagtagtatt 11160
   cgaacagata ttattgtgac cagctctgaa cgatatgctc cctaatctgg tagacaagca 11220
   ctgatctacc ccttggaacg cagcatctag gctctggctg tgctctaacc ctaactagac 11280
   gattgatcgc agaccatcca atactgaaaa gtctctatca gaggaaatcc ccaacattgt 11340
   agtagtcagg ttcctttgtg gctgggagag aattggttcg ctccactgat tccagttgag 11400
   aaagtgggct agaaaaaagt cttgaagatt ggagttgggc tgtggttatc tagtacttct 11460
   cgagctctgt acatgtccgg tcgcgacgta cgcgtatcga tggcgccagc tgcaggcggc 11520
   cgcctgcagc cacttgcagt cccgtggaat tctcacggtg aatgtaggcc ttttgtaggg 11580
   taggaattgt cactcaagca cccccaacct ccattacgcc tcccccatag agttcccaat 11640
   cagtgagtca tggcactgtt ctcaaataga ttggggagaa gttgacttcc gcccagagct 11700
   gaaggtcgca caaccgcatg atatagggtc ggcaacggca aaaaagcacg tggctcaccg 11760
   aaaagcaaga tgtttgcgat ctaacatcca ggaacctgga tacatccatc atcacgcacg 11820
   accactttga tctgctggta aactcgtatt cgccctaaac cgaagtgcgt ggtaaatcta 11880
   cacgtgggcc cctttcggta tactgcgtgt gtcttctcta ggtgccattc ttttcccttc 11940
   ctctagtgtt gaattgtttg tgttggagtc cgagctgtaa ctacctctga atctctggag 12000
   aatggtggac taacgactac cgtgcacctg catcatgtat ataatagtga tcctgagaag 12060
   gggggtttgg agcaatgtgg gactttgatg gtcatcaaac aaagaacgaa gacgcctctt 12120
   ttgcaaagtt ttgtttcggc tacggtgaag aactggatac ttgttgtgtc ttctgtgtat 12180
   ttttgtggca acaagaggcc agagacaatc tattcaaaca ccaagcttgc tcttttgagc 12240
   tacaagaacc tgtggggtat atatctagag ttgtgaagtc ggtaatcccg ctgtatagta 12300
   atacgagtcg catctaaata ctccgaagct gctgcgaacc cggagaatcg agatgtgctg 12360
   gaaagcttct agcgagcggc taaattagca tgaaaggcta tgagaaattc tggagacggc 12420
   ttgttgaatc atggcgttcc attcttcgac aagcaaagcg ttccgtcgca gtagcaggca 12480
   ctcattcccg aaaaaactcg gagattccta agtagcgatg gaaccggaat aatataatag 12540
   gcaatacatt gagttgcctc gacggttgca atgcaggggt actgagcttg gacataactg 12600
   ttccgtaccc cacctcttct caacctttgg cgtttccctg attcagcgta cccgtacaag 12660
   tcgtaatcac tattaaccca gactgaccgg acgtgttttg cccttcattt ggagaaataa 12720
   tgtcattgcg atgtgtaatt tgcctgcttg accgactggg gctgttcgaa gcccgaatgt 12780
   aggattgtta tccgaactct gctcgtagag gcatgttgtg aatctgtgtc gggcaggaca 12840
   cgcctcgaag gttcacggca agggaaacca ccgatagcag tgtctagtag caacctgtaa 12900
   agccgcaatg cagcatcact ggaaaataca aaccaatggc taaaagtaca taagttaatg 12960
   cctaaagaag tcatatacca gcggctaata attgtacaat caagtggcta aacgtaccgt 13020
   aatttgccaa cggcttgtgg ggttgcagaa gcaacggcaa agccccactt ccccacgttt 13080
   gtttcttcac tcagtccaat ctcagctggt gatcccccaa ttgggtcgct tgtttgttcc 13140
   ggtgaagtga aagaagacag aggtaagaat gtctgactcg gagcgttttg catacaacca 13200
   agggcagtga tggaagacag tgaaatgttg acattcaagg agtatttagc cagggatgct 13260
   tgagtgtatc gtgtaaggag gtttgtctgc cgatacgacg aatactgtat agtcacttct 13320
   gatgaagtgg tccatattga aatgtaagtc ggcactgaac aggcaaaaga ttgagttgaa 13380
   actgcctaag atctcgggcc ctcgggcctt cggcctttgg gtgtacatgt ttgtgctccg 13440
   ggcaaatgca aagtgtggta ggatcgaaca cactgctgcc tttaccaagc agctgagggt 13500
   atgtgatagg caaatgttca ggggccactg catggtttcg aatagaaaga gaagcttagc 13560
   caagaacaat agccgataaa gatagcctca ttaaacggaa tgagctagta ggcaaagtca 13620
   gcgaatgtgt atatataaag gttcgaggtc cgtgcctccc tcatgctctc cccatctact 13680
   catcaactca gatcctccag gagacttgta caccatcttt tgaggcacag aaacccaata 13740
   gtcaaccatc acaagtttgt acaaaaaagc aggctgaatt ccaccatgag gtcacagact 13800
   ctggctgttg ccctcttggc ggcagctgac caggttgcag ctgctgtaac cacagaacga 13860
   cagctacaca aggtacgtta ctcggtctgt ctgttgttct ctgtggcgcc gtcgttggcg 13920
   actcgccagc cttgcccttc ttggaaatcc aagacatccc cattcccttc gggcctgtgt 13980
   gtggcgagca ggcccgccgc ggtgttatat cccgattttg acgtcttccc tctctatttc 14040
   gtatcgcatt ggcgtctcgt ctcgtctctc tcctttagca acacagagaa cccggttccg 14100
   cagtcatgcg tttcctttca ctcgcaggct cggccgtcgg cctgtcacaa ctctcgagcc 14160
   gctcagatga cttacaatgt ttacagcgcg acctcgcata ctcgccgccc gtctatccgt 14220
   caccatggat ggatcccaat gccgacggct ggactgatgc ttacgccaag gccaaggact 14280
   ttgtctccca gctcacgctt ctcgagaagg ttaatttgac caccggagtc gggtgagtga 14340
   attcgcatca actacgatgg gaacacatgc tgataggaat atacagttgg caaggagacc 14400
   tgtgcgtcgg caacgttggt tccgtccccc gtttgggact ccgcggcctt tgcatgcagg 14460
   acggccctgt cggcatccgc ttctccgact acaactcggt cttcccctcc ggccagaccg 14520
   ccgccgcgac ttgggaccga gagttgatct accgcagagc cgaagctatc gggttcgagc 14580
   accgcgccaa gggcgtcgac gttgtcctgg ccccggttgc cggacctatt ggtcgtgcac 14640
   cagccggcgg ccgcaactgg gaaggctttt cgtccgatcc ttacctgact ggcgttgcca 14700
   tggccgagtc agtcaaggga atccagcagc acgccatcgc ctgcgccaag catttcatcg 14760
   gcaacgaaca ggagcacttc agacaagctc cagaggcaat tggctataac tacaccatag 14820
   acgagtccat cagctcgaat attgatgaca aaactctcca cgaattatat ttgtggccat 14880
   tccaggacgc agtggctgct ggggtaggtt ccttcatgtg ctcatacaat caggtgaaca 14940
   actcgtatgg ctgccagaac tccaagctca tgaacggcat cttgaaagac gagcttggtt 15000
   tccagggatt catcatgtct gattggtcag tacttccttc cattctcttt agcccctcct 15060
   acaagcactt cttcaacctc cgttccatat ctactcagct ccatcccctc tttcatactc 15120
   tctttccttc catcagatgc cgacttcagt agctaacttt cttacagggc agcgcagcat 15180
   gtacgtatcc ccggttttgt tccatttcat atgatgatgc ccacccgcct tcctttccca 15240
   tcaatgactt atgactgaca atcgaacagg ctggagttgc tactgcggtt gccggtctcg 15300
   atatggccat gcctggtgac acagcgttca actctggcat gacgttctgg ggaaccaatc 15360
   taaccgtggc cgtcctgaac ggcacgctcc ccgagtaccg gctcgatgac atggcgatgc 15420
   gcatcatggc cgcattcttt aaggtcggct tcgaactgaa tgaggtaccc gagatcaact 15480
   tttcctcgtg gacgacggac accgttggtc cgctacagta ctacgccaag gagaacgttc 15540
   aagtcatcaa ccagcatgtc gacgttcgaa gaggtcagga acacggtaaa ctcattcgtg 15600
   agatcgccgc caaggccact gtgctgctga agaatgaggg cgctctcccg ctgaagaagc 15660
   ccaagttcct ggctgttatc ggagaggatg ccggccccaa cctcagtggg cccaacgggt 15720
   gctccgacca tggctgtaac gaaggaacgc tcggcgctgg ctggggatcc ggaacctcga 15780
   actaccccta cctcatcaca ccggaccagg cactgcaagc tcgggctgtt gcggagggtt 15840
   ctcggtatga gagcattctc agcaactatg acttcgccgc caccacggcc ctggttacgc 15900
   aaccagacgc cacggccatc gtcttcgtca atgcggatag cggtgaaggc tacatcgatg 15960
   tgggtgggaa cgaaggcgac cgtcagaact tgacgctttg gaatggggga gacgagctgg 16020
   tcaagaacgt ggctgcaggc aacaacaaca ctattgtcgt gatccactcc gtcggccctg 16080
   tgttgctggc tgacatgaag aacaacccca atatcacggc gattgtctgg gccggtcttc 16140
   cgggtcagga gtctggtaat tcgatcacgg acgtgctcta cggagacgtt aaccccggag 16200
   gcaaatcccc gttcacttgg ggtcccacgc gcgagagtta tggcaccgat gttctgtacg 16260
   agcccaacaa tggtgagggt gctcctcaag acgactttag cgagggtgtc ttcatcgact 16320
   accgctactt cgaccgggcg acctcgggtt ccaacgagac ctccactggc gcagcccccg 16380
   tctacccatt cggatttggt ctctcgtaca cgacgtttga atactccaac ttggtcgtaa 16440
   cccccaagga ggcaggcgag tacacgccca ccactggcgt gacggagaag gcgccgacct    16500
   ttggcaacta cagcaccgac ccggcggcct acgttttccc gagcggagag ttccgttaca    16560
   tctacaactt catctacccc tacctcaaca ctaccgacat tagcaagtcg gccaacgacc    16620
   ccgcgtacgg acagacggcg gacgagtttc tgcctcccaa ggctctggag agcggtcccc    16680
   agcccaagca cccagcatcg ggcgcccctg gcggcaaccc tcaactttgg gacgtcctgt    16740
   acactgtgac tgccacgatc actaacaagg gcgacgttgc cggcgacgag gttgcccagt    16800
   tgtacgtctc gctcggcggt ccgaatgatc cggtcaaggt gctgcgtggg ttcgaccgca    16860
   ttggtatcgc gcctggcgag tcggccactt tcacggcgga catcaccaga cgcgacctca    16920
   gcaactggga cacggtgagc caaaactggg tcatcagcaa gtacccgaag aaggtgtggg    16980
   tgggaggctc gtcgagggag ttgcctctga gcgcgtcgct ctaagcggcc gcacccagct    17040
   ttc                                                                   17043
<210> 43
   <211> 87
   <212> DNA
   <213> Zymomonas mobilis
<400> 43
   atgataaaag tcccgcggtt catctgtatg atcgcgctta catccagcgt tctggcaagc       60
   ggcctttctc aaagcgtttc agctcat                                           87
<210> 44
   <211> 90
   <212> DNA
   <213> Zymomonas mobilis
<400> 44
   atgaaaagaa agcttggtcg tcgccagtta ttaactggct ttgttgccct tggcggtatg       60
   gcgattacag ctggtaaggc gcaggcttct                                        90
<210> 45
   <211> 19
   <212> PRT
   <213> Glomerella graminicola
<400> 45
   Met Arg Ser Gln Thr Leu Ala Val Ala Leu Leu Ala Ala Ala Asp Gln
   1               5                   10                  15
   Val Ala Ala

## Description

### TECHNICAL FIELD

The present compositions and methods relate to a beta-glucosidase polypeptide obtainable from *Glomerella graminicola,* polynucleotides encoding the beta-glucosidase polypeptide, and methods of making and using thereof. Formulations and compositions comprising the beta-glucosidase polypeptide may be useful for degrading or hydrolyzing lignocellulosic biomass, for example.

### BACKGROUND

Cellulose and hemicellulose are the most abundant plant materials produced by photosynthesis. They can be degraded and used as an energy source by numerous microorganisms (e.g., bacteria, yeast and fungi) that produce extracellular enzymes capable of hydrolysis of the polymeric substrates to monomeric sugars (Aro et al., J. Biol. Chem., 276: 24309-24314, 2001). As the limits of non-renewable resources approach, the potential of cellulose to become a major renewable energy resource is enormous (Krishna et al., Bioresource Tech., 77: 193-196, 2001). The effective utilization of cellulose through biological processes is one approach to overcoming the shortage of foods, feeds, and fuels (Ohmiya et al., Biotechnol. Gen. Engineer Rev., 14: 365-414, 1997).

Cellulases are enzymes that hydrolyze cellulose (comprising beta-1,4-glucan or beta D-glucosidic linkages) resulting in the formation of glucose, cellobiose, cellooligosaccharides, and the like. Cellulases have been traditionally divided into three major classes: endoglucanases (EC 3.2.1.4) ("EG"), exoglucanases or cellobiohydrolases (EC 3.2.1.91) ("CBH") and beta-glucosidases ([beta]-D-glucoside glucohydrolase; EC 3.2.1.21) ("BG") (Knowles et al., TIBTECH 5: 255-261, 1987; and Schulein, Methods Enzymol., 160: 234-243, 1988). Endoglucanases act mainly on the amorphous parts of the cellulose fiber, whereas cellobiohydrolases are also able to degrade crystalline cellulose (Nevalainen and Penttila, Mycota, 303-319, 1995). Thus, the presence of a cellobiohydrolase in a cellulase system is required for efficient solubilization of crystalline cellulose (Suurnakki et al., Cellulose, 7: 189-209, 2000). Beta-glucosidase acts to liberate D-glucose units from cellobiose, cellooligosaccharides, and other glucosides (Freer, J. Biol. Chem., 268: 9337-9342, 1993).

Cellulases are known to be produced by a large number of bacteria, yeast and fungi. Certain fungi produce a complete cellulase system capable of degrading crystalline forms of cellulose. These fungi can be fermented to produce suites of cellulases or cellulase mixtures. The same fungi and other fungi can also be engineered to produce or overproduce certain cellulases, resulting in mixtures of cellulases that comprise different types or proportions of cellulases. The fungi can also be engineered such that they produce in large quantities *via* fermentation the various cellulases. Filamentous fungi play a special role since many yeast, such as *Saccharomyces cerevisiae,* lack the ability to hydrolyze cellulose in their native state *(see, e.g.,* Wood et al., Methods in Enzymology, 160: 87-116, 1988).

The fungal cellulase classifications of CBH, EG and BG can be further expanded to include multiple components within each classification. For example, multiple CBHs, EGs and BGs have been isolated from a variety of fungal sources including *Trichoderma reesei* (also referred to as *Hypocrea jecorina*), which contains known genes for two CBHs, *i.e.,* CBH I ("CBH1") and CBH II ("CBH2"), at least eight EGs, *i.e.,* EG I, EG II, EG III, EGIV, EGV, EGVI, EGVII and EGVIII, and at least five BGs, *i.e.,* BG1, BG2, BG3, BG4, BG5 and BG7 (Foreman et al. (2003), J. Biol. Chem. 278(34):31988-31997). EGIV, EGVI and EGVIII also have xyloglucanase activity.

In order to efficiently convert crystalline cellulose to glucose the complete cellulase system comprising components from each of the CBH, EG and BG classifications is required, with isolated components less effective in hydrolyzing crystalline cellulose (Filho et al., Can. J. Microbiol., 42:1-5, 1996). Endo-1,4-beta-glucanases (EG) and exo-cellobiohydrolases (CBH) catalyze the hydrolysis of cellulose to cellooligosaccharides (cellobiose as a main product), while beta-glucosidases (BGL) convert the oligosaccharides to glucose. A synergistic relationship has been observed between cellulase components from different classifications. In particular, the EG-type cellulases and CBH-type cellulases synergistically interact to efficiently degrade cellulose. The beta-glucosidases serves the important role of liberating glucose from the cellooligosaccharides such as cellobiose, which is toxic to the microorganisms, such as, for example, yeasts, that are used to ferment the sugars into ethanol; and which is also inhibitory to the activities of endoglucanases and cellobiohydrolases, rendering them ineffective as further hydrolyzing the crystalline cellulose.

In view of the important role played by beta-glucosidases in the degradation or conversion of cellulosic materials, discovery, characterization, preparation, and application of beta-glucosidase homologs with improved efficacy or capability to hydrolyze cellulosic feedstock is desirable and advantageous.

Certain sequences related to those described herein were deposited in online databases prior to the presently claimed priority date:
UniProt 1-16 11 January 2011. "SubName: Full=Glycosyl hydrolase family 3 N terminal domain-containing protein (EBI accession no. UNI PROT: E3QPM6).
EMBL 20 March 2008 "C. graminicola genomic sequence Glomerella graminicola genomic, genomic survey sequence." (EBI accession no. EM GSS:ET519535).
UniProt 26 June 2013 "RecName: Full=Beta-glucosidase (EBI accession no. UNIPROT: N4VJC4)
UniProt 6 March 2013 "RecName: Full-Beta-glucosidase (EBI accession no. UNIPROT: L2G5M9).

### SUMMARY

### Beta-glucosidase obtainable from Glomerella graminicola and their use

Enzymatic hydrolysis of cellulose remains one of the main limiting steps of the biological production from lignocellulosic biomass feedstock of a material, which may be cellulosic sugars and/or downstream products. Beta-glucosidases play the important role of catalyzing the last step of that process, releasing glucose from the inhibitory cellobiose, and therefore its activity and efficacy directly contributes to the overall efficacy of enzymatic lignocellulosic biomass conversion, and consequently to the cost in use of the enzyme solution. Accordingly there is great interest in finding, making and using new and more effective beta-glucosidases.

While a number of beta-glucosidases are known, including the beta-glucosidases Bgll, Bgl3, Bgl5, Bgl7, etc, from *Trichoderma reesei* or *Hypocrea jecorina* (Korotkova O.G. et al., Biochemistry 74:569-577 (2009); Chauve, M. et al., Biotechnol. Biofuels 3:3-3 (2010)), the beta-glucosidases from *Humicola grisea var. thermoidea* (Nascimento, C.V. et al., J. Microbiol. 48, 53-62 (2010)); from *Sporotrichum pulverulentum,* Deshpande V. et al., Methods Enzymol., 160:415-424 (1988)); of *Aspergillus oryzae* (Fukuda T. et al, Appl. Microbiol. Biotechnol. 76:1027-1033 (2007), from *Talaromyces thermophilus* CBS 236.58 (Nakkharat P. et al., J. Biotechnol., 123:304-313 (2006)), from *Talaromyces emersonii* (Murray P., et al, Protein Expr. Purif. 38:248-257 (2004)), so far the *Trichoderma reesei* beta-glucosidase Bgll and the

*Aspergillus niger* beta-glucosidase SP188 are deemed benchmark beta-glucosidases against which the activities and performance of other beta-glucosidases are evaluated. It has been reported that *Trichoderma reesei* Bgl1 has higher specific activity than *Aspergillus niger* beta-glucosidase SP188, but the former can be poorly secreted, while the latter is more sensitive to glucose inhibition (Chauve, M. et al., Biotechnol. Biofuels, 3(1):3 (2010)).

The invention relates to a method for hydrolyzing a lignocellulosic biomass substrate, or to a saccharification process, according to the appended claims.

One aspect of the present compositions and methods is the application or use of a highly active beta-glucosidase isolated from the fungal species *Glomerella graminicola*,(anamorph *Colletotrichum graminicola)* to hydrolyze a lignocellulosic biomass substrate. The genome of *Glomerella graminicola,* the causative agent of anthracnose stalk rot and leaf blight of maize, was sequenced by the *Colletotrichum* Sequencing Project, Broad Institute of Harvard and MIT (http://www.broadinstitute.org/). The herein described sequence of SEQ ID NO:2 was published by National Center for Biotechnology Information, U.S. National Library of Medicine (NCBI) with the Accession No. EFQ32803.1, and designated to be a GH3 family beta-glucosidase.

The enzyme has not been previously made in recombinant forms, or included in an enzyme composition useful for hydrolyzing a lignocellulosic biomass substrate. Nor has it or a composition comprising such an enzyme been applied to a lignocellulosic biomass substrate in a suitable method of enzymatic hydrolysis of such a substrate. Furthermore, the beta-glucosidase of *Glomerella graminicola* has not previously been expressed by an engineered microorganism. Nor has it been co-expressed with one or more cellulase genes and/or one or more hemicellulase genes. Expression in suitable microorganisms, which have, through many years of development, become highly effective and efficient producers of heterologous proteins and enzymes, with the aid of an arsenal of genetic tools, makes it possible to express these useful beta-glucosidases in substantially larger amounts than when they are expressed endogenously in an unengineered microorganism, or when they are expressed in plants.

Enzymes classified as beta-glucosidases are diverse not only in their origins but also in their activities on lignocellulosic substrates, although most if not all beta-glucosidases can catalyze cellobiose hydrolysis under suitable conditions. For example, some are active on not only cellobiose but also on longer-chain oligosaccharides, whereas others are more exclusively active only on cellobiose. Even for those beta-glucosidases that have similar substrate preferences, some have enzyme kinetics profiles that make them more catalytically active and efficient, and accordingly more useful in industrial applications where the enzymatically catalyzed hydrolysis cannot afford to take longer than a few days at most.

Furthermore, no fermenting or ethanologen microorganism capable of converting cellulosic sugars obtained from enzymatic hydrolysis of lignocellulosic biomass has been engineered to express a beta-glucosidase from *Glomerella graminicola,* such as a Ggr3A polypeptide herein. Expression of beta-glucosidases in ethanologen microorganisms provides an important opportunity to further liberating D-glucose from the remaining cellobiose that are not completely converted by the enzyme saccharification, where the D-glucose thus produced can be immediately consumed or fermented just in time by the ethanologen.

An aspect of the present composition and methods pertains to a beta-glucosidase polypeptide of glycosyl hydrolase family 3 derived from *Glomerella graminicola,,* referred to herein as "Ggr3A" or "Ggr3A polypeptides," nucleic acids encoding the same, compositions comprising the same, and methods of producing and applying the beta-glucosidase polypeptides and compositions comprising thereof in hydrolyzing or converting lignocellulosic biomass into soluble, fermentable sugars. Such fermentable sugars can then be converted into cellulosic ethanol, fuels, and other biochemicals and useful products. In certain embodiments, the Ggr3A beta-glucosidase polypeptides have higher beta-gluclosidase activity and/or exhibits an increased capacity to hydrolyze a given lignocellulosic biomass substrate as compared to the benchmark *Trichderma reesei* Bgl1, which is a known, high fidelity beta-glucosidase. (Chauve, M. et al., Biotechnol. Biofuels, 3(1):3 (2010)).

In some embodiments, a Ggr3A polypeptide is applied together with, or in the presence of, one or more other cellulases in an enzyme composition to hydrolyze or breakdown a suitable biomass substrate. The one or more other cellulases may be, for example, other beta-glucosidases, cellobiohydrolases, and/or endoglucanases. For example, the enzyme composition may comprise a Ggr3A polypeptide, a cellobiohydrolase, and an endoglucanase. In some embodiments, the Ggr3A polypeptide is applied together with, or in the presence of, one or more hemicellulases in an enzyme composition. The one or more hemicellulases may be, for example, xylanases, beta-xylosidases, and/or L-arabinofuranosidases. In further embodiments, the Ggr3A polypeptide is applied together with, or in the presence of, one or more cellulases and one or more hemicellulases in an enzyme composition. For example, the enzyme composition comprises a Ggr3A polypeptide, no or one or two other beta-glucosidases, one or more cellobiohydrolases, one or more endoglucanases; optionally no or one or more xylanases, no or one or more beta-xylosidases, and no or one or more L-arabinofuranosidases.

In certain embodiments, a Ggr3A polypeptide, or a composition comprising the Ggr3A polypeptide is applied to a lignocellulosic biomass substrate or a partially hydrolyzed lignocellulosic biomass substrate in the presence of an ethanologen microbe, which is capable of metabolizing the soluble fermentable sugars produced by the enzymatic hydrolysis of the lignocellulosic biomass substrate, and converting such sugars into ethanol, biochemicals or other useful materials. Such a process may be a strictly sequential process whereby the hydrolysis step occurs before the fermentation step. Such a process may, alternatively, be a hybrid process, whereby the hydrolysis step starts first but for a period overlaps the fermentation step, which starts later. Such a process may, in a further alternative, be a simultaneous hydrolysis and fermentation process, whereby the enzymatic hydrolysis of the biomass substrate occurs while the sugars produced from the enzymatic hydrolysis are fermented by the ethanologen.

The Ggr3A polypeptide, for example, may be a part of an enzyme composition, contributing to the enzymatic hydrolysis process and to the liberation of D-glucose from oligosaccharides such as cellobiose. In certain embodiments, the Ggr3A polypeptide may be genetically engineered to express in an ethanologen, such that the ethanologen microbe expresses and/or secrets such a beta-glucosidase activity. Moreover, the Ggr3A polypeptide may be a part of the hydrolysis enzyme composition while at the same time also expressed and/or secreted by the ethanologen, whereby the soluble fermentable sugars produced by the hydrolysis of the lignocellulosic biomass substrate using the hydrolysis enzyme composition is metabolized and/or converted into ethanol by an ethanologen microbe that also expresses and/or secrets the Ggr3A polypeptide. The hydrolysis enzyme composition can comprise the Ggr3A polypeptide in addition to one or more other cellulases and/or one or more hemicellulases. The ethanologen can be engineered such that it expresses the Ggr3A polypeptide, one or more other cellulases, one or more other hemicellulases, or a combination of these enzymes. One or more of the beta-glucosidases may be in the hydrolysis enzyme composition and expressed and/or secreted by the ethanologen. For example, the hydrolysis of the lignocellulosic biomass substrate may be achieved using an enzyme composition comprising a Ggr3A polypeptide, and the sugars produced from the hydrolysis can then be fermented with a microorganism engineered to express and/or secret Ggr3A polypeptide. Alternatively, an enzyme composition comprising a first beta-glucosidase participates in the hydrolysis step and a second beta-glucosidase, which is different from the first beta-glucosidase, is expressed and/or secreted by the ethanologen. For example, the hydrolysis of the lignocellulosic biomass substrate may be achieved using a hydrolysis enzyme composition comprising *Trichoderma reesei* Bgl1, and the fermentable sugars produced from hydrolysis are fermented by an ethanologen microorganism expressing and/or secreting a Ggr3A polypeptide, or *vice versa.*

As demonstrated herein, Ggr3A polypeptides and compositions comprising Ggr3A polypeptides have improved efficacy at conditions under which saccharification and degradation of lignocellulosic biomass take place. The improved efficacy of an enzyme composition comprising a Ggr3A polypeptide is shown when its performance of hydrolyzing a given biomass substrate is compared to that of an otherwise comparable enzyme composition comprising Bgl1 of *Trichoderma reesei.*

In certain embodiments, the improved or increased beta-glucosidase activity is reflected in an improved or increased cellobiase activity of the Ggr3A polypeptides, which is measured using cellobiose as substrate, for example, at a temperature of about 30°C to about 65°C (e.g., about 35°C to about 60°C, about 40°C to about 55°C, about 45°C to about 55°C, about 48°C to about 52°C, about 40°C, about 45°C, about 50°C, about 55°C, etc). In some embodiments, the improved beta-glucosidase activity of a Ggr3A polypeptide as compared to that of *Trichoderma reesei* Bgll, is observed when the beta-glucosidase polypeptides are used to hydrolyze a phosphoric acid swollen cellulose (PASC), for example, a thus pretreated Avicel pretreated using an adapted protocol of Walseth, TAPPI 1971, 35:228 and Wood, Biochem. J. 1971, 121:353-362. In some embodiments, the improved beta-glucosidase activity of a Ggr3A polypeptide as compared to that of *Trichoderma reesei* Bgl1, is observed when the beta-glucosidase polypeptides are used to hydrolyze a dilute ammonia pretreated corn stover, for example, one described in International Published Patent Applications: WO2006110891, WO2006110899, WO2006110900, WO2006110901, and WO2006110902; US Patent Nos. 7,998,713, 7,932,063.

In some aspects, a Ggr3A polypeptide and/or as it is applied in an enzyme composition or in a method to hydrolyze a lignocellulosic biomass substrate is (a) derived from, obtainable from, or produced by *Glomerella graminicola;* (b) a recombinant polypeptide comprising an amino acid sequence that is at least 90%, e.g., at least 91%, 92%, 93%, 94%, 95%, 96%, 97%, 98%, 99%, or 100%) identical to the amino acid sequence of SEQ ID NO:2; (c) a recombinant polypeptide comprising an amino acid sequence that is at least 90%, e.g., at least 91%, 92%, 93%, 94%, 95%, 96%, 97%, 98%,
99%, or 100%) identical to the mature form of amino acid sequence of SEQ ID NO:3, namely amino acid residues 20-876 of SEQ ID NO:2. In certain embodiments, it is provided a variant polypeptide having beta-glucosidase activity, which comprises a substitution, a deletion and/or an insertion of one or more amino acid residues of SEQ ID NO:2 or SEQ ID NO:3.

In some aspects, a Ggr3A polypeptide and/or as it is applied in an enzyme composition or in a method to hydrolyze a lignocellulosic biomass substrate is (a) a polypeptide encoded by a nucleic acid sequence that is at least 75% (e.g., at least 75%, 80%, 85%, 90%, 91%, 92%, 93%, 94%, 95%, 96%, 97%, 98%, 99% or 100%) sequence identity to SEQ ID NO:1, or (b) one that hybridizes under medium stringency conditions, high stringency conditions or very high stringency conditions to SEQ ID NO:1 or to a subsequence of SEQ ID NO:1 of at least 100 contiguous nucleotides, or to the complementary sequence thereof, wherein the polypeptide has beta-glucosidase activity. In some embodiments, a Ggr3A polypeptide and/or as it is applied in a composition or in a method to hydrolyze a lignocellulosic biomass substrate is one that, due to the degeneracy of the genetic code, does not hybridize under medium stringency conditions, high stringency conditions or very high stringency conditions to SEQ ID NO:1 or to a subsequence of SEQ ID NO:1 of at least 100 contiguous nucleotide, but nevertheless encodes a polypeptide having beta-glucosidase activity and comprising an amino acid sequence that is at least 75% (e.g., at least 75%, 80%, 85%, 90%, 91%, 92%, 93%, 94%, 95%, 96%, 97%, 98%, 99% or 100%) identical to that of SEQ ID NO:2 or to the mature beta-glucosidase sequence of SEQ ID NO:3. The nucleic acid sequences can be synthetic, and is not necessarily derived from *Glomerella graminicola,* but the nucleic acid sequence encodes a polypeptide having beta-glucosidase activity and comprises an amino acid sequence that is least 75% identical to SEQ ID NO:2 or to SEQ ID NO:3.

The Ggr3A polypeptide or the composition comprising the Ggr3A polypeptide has improved beta-glucosidase activity, as compared to that of the wild type *Trichoderma reesei* Bgl1 (of SEQ ID NO:4), or the enzyme composition comprising the *Trichoderma reesei* Bgll. For example, the beta-glucosidase activity of the Ggr3A polypeptide of the compositions and methods herein, as measured using a cellobiose hydrolysis assay, is at least about 10% higher (e.g., at least about 10% higher, at least about 20% higher, at least about 30% higher, at least about 40% higher, at least about 50% higher, at least about 60% higher, at least about 70% higher, at least about 80% higher, at least about 85% higher, such as, for example at least about 87% higher) than that of the *Trichoderma reesei* Bgl1. The cellobiose hydrolysis assay is described in Example 3 herein.

In some embodiments, the Ggr3A polypeptides of the compositions and methods herein have substantially increased (e.g., at least about 10% higher, at least about 20% higher, at least about 30% higher, at least about 40% higher, at least about 50% higher, at least about 60% higher, at least about 70% higher, at least about 80% higher, at least about 85% higher, such as, for example at least about 87% higher) cellobiose hydrolysis activity.

In certain aspects, the Ggr3A polypeptides and the compositions comprising the *Ggr3A* polypeptides of the invention have improved performance hydrolyzing lignocellulosic biomass substrates, as compared to that of the wild type *Trichoderma reesei* Bgll (of SEQ ID NO:4). In some embodiments, the improved hydrolysis performance of *Ggr3A* polypeptides or compositions comprising *Ggr3A* polypeptides is observable by the production of a greater amount of glucose from a given lignocellulosic biomass substrate, pretreated in a certain way, as compared to the level of glucose produced by *Trichoderma reesei* Bgl1 or an identical enzyme composition comprising *Trichoderma reesei* Bgll from the same biomass pretreated the same way, under the same saccharification conditions. For example, the amount of glucose produced by the *Ggr3A* polypeptides or by the enzyme compositions comprising the *Ggr3A* polypeptides is at least about 5% (e.g., at least about 5%, at least about 10%, at least about 15%, at least about 20%, or at least about 25%) greater than the amount of glucose produced by the *Trichoderma reesei* Bgl1 or an otherwise identical enzyme composition comprising the *Trichoderma reesei* Bgl1 (rather than a *Ggr3A* polypeptide), when 0-10 mg (e.g., about 1 mg, about 2 mg, about 3 mg, about 4 mg, about 5 mg, about 6 mg, about 7 mg, about 8 mg, about 9 mg, about 10 mg) of beta-glucosidase (a *Ggr3A* polypeptide or *Trichoderma reesei* Bgll) is used to hydrolyze 1 g glucan in the biomass substrate.

In some aspects, the improved hydrolysis performance of *Ggr3A* polypeptides or compositions comprising *Ggr3A* polypeptides is observable by increased % glucan conversion from a given lignocellulosic biomass substrate pretreated in a certain way, as compared to the level of % glucan conversion by *Trichoderma reesei* Bgll or an otherwise identical enzyme composition comprising *Trichoderma reesei* Bgl1 from the same biomass pretreated the same way, under the same saccharification conditions. For example, the %glucan conversion by the *Ggr3A* polypeptides or the enzyme compositions comprising the *Ggr3A* polypeptides is at least about 5% (e.g., at least about 5%, at least about 10%, or at least about 15%) higher than the %glucan conversion by *Trichoderma reesei* Bgl1 or an otherwise identical enzyme composition comprising *Trichoderma reesei* Bgl1 (rather than a *Ggr3A* polypeptide), when 0-10 mg (e.g., about 1 mg, about 2 mg, about 3 mg, about 4 mg, about 5 mg, about 6 mg, about 7 mg, about 8 mg, about 9 mg, about 10 mg) of beta-glucosidase (a *Ggr3A* polypeptide or *Trichoderma reesei* Bgl1) is used to hydrolyze 1 g glucan in the biomass substrate.

In further aspects, the improved hydrolysis performance of *Ggr3A* polypeptides and compositions comprising *Ggr3A* polypeptides is observable by a higher cellobiase activity and/or reduced amount of residual cellobiose in the product mixture, from hydrolyzing a given lignocellulosic biomass substrate pretreated in a certain way, as compared to the residual amount of cellobiose when the same biomass substrate is hydrolyzed by *Trichoderma reesei* Bgl1 or an otherwise identical composition comprising *Trichoderma reesei* Bgl1 under the same saccharification conditions. For example, the amount of residual cellobiose in the product mixture produced from the hydrolysis of a given biomass substrate pretreated a certain way, by the *Ggr3A* polypeptides or the compositions comprising the *Ggr3A* polypeptides is at least about 5% (e.g., at least about 5%, at least about 10%, at least about 15%, or even at least about 20%) less than the amount of residual cellobiose produced in the product mixture produced from hydrolysis of the same biomass substrate pretreated the same way by the *Trichoderma reesei* Bgll or by an otherwise identical enzyme composition comprising *Trichoderma reesei* Bgll under the same saccharification conditions. This is the case when 0-10 mg beta-glucosidase (e.g., about 1 mg, about 2 mg, about 3 mg, about 4 mg, about 5 mg, about 6 mg, about 7 mg, about 8 mg, about 9 mg, about 10 mg) of beta-glucosidase (e.g., a *Ggr3A* polypeptide or a *Trichoderma reesei* Bgl1) is used to hydrolyze 1 g glucan in the biomass substrate.

Aspects of the present compositions and methods include a composition comprising a recombinant *Ggr3A* polypeptide as detailed above and a lignocellulosic biomass. Suitable lignocellulosic biomass may be, for example, derived from an agricultural crop, a byproduct of a food or feed production, a lignocellulosic waste product, a plant residue, including, for example, a grass residue, or a waste paper or waste paper product. In certain embodiments, the lignocellulosic biomass has been subject to one or more pretreatment steps in order to render xylan, hemicelluloses, cellulose and/or lignin material more accessible or susceptible to enzymes and thus more amendable to enzymatic hydrolysis. A suitable pretreatment method may be, for example, subjecting biomass material to a catalyst comprising a dilute solution of a strong acid and a metal salt in a reactor. *See, e.g.,* U.S. Patent Nos. 6,660,506, 6,423,145. Alternatively, a suitable pretreatment may be, for example, a multi-stepped process as described in U.S. Patent No. 5,536,325. In certain embodiments, the biomass material may be subject to one or more stages of dilute acid hydrolysis using about 0.4% to about 2% of a strong acid, in accordance with the disclosures of U.S. Patent No. 6,409,841. Further embodiments of pretreatment methods may include those described in, for example, U.S. Patent No. 5,705,369; in Gould, Biotech. & Bioengr., 26:46-52 (1984); in Teixeira et al., Appl. Biochem & Biotech., 77-79:19-34 (1999); in International Published Patent Application WO2004/081185; or in U.S. Patent Publication No. 20070031918, or International Published Patent Application WO06110901.

Disclosed herein are isolated polynucleotides encoding polypeptides having beta-glucosidase activity, wherein the isolated polynucleotides are selected from:
(1) a polynucleotide encoding a polypeptide comprising an amino acid sequence having at least 75% (e.g., at least 75%, 80%, 85%, 90%, 91%, 92%, 93%, 94%, 95%, 96%, 97%, 98%, 99%, or 100%) identity to SEQ ID NO:2 or to SEQ ID NO:3;
(2) a polynucleotide having at least 75% (e.g., at least 75%, 80%, 85%, 90%, 91%, 92%, 93%, 94%, 95%, 96%, 97%, 98%, 99%, or 100%) identity to SEQ ID NO:1, or hybridizes under medium stringency conditions, high stringency conditions, or very high stringency conditions to SEQ ID NO:1, or to a complementary sequence thereof.

Aspects of the present compositions and methods include methods of making or producing a *Ggr3A* polypeptide having beta-glucosidase activity, employing an isolated nucleic acid sequence encoding the recombinant polypeptide comprising an amino acid sequence that is at least 75% identical (e.g., at least 75%, 80%, 85%, 90%, 91%, 92%, 93%, 94%, 95%, 96%, 97%, 98%, 99%, or 100%) to that of SEQ ID NO:2, or that of the mature sequence SEQ ID NO:3. In some embodiments, the polypeptide further comprises a native or non-native signal peptide such that the *Ggr3A* polypeptide that is produced is secreted by a host organism, for example, the signal peptide comprises a sequence that is at least 90% identical to SEQ ID NO:11 (the signal sequence of *Trichoderma reesei* Bgl1). In certain embodiments the isolated nucleic acid comprises a sequence that is at least 75% (e.g., at least 75%, 80%, 85%, 90%, 91%, 92%, 93%, 94%, 95%, 96%, 97%, 98%, 99%, or 100%) identical to SEQ ID NO:1. In certain embodiments, the isolated nucleic acid further comprises a nucleic acid sequence encoding a signal peptide sequence. In certain embodiments, the signal peptide sequence may be one selected from SEQ ID NOs:11-40. In certain particular embodiments, a nucleic acid sequence encoding the signal peptide sequence of SEQ ID NO:11 is used to express a *Ggr3A* polypeptide in *Trichoderma reesei.*

Aspects of the present compositions and methods include an expression vector comprising the isolated nucleic acid as described above in operable combination with a regulatory sequence.

Aspects of the present compositions and methods include a host cell comprising the expression vector. In certain embodiments, the host cell is a bacterial cell or a fungal cell. In certain embodiments, the host cell comprising the expression vector is an ethanologen microbe capable of metabolizing the soluble sugars produced from a hydrolysis of a lignocellulosic biomass, wherein the hydrolysis is the result of a chemical and/or enzymatic process.

Aspects of the present compositions and methods include a composition comprising the host cell described above and a culture medium. Aspects of the present compositions and methods include a method of producing a *Ggr3A* polypeptide comprising: culturing the host cell described above in a culture medium, under suitable conditions to produce the beta-glucosidase.

Aspects of the present compositions and methods include a composition comprising a *Ggr3A* polypeptide in the supernatant of a culture medium produced in accordance with the methods for producing the beta-glucosidase as described above.

Disclosed Disclosed herein are nucleic acid constructs, recombinant expression vectors, engineered host cells comprising a polynucleotide encoding a polypeptide having beta-glucosidase activity, as described above and herein. Also disclosed herein are methods of preparing or producing the beta-glucosidase polypeptides disclosed herein or compositions comprising such beta-glucosidase polypeptides using the nucleic acid constructs, recombinant expression vectors, and/or engineered host cells. Also disclosed Disclosed herein is a nucleic acid constructs comprising a suitable signal peptide operably linked to the mature sequence of the beta- glucosidase that is at least 75% identical to SEQ ID NO:2 or to the mature sequence of SEQ ID NO:3, or is encoded by a polynucleotide that is at least 75% identical to SEQ ID NO:1, an isolated polynucleotide, a nucleic acid construct, a recombinant expression vector, or an engineered host cell comprising such a nucleic acid construct. In some embodiments, the signal peptide and beta-glucosidase sequences are derived from different microorganisms.

Also provided is an expression vector comprising the isolated nucleic acid in operable combination with a regulatory sequence. Additionally, a host cell is provided comprising the expression vector. In still further embodiments, a composition is provided, which comprises the host cell and a culture medium.

In some embodiments, the host cell is a bacterial cell or a fungal cell. In certain embodiments, the host cell is an ethanologen microbe, which is capable of metabolizing the soluble sugars produced from hydrolyzing a lignocellulosic biomass substrate, wherein the hydrolyzing can be through a chemical hydrolysis or enzymatic hydrolysis or a combination of these processes, but is also capable of expression of heterologous enzymes. In some embodiments, the host cell is a *Saccharomyces cerevisiae* or a *Zymomonas mobilis* cell, which are not only capable of expressing a heterologous polypeptide such as a *Ggr3A* polypeptide described herein, but also capable of fermenting sugars into ethanol and/or downstream products. In certain particular embodiments, the *Saccharomyces cerevisiae* cell or *Zymomonas mobilis* cell, which expresses the beta-glucosidase, is capable of fermenting the sugars produced from a lignocellulosic biomass by an enzyme composition comprising one or more beta-glucosidases. The enzyme composition comprising one or more beta-glucosidases may comprise the same beta-glucosidase or may comprise one or more different beta-glucosidases. In certain embodiments, the enzyme composition comprising one or more beta-glucosidases may be an enzyme mixture produced by an engineered host cell, which may be a bacterial or a fungal cell. When a *Saccharomyces cerevisiae* or a *Zymomonas mobilis* cell expressing the *Ggr3A* polypeptide of the present disclosure, the *Ggr3A* polypeptide may be expressed but not secreted. Accordingly the cellobiose must be introduced or "transported" into such a host cell in order for the beta-glucosidase *Ggr3A* polypeptide to catalyze the liberation of D-glucose. Therefore in certain embodiments, the *Saccharomyces cerevisiae* or a *Zymomonas mobilis* cell are transformed with a cellobiose transporter gene in addition to one that encodes the *Ggr3A* polypeptide. A cellobiose transporter and a beta-glucosidase have been expressed in *Saccharomyces cerevisiae* such that the resulting microbe is capable of fermenting cellobiose, for example, in Ha et al., PNAS, 108(2):504-509 (2011). Another cellobiose transporter has been expressed in a *Pichia* yeast, for example in published U.S. Patent Application No. 20110262983. A cellobiose transporter has been introduced into an *E.coli,* for example, in Sekar et al., Applied Environmental Microbiology, 78(5):1611-1614 (2012).

In further embodiments, the *Ggr3A* polypeptide is heterologously expressed by a host cell. For example, the *Ggr3A* polypeptide is expressed by an engineered microorganism that is not *Glomerella graminicola.* In some embodiments, the *Ggr3A* polypeptide is co-expressed with one or more different cellulase genes. In some embodiments, the *Ggr3A* polypeptide is co-expressed with one or more hemicellulase genes.

In some aspects, compositions comprising the recombinant *Ggr3A* polypeptides of the preceding paragraphs and methods of preparing such compositions are provided. In some embodiments, the composition further comprises one or more other cellulases, whereby the one or more other cellulases are co-expressed by a host cell with the *Ggr3A* polypeptide. For example, the one or more other cellulases can be selected from no or one or more other beta-glucosidases, one or more cellobiohydrolases, and/or one or more endoglucanases. Such other beta-glucosidases, cellobiohydrolases and/or endoglucanases, if present, can be co-expressed with the *Ggr3A* polypeptide by a single host cell. At least two of the two or more cellulases may be heterologous to each other or derived from different organisms. For example, the composition may comprise two beta-glucosidases, with the first one being a *Ggr3A* polypeptide, and the second beta-glucosidase being not derived from a *Glomerella graminicola* strain. For example, the composition may comprise at least one cellobiohydrolase, one endoglucanase, or one beta-glucosidase that is not derived from *Glomerella graminicola.* In some embodiments, one or more of the cellulases are endogenous to the host cell, but are overexpressed or expressed at a level that is different from that would otherwise be naturally-occurring in the host cell. For example, one or more of the cellulases may be a *Trichoderma reesei* CBH1 and/or CBH2, which are native to a *Trichoderma reesei* host cell, but either or both CBH1 and CBH2 are overexpressed or underexpressed when they are co-expressed in the *Trichoderma reesei* host cell with a *Ggr3A* polypeptide.

In certain embodiments, the composition comprising the recombinant *Ggr3A* polypeptide may further comprise one or more hemicellulases, whereby the one or more hemicellulases are co-expressed by a host cell with the *Ggr3A* polypeptide. For example, the one or more hemicellulases can be selected from one or more xylanase, one or more beta-xylosidases, and/or one or more L-arabinofuranosidases. Such other xylanases, beta-xylosidases and L-arabinofuranosidases, if present, can be co-expressed with the *Ggr3A* polypeptide by a single host cell. In some embodiments, the composition may comprise at least one beta-xylosidase, xylanase or arabinofuranosidase that is not derived from *Glomerella graminicola.*

In further aspects, the composition comprising the recombinant *Ggr3A* polypeptide may further comprise one or more other celluases and one or more hemicelluases, whereby the one or more cellulases and/or one or more hemicellulases are co-expressed by a host cell with the *Ggr3A* polypeptide. For example, a *Ggr3A* polypeptide may be co-expressed with one or more other beta-glucosidases, one or more cellobiohydrolases, one or more endoglucanases, one or more endo-xylanases, one or more beta-xylosidases, and one or more L-arabinofuranosidases, in addition to other non-cellulase non-hemicellulase enzymes or proteins in the same host cell. Aspects of the present compositions and methods accordingly include a composition comprising the host cell described above co-expressing a number of enzymes in addition to the *Ggr3A* polypeptide and a culture medium. Aspects of the present compositions and methods accordingly include a method of producing a *Ggr3A* -containing enzyme composition comprising: culturing the host cell, which co-expresses a number of enzymes as described above with the *Ggr3A* polypeptide in a culture medium, under suitable conditions to produce the *Ggr3A* and the other enzymes. Also provided are compositions that comprise the *Ggr3A* polypeptide and the other enzymes produced in accordance with the methods herein in supernatant of the culture medium. Such supernatant of the culture medium can be used as is, with minimum or no post-production processing, which may typically include filtration to remove cell debris, cell-kill procedures, and/or ultrafiltration or other steps to enrich or concentrate the enzymes therein. Such supernatants are called "whole broths" or "whole cellulase broths" herein.

In further aspects, the present invention pertains to a method of applying or using the composition as described above under conditions suitable for degrading or converting a cellulosic material and for producing a substance from a cellulosic material.

In a further aspect, methods for degrading or converting a cellulosic material into fermentable sugars are provided, comprising: contacting the cellulosic material, preferably having already been subject to one or more pretreatment steps, with the *Ggr3A* polypeptides or the compositions comprising such polypeptides of one of the preceding paragraphs to yield fermentable sugars.

These and other aspects of *Ggr3A* compositions and methods will be apparent from the following description.

### BRIEF DESCRIPTION OF THE DRAWINGS

Figure 1 depicts a map of the pENTR/D-TOPO-Bgl1(943/942) plasmid.
Figure 2 depicts a map of the pTrex3g 943/942 plasmid.
Figure 3 depicts a map of the pTTT-pyr2 plasmid (SEQ ID NO:41).
Figure 4 depicts a map of the pTTT-pyr2-Ggr3A plasmid (SEQ ID NO:42).
Figure 5 depicts a map of the pSC11 plasmid.
Figure 6 depicts a map of the pZC11 plasmid.
Figure 7 is a comparison of dose curves of Ggr3A vs. *Trichoderma reesei* Bgl1, each in a Spezyme CP enzyme background, on PASC substrate. The left panel depicts the glucose yield over varying enzyme doses whereas the right panel depicts the residual cellobiose concentration over varying enzyme doses.
Figure 8 is a comparison of cellobiose hydrolysis kinetics of Ggr3A vs. *Trichoderma reesei* Bgl1, as measured on a 100mM cellobiose substrate in accordance with Example 8.
Figures 9A-9B depict comparison of dose curves of Ggr3A vs. *Trichoderma reesei* Bgll, each in a Spezyme CP enzyme background, on whPCS substrate. Figure 9A depicts the dose curves on glucose yield from the substrate. Figure 9B depicts the residual cellobiose concentration.

### DETAILED DESCRIPTION

Described herein are compositions and methods relating to a recombinant beta-glucosidase *Ggr3A* belonging to glycosyl hydrolase family 3 from *Glomerella graminicola.* The present compositions and methods are based, in part, on the observations that recombinant *Ggr3A* polypeptides have higher cellulase activities and are more robust as a component of an enzyme composition when the composition is used to hydrolyze a lignocellulosic biomass material or feedstock than, for example, a known benchmark high fidelity beta-glucosidase Bgl1 of *Trichoderma reesei.* These features of *Ggr3A* polypeptides make them, or variants thereof, suitable for use in numerous processes, including, for example, in the conversion or hydrolysis of a lignocellulosic biomass feedstock.

Before the present compositions and methods are described in greater detail, it is to be understood that the present compositions and methods are not limited to particular embodiments described, as such may, of course, vary. It is also to be understood that the terminology used herein is for the purpose of describing particular embodiments only, and is not intended to be limiting, since the scope of the present compositions and methods will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the present compositions and methods. The upper and lower limits of these smaller ranges may independently be included in the smaller ranges and are also encompassed within the present compositions and methods, subject to any specifically excluded limit in the stated range. Where the stated range includes one or both of the limits, ranges excluding either or both of those included limits are also included in the present compositions and methods.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number. For example, in connection with a numerical value, the term "about" refers to a range of -10% to +10% of the numerical value, unless the term is otherwise specifically defined in context. In another example, the phrase a "pH value of about 6" refers to pH values of from 5.4 to 6.6, unless the pH value is specifically defined otherwise.

The headings provided herein are not limitations of the various aspects or embodiments of the present compositions and methods which can be had by reference to the specification as a whole. Accordingly, the terms defined immediately below are more fully defined by reference to the specification as a whole.

The present document is organized into a number of sections for ease of reading; however, the reader will appreciate that statements made in one section may apply to other sections. In this manner, the headings used for different sections of the disclosure should not be construed as limiting.

Unless defined otherwise, all technical and scientific terms used herein have the same meaning as commonly understood by one of ordinary skill in the art to which the present compositions and methods belongs. Although any methods and materials similar or equivalent to those described herein can also be used in the practice or testing of the present compositions and methods, representative illustrative methods and materials are now described.

In accordance with this detailed description, the following abbreviations and definitions apply. Note that the singular forms "a," "an," and "the" include plural referents unless the context clearly dictates otherwise. Thus, for example, reference to "an enzyme" includes a plurality of such enzymes, and reference to "the dosage" includes reference to one or more dosages and equivalents thereof known to those skilled in the art, and so forth.

It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

The term "recombinant," when used in reference to a subject cell, nucleic acid, polypeptides/enzymes or vector, indicates that the subject has been modified from its native state. Thus, for example, recombinant cells express genes that are not found within the native (non-recombinant) form of the cell, or express native genes at different levels or under different conditions than found in nature. Recombinant nucleic acids may differ from a native sequence by one or more nucleotides and/or are operably linked to heterologous sequences, e.g., a heterologous promoter, signal sequences that allow secretion, etc., in an expression vector. Recombinant polypeptides/enzymes may differ from a native sequence by one or more amino acids and/or are fused with heterologous sequences. A vector comprising a nucleic acid encoding a beta-glucosidase is, for example, a recombinant vector.

It is further noted that the term "consisting essentially of," as used herein refers to a composition wherein the component(s) after the term is in the presence of other known component(s) in a total amount that is less than 30% by weight of the total composition and do not contribute to or interferes with the actions or activities of the component(s).

It is further noted that the term "comprising," as used herein, means including, but not limited to, the component(s) after the term "comprising." The component(s) after the term "comprising" are required or mandatory, but the composition comprising the component(s) may further include other non-mandatory or optional component(s).

It is also noted that the term "consisting of," as used herein, means including, and limited to, the component(s) after the term "consisting of." The component(s) after the term "consisting of" are therefore required or mandatory, and no other component(s) are present in the composition.

As will be apparent to those of skill in the art upon reading this disclosure, each of the individual embodiments described and illustrated herein has discrete components and features which may be readily separated from or combined with the features of any of the other several embodiments without departing from the scope or spirit of the present compositions and methods described herein. Any recited method can be carried out in the order of events recited or in any other order which is logically possible.

"Beta-glucosidase" refers to a beta-D-glucoside glucohydrolase of E.C. 3.2.1.21. The term "beta-glucosidase activity" therefore refers the capacity of catalyzing the hydrolysis of beta-D-glucose or cellobiose to release D-glucose. Beta-glucosidase activity may be determined using a cellobiase assay, for example, which measures the capacity of the enzyme to catalyze the hydrolysis of a cellobiose substrate to yield D-glucose, as described in Example 2C of the present disclosure.

As used herein, *"Ggr3A"* or "a *Ggr3A* polypeptide" refers to a beta-glucosidase belonging to glycosyl hydrolase family 3 (e.g., a recombinant beta-glucosidase) derived from *Glomerella graminicola* (and variants thereof), that has improved performance hydrolyzing a lignocellulosic biomass substrate when compared to a benchmark beta-glucosidase, the wild type *Trichoderma reesei* Bgll polypeptide having the amino acid sequence of SEQ ID NO:4. According to aspects of the present compositions and methods, *Ggr3A* polypeptides include those having the amino acid sequence depicted in SEQ ID NO:2, as well as derivative or variant polypeptides having at least 90%, at least 91%, at least 92%, at least 93%, at least 94%, at least 95%, at least 96%, at least 97%, at least 98%, or at least 99% sequence identity to the amino acid sequence of SEQ ID NO:2, or to the mature sequence SEQ ID NO:2, or to a fragment of at least 100 residues in length of SEQ. ID NO:2, wherein the *Ggr3A* polypeptides not only have beta-glucosidase activity and capable of catalyzing the conversion of cellobiose into D-glucose, but also have higher beta-glucosidase activity and have higher capacity to catalyze the conversion of cellobiose to D-glucose than *Trichoderma reesei* Bgll.

The *Ggr3A* polypeptides to be used in the compositions and methods of the present disclosure would have at least 5%, at least 10%, preferably at least 20%, more preferably at least 30%, and even more preferably at least 40%, more preferably at least 50%, even more preferably at least 60%, and preferably at least 70%, more preferably at least 90%, even more preferably at least 100% or more of the beta-glucosidase activity of the polypeptide of the amino acid sequence of SEQ ID NO:2, or of the polypeptide consisting of residues 20 to 876 of the SEQ ID NO:2; or of the mature sequence SEQ ID NO:3.

"Family 3 glycosyl hydrolase" or "GH3" refers to polypeptides falling within the definition of glycosyl hydrolase family 3 according to the classification by Henrissat, Biochem. J. 280:309-316 (1991), and by Henrissat & Cairoch, Biochem. J., 316:695-696 (1996).

*Ggr3A* polypeptides according to the present compositions and methods described herein are isolated or purified. By purification or isolation is meant that the *Ggr3A* polypeptide is altered from its natural state by virtue of separating the *Ggr3A* from some or all of the naturally occurring constituents with which it is associated in nature. Such isolation or purification may be accomplished by art-recognized separation techniques such as ion exchange chromatography, affinity chromatography, hydrophobic separation, dialysis, protease treatment, ammonium sulphate precipitation or other protein salt precipitation, centrifugation, size exclusion chromatography, filtration, microfiltration, gel electrophoresis or separation on a gradient to remove whole cells, cell debris, impurities, extraneous proteins, or enzymes undesired in the final composition. It is further possible to then add constituents to the *Ggr3A-*containing composition which provide additional benefits, for example, activating agents, anti-inhibition agents, desirable ions, compounds to control pH or other enzymes or chemicals.

As used herein, "microorganism" refers to a bacterium, a fungus, a virus, a protozoan, and other microbes or microscopic organisms.

As used herein, a "derivative" or "variant" of a polypeptide means a polypeptide, which is derived from a precursor polypeptide (e.g., the native polypeptide) by addition of one or more amino acids to either or both the C- and N-terminal end, substitution of one or more amino acids at one or a number of different sites in the amino acid sequence, deletion of one or more amino acids at either or both ends of the polypeptide or at one or more sites in the amino acid sequence, or insertion of one or more amino acids at one or more sites in the amino acid sequence. The preparation of a *Ggr3A* derivative or variant may be achieved in any convenient manner, e.g., by modifying a DNA sequence which encodes the native polypeptides, transformation of that DNA sequence into a suitable host, and expression of the modified DNA sequence to form the derivative/variant *Ggr3A.* Derivatives or variants further include *Ggr3A* polypeptides that are chemically modified, e.g., glycosylation or otherwise changing a characteristic of the *Ggr3A* polypeptide. While derivatives and variants of *Ggr3A* are encompassed by the present compositions and methods, such derivates and variants will display improved beta-glucosidase activity when compared to that of the wild type *Trichoderma reesei* Bgl1 of SEQ ID NO:4, under the same lignocellulosic biomass substrate hydrolysis conditions.

In certain aspects, a *Ggr3A* polypeptide of the compositions and methods herein may also encompasses functional fragment of a polypeptide or a polypeptide fragment having beta-glucosidase activity, which is derived from a parent polypeptide, which may be the full length polypeptide comprising or consisting of SEQ ID NO:2, or the mature sequence comprising or consisting SEQ ID NO:3. The functional polypeptide may have been truncated either in the N-terminal region, or the C-terminal region, or in both regions to generate a fragment of the parent polypeptide. For the purpose of the present disclosure, a functional fragment must have at least 20%, more preferably at least 30%, 40%, 50%, or preferably, at least 60%, 70%, 80%, or even more preferably at least 90% of the beta-glucosidase activity of that of the parent polypeptide.

In certain aspects, a *Ggr3A* derivative/variant will have anywhere from 75% to 99% (or more) amino acid sequence identity to the amino acid sequence of SEQ ID NO:2, or to the mature sequence SEQ ID NO:3, e.g., 90%, 91%, 92%, 93%, 94%, 95%, 96%, 97%, 98%, or 99% amino acid sequence identity to the amino acid sequence of SEQ ID NO:2 or to the mature sequence SEQ ID NO:3. In some embodiments, amino acid substitutions are "conservative amino acid substitutions" using L-amino acids, wherein one amino acid is replaced by another biologically similar amino acid. Conservative amino acid substitutions are those that preserve the general charge, hydrophobicity/hydrophilicity, and/or steric bulk of the amino acid being substituted. Examples of conservative substitutions are those between the following groups: Gly/Ala, Val/Ile/Leu, Lys/Arg, Asn/Gln, Glu/Asp, Ser/Cys/Thr, and Phe/Trp/Tyr. A derivative may, for example, differ by as few as 1 to 10 amino acid residues, such as 6-10, as few as 5, as few as 4, 3, 2, or even 1 amino acid residue. In some embodiments, a *Ggr3A* derivative may have an N-terminal and/or C-terminal deletion, where the *Ggr3A* derivative excluding the deleted terminal portion(s) is identical to a contiguous sub-region in SEQ ID NO: 2 or SEQ ID NO:3.

As used herein, "percent (%) sequence identity" with respect to the amino acid or nucleotide sequences identified herein is defined as the percentage of amino acid residues or nucleotides in a candidate sequence that are identical with the amino acid residues or nucleotides in a *Ggr3A* sequence, after aligning the sequences and introducing gaps, if necessary, to achieve the maximum percent sequence identity, and not considering any conservative substitutions as part of the sequence identity.

By "homologue" shall mean an entity having a specified degree of identity with the subject amino acid sequences and the subject nucleotide sequences. A homologous sequence is taken to include an amino acid sequence that is at least 75%, 80%, 85%, 86%, 87%, 88%, 89%, 90%, 91%, 92%, 93%, 94%, 95%, 96%, 97%, 98% or even 99% identical to the subject sequence, using conventional sequence alignment tools (e.g., Clustal, BLAST, and the like). Typically, homologues will include the same active site residues as the subject amino acid sequence, unless otherwise specified.

Methods for performing sequence alignment and determining sequence identity are known to the skilled artisan, may be performed without undue experimentation, and calculations of identity values may be obtained with definiteness. See, for example, Ausubel et al., eds. (1995) Current Protocols in Molecular Biology, Chapter 19 (Greene Publishing and Wiley-Interscience, New York); and the ALIGN program (Dayhoff (1978) in Atlas of Protein Sequence and Structure 5:Suppl. 3 (National Biomedical Research Foundation, Washington, D.C.). A number of algorithms are available for aligning sequences and determining sequence identity and include, for example, the homology alignment algorithm of Needleman et al. (1970) J. Mol. Biol. 48:443; the local homology algorithm of Smith et al. (1981) Adv. Appl. Math. 2:482; the search for similarity method of Pearson et al. (1988) Proc. Natl. Acad. Sci. 85:2444; the Smith-Waterman algorithm (Meth. Mol. Biol. 70:173-187 (1997); and BLASTP, BLASTN, and BLASTX algorithms (see Altschul et al. (1990) J. Mol. Biol. 215:403-410).

Computerized programs using these algorithms are also available, and include, but are not limited to: ALIGN or Megalign (DNASTAR) software, or WU-BLAST-2 (Altschul et al., Meth. Enzym., 266:460-480 (1996)); or GAP, BESTFIT, BLAST, FASTA, and TFASTA, available in the Genetics Computing Group (GCG) package, Version 8, Madison, Wisconsin, USA; and CLUSTAL in the PC/Gene program by Intelligenetics, Mountain View, California. Those skilled in the art can determine appropriate parameters for measuring alignment, including algorithms needed to achieve maximal alignment over the length of the sequences being compared. Preferably, the sequence identity is determined using the default parameters determined by the program. Specifically, sequence identity can determined by using Clustal W (Thompson J.D. et al. (1994) Nucleic Acids Res. 22:4673-4680) with default parameters, i.e.:

| | |
|---|---|
| Gap opening penalty: | 10.0 |
| Gap extension penalty: | 0.05 |
| Protein weight matrix: | BLOSUM series |
| DNA weight matrix: | IUB |
| Delay divergent sequences %: | 40 |
| Gap separation distance: | 8 |
| DNA transitions weight: | 0.50 |
| List hydrophilic residues: | GPSNDQEKR |
| Use negative matrix: | OFF |
| Toggle Residue specific penalties: | ON |
| Toggle hydrophilic penalties: | ON |
| Toggle end gap separation penalty | OFF |

As used herein, "expression vector" means a DNA construct including a DNA sequence which is operably linked to a suitable control sequence capable of affecting the expression of the DNA in a suitable host. Such control sequences may include a promoter to affect transcription, an optional operator sequence to control transcription, a sequence encoding suitable ribosome-binding sites on the mRNA, and sequences which control termination of transcription and translation. Different cell types may be used with different expression vectors. An exemplary promoter for vectors used in *Bacillus subtilis* is the AprE promoter; an exemplary promoter used in *Streptomyces lividans* is the A4 promoter (from *Aspergillus niger*); an exemplary promoter used in *E. coli* is the Lac promoter, an exemplary promoter used in *Saccharomyces cerevisiae* is *PGK1,* an exemplary promoter used in *Aspergillus niger* is *glaA,* and an exemplary promoter for *Trichoderma reesei* is *cbhI.* The vector may be a plasmid, a phage particle, or simply a potential genomic insert. Once transformed into a suitable host, the vector may replicate and function independently of the host genome, or may, under suitable conditions, integrate into the genome itself. In the present specification, plasmid and vector are sometimes used interchangeably. However, the present compositions and methods are intended to include other forms of expression vectors which serve equivalent functions and which are, or become, known in the art. Thus, a wide variety of host/expression vector combinations may be employed in expressing the DNA sequences described herein. Useful expression vectors, for example, may consist of segments of chromosomal, non-chromosomal and synthetic DNA sequences such as various known derivatives of SV40 and known bacterial plasmids, e.g., plasmids from *E. coli* including col E1, pCR1, pBR322, pMb9, pUC 19 and their derivatives, wider host range plasmids, e.g., RP4, phage DNAs e.g., the numerous derivatives of phage λ, e.g., NM989, and other DNA phages, e.g., M13 and filamentous single stranded DNA phages, yeast plasmids such as the 2µ plasmid or derivatives thereof, vectors useful in eukaryotic cells, such as vectors useful in animal cells and vectors derived from combinations of plasmids and phage DNAs, such as plasmids which have been modified to employ phage DNA or other expression control sequences. Expression techniques using the expression vectors of the present compositions and methods are known in the art and are described generally in, for example, Sambrook et al., Molecular Cloning: A Laboratory Manual, Second Edition, Cold Spring Harbor Press (1989). Often, such expression vectors including the DNA sequences described herein are transformed into a unicellular host by direct insertion into the genome of a particular species through an integration event (see e.g., Bennett & Lasure, More Gene Manipulations in Fungi, Academic Press, San Diego, pp. 70-76 (1991) and articles cited therein describing targeted genomic insertion in fungal hosts).

As used herein, "host strain" or "host cell" means a suitable host for an expression vector including DNA according to the present compositions and methods. Host cells useful in the present compositions and methods are generally prokaryotic or eukaryotic hosts, including any transformable microorganism in which expression can be achieved. Specifically, host strains may be *Bacillus subtilis, Streptomyces lividans, Escherichia coli, Trichoderma reesei, Saccharomyces cerevisiae* or *Aspergillus niger.* In certain embodiments, the host cell may be an ethanologen microbe, which may be, for example, a yeast such as *Saccharomyces cerevisiae* or a bacterium ethanologen such as a *Zymomonas mobilis.* When a *Saccharomyces cerevisiae* or *Zymomonas mobilis* is used as the host cell, and if the beta-glucosidase gene is not made to secret from host cell but is expressed intracellularly, a cellibiose transporter gene can be introduced into the host cell in order to allow the intracellularly expressed beta-glucosidase to act upon the cellobiose substrate and liberate glucose, which will then be metabolized subsequently or immediately by the microorganisms and converted into ethanol.

Host cells are transformed or transfected with vectors constructed using recombinant DNA techniques. Such transformed host cells may be capable of one or both of replicating the vectors encoding *Ggr3A* (and its derivatives or variants (mutants)) and expressing the desired peptide product. In certain embodiments according to the present compositions and methods, "host cell" means both the cells and protoplasts created from the cells of *Trichoderma sp.*

The terms "transformed," "stably transformed," and "transgenic," used with reference to a cell means that the cell contains a non-native (e.g., heterologous) nucleic acid sequence integrated into its genome or carried as an episome that is maintained through multiple generations.

The term "introduced" in the context of inserting a nucleic acid sequence into a cell, means "transfection", "transformation" or "transduction," as known in the art.

A "host strain" or "host cell" is an organism into which an expression vector, phage, virus, or other DNA construct, including a polynucleotide encoding a polypeptide of interest *(e.g.,* a beta-glucosidase) has been introduced. Exemplary host strains are microbial cells (*e.g.,* bacteria, filamentous fungi, and yeast) capable of expressing the polypeptide of interest. The term "host cell" includes protoplasts created from cells.

The term "heterologous" with reference to a polynucleotide or polypeptide refers to a polynucleotide or polypeptide that does not naturally occur in a host cell.

The term "endogenous" with reference to a polynucleotide or polypeptide refers to a polynucleotide or polypeptide that occurs naturally in the host cell.

The term "expression" refers to the process by which a polypeptide is produced based on a nucleic acid sequence. The process includes both transcription and translation.

Accordingly the process of converting a lignocellulosic biomass substrate to an ethanol can, in some embodiments, comprise two beta-glucosidase activities. For example, a first beta-glucosidase activity may be applied to the lignocellulosic biomass substrate during the saccharification or hydrolysis step, and a second beta-glucosidase activity can be applied as part of the ethanologen microbe in the fermentation step during which the monomeric or fermentable sugars that resulted from the saccharification or hydrolysis step are metabolized. The first and second beta-glucosidase activities may, in some embodiments, result from the presence of the same beta-glucosidase polypeptide. For example, the first beta-glucosidase activity in the saccharification may result from the presence of a *Ggr3A* polypeptide of the invention, whereas the second beta-glucosidase activity in the fermentation stage may result from the expression of a different beta-glucosidase by the ethanologen microbe. In another example, the first and second beta-glucosidase activities may result from the presence of the same polypeptide in the saccharification or hydrolysis step and the fermentation step. For example, the same *Ggr3A* polypeptide of the invention may, in some embodiments, provide the beta-glucosidase activities for both the hydrolysis or saccharification step and the fermentation step.

In certain other embodiments, the process of converting a lignocellulosic biomass substrate to an ethanol can, comprise two beta-glucosidase activities whereas the saccharification or hydrolysis step and the fermentation step occurs simultaneously, for example, in the same tank. Two or more beta-glucosidase polypeptides may contribute to the beta-glucosidase activities, one of which may be a *Ggr3A* polypeptide of the invention.

In certain further embodiments, the process of converting a lignocellulosic biomass to an ethanol can comprise a single beta-glucosidase activity whereas either the saccharification or hydrolysis step or the fermentation step, but not both steps involves the participation of a beta-glucosidase. For example, a *Ggr3A* polypeptide of the invention or a composition comprising the *Ggr3A* polypeptide may be used in the saccharification step. In another example, the enzyme composition that is used to hydrolyze the lignocellulosic biomass substrate does not comprise a beta-glucosidase activity, whereas the ethanologen microbe expresses a beta-glucosidase polypeptide, for example, a *Ggr3A* polypeptide of the invention.

As used herein, "signal sequence" means a sequence of amino acids bound to the N-terminal portion of a polypeptide which facilitates the secretion of the mature form of the polypeptide outside of the cell. This definition of a signal sequence is a functional one. The mature form of the extracellular polypeptide lacks the signal sequence which is cleaved off during the secretion process. While the native signal sequence of *Ggr3A* may be employed in aspects of the present compositions and methods, other non-native signal sequences may be employed (e.g., SEQ ID NO: 11). The term "mature," when referring to a polypeptide herein, is meant a polypeptide in its final form(s) following translation and any post-translational modifications. For example, the *Ggr3A* polypeptides of the invention has one or more mature forms, at least one of which has the amino acid sequence of SEQ ID NO:3.

The beta-glucosidase polypeptides of the invention may be referred to as "precursor," "immature," or "full-length," in which case they include a signal sequence, or may be referred to as "mature," in which case they lack a signal sequence. Mature forms of the polypeptides are generally the most useful. Unless otherwise noted, the amino acid residue numbering used herein refers to the mature forms of the respective amylase polypeptides. The beta-glucosidase polypeptides of the invention may also be truncated to remove the N or C-termini, so long as the resulting polypeptides retain beta-glucosidase activity.

The beta-glucosidase polypeptides of the invention may also be a "chimeric" or "hybrid" polypeptide, in that it includes at least a portion of a first beta-glucosidase polypeptide, and at least a portion of a second beta-glucosidase polypeptide (such chimeric beta-glucosidase polypeptides may, for example, be derived from the first and second beta-glucosidases using known technologies involving the swapping of domains on each of the beta-glucosidases). The present beta-glucosidase polypeptides may further include heterologous signal sequence, an epitope to allow tracking or purification, or the like. When the term "heterologous" is used to refer to a signal sequence used to express a polypeptide of interest, it is meant that the signal sequence is, for example, derived from a different microorganism as the polypeptide of interest. Examples of suitable heterologous signal sequences for expressing the *Ggr3A* polypeptides herein, may be, for example, those from *Trichoderma reesei,* such as, for example, any one of SEQ ID NOs: 11, 12, 13, 14, or 15.

As used herein, "functionally attached" or "operably linked" means that a regulatory region or functional domain having a known or desired activity, such as a promoter, terminator, signal sequence or enhancer region, is attached to or linked to a target (e.g., a gene or polypeptide) in such a manner as to allow the regulatory region or functional domain to control the expression, secretion or function of that target according to its known or desired activity.

As used herein, the terms "polypeptide" and "enzyme" are used interchangeably to refer to polymers of any length comprising amino acid residues linked by peptide bonds. The conventional one-letter or three-letter codes for amino acid residues are used herein. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art.

As used herein, "wild-type" and "native" genes, enzymes, or strains, are those found in nature.

The terms "wild-type," "parental," or "reference," with respect to a polypeptide, refer to a naturally-occurring polypeptide that does not include a man-made substitution, insertion, or deletion at one or more amino acid positions. Similarly, the term "wild-type," "parental," or "reference," with respect to a polynucleotide, refers to a naturally-occurring polynucleotide that does not include a man-made nucleoside change. However, a polynucleotide encoding a wild-type, parental, or reference polypeptide is not limited to a naturally-occurring polynucleotide, but rather encompasses any polynucleotide encoding the wild-type, parental, or reference polypeptide.

As used herein, a "variant polypeptide" refers to a polypeptide that is derived from a parent (or reference) polypeptide by the substitution, addition, or deletion, of one or more amino acids, typically by recombinant DNA techniques. Variant polypeptides may differ from a parent polypeptide by a small number of amino acid residues. They may be defined by their level of primary amino acid sequence homology/identity with a parent polypeptide. Suitably, variant polypeptides have at least 70%, at least 75%, at least 80%, at least 85%, at least 90%, at least 91%, at least 92%, at least 93%, at least 94%, at least 95%, at least 96%, at least 97%, at least 98%, or even at least 99% amino acid sequence identity to a parent polypeptide.

As used herein, a "variant polynucleotide" encodes a variant polypeptide, has a specified degree of homo logy/identity with a parent polynucleotide, or hybridized under stringent conditions to a parent polynucleotide or the complement thereof. Suitably, a variant polynucleotide has at least 80%, at least 85%, at least 90%, at least 91%, at least 92%, at least 93%, at least 94%, at least 95%, at least 96%, at least 97%, at least 98%, or even at least 99% nucleotide sequence identity to a parent polynucleotide or to a complement of the parent polynucleotide. Methods for determining percent identity are known in the art and described above.

The term "derived from" encompasses the terms "originated from," "obtained from," "obtainable from," "isolated from," and "created from," and generally indicates that one specified material find its origin in another specified material or has features that can be described with reference to the another specified material.

As used herein, the term "hybridization conditions" refers to the conditions under which hybridization reactions are conducted. These conditions are typically classified by degree of "stringency" of the conditions under which hybridization is measured. The degree of stringency can be based, for example, on the melting temperature (Tm) of the nucleic acid binding complex or probe. For example, "maximum stringency" typically occurs at about Tm -5°C (5°C below the Tm of the probe); "high stringency" at about 5-10°C below the Tm; "intermediate stringency" at about 10-20°C below the Tm of the probe; and "low stringency" at about 20-25°C below the Tm. Alternatively, or in addition, hybridization conditions can be based upon the salt or ionic strength conditions of hybridization, and/or upon one or more stringency washes, e.g.,: 6X SSC = very low stringency; 3X SSC = low to medium stringency; 1X SSC = medium stringency; and 0.5X SSC = high stringency. Functionally, maximum stringency conditions may be used to identify nucleic acid sequences having strict identity or near-strict identity with the hybridization probe; while high stringency conditions are used to identify nucleic acid sequences having about 80% or more sequence identity with the probe. For applications requiring high selectivity, it is typically desirable to use relatively stringent conditions to form the hybrids (e.g., relatively low salt and/or high temperature conditions are used).

As used herein, the term "hybridization" refers to the process by which a strand of nucleic acid joins with a complementary strand through base pairing, as known in the art. More specifically, "hybridization" refers to the process by which one strand of nucleic acid forms a duplex with, *i.e.,* base pairs with, a complementary strand, as occurs during blot hybridization techniques and PCR techniques. A nucleic acid sequence is considered to be "selectively hybridizable" to a reference nucleic acid sequence if the two sequences specifically hybridize to one another under moderate to high stringency hybridization and wash conditions. Hybridization conditions are based on the melting temperature (Tm) of the nucleic acid binding complex or probe. For example, "maximum stringency" typically occurs at about Tm-5°C (5° below the Tm of the probe); "high stringency" at about 5-10°C below the Tm; "intermediate stringency" at about 10-20°C below the Tm of the probe; and "low stringency" at about 20-25°C below the Tm. Functionally, maximum stringency conditions may be used to identify sequences having strict identity or near-strict identity with the hybridization probe; while intermediate or low stringency hybridization can be used to identify or detect polynucleotide sequence homologs.

Intermediate and high stringency hybridization conditions are well known in the art. For example, intermediate stringency hybridizations may be carried out with an overnight incubation at 37°C in a solution comprising 20% formamide, 5 x SSC (150mM NaCl, 15 mM trisodium citrate), 50 mM sodium phosphate (pH 7.6), 5 x Denhardt's solution, 10% dextran sulfate and 20 mg/ml denatured sheared salmon sperm DNA, followed by washing the filters in 1x SSC at about 37 - 50°C. High stringency hybridization conditions may be hybridization at 65°C and 0.1X SSC (where 1X SSC = 0.15 M NaCl, 0.015 M Na₃ citrate, pH 7.0). Alternatively, high stringency hybridization conditions can be carried out at about 42°C in 50% formamide, 5X SSC, 5X Denhardt's solution, 0.5% SDS and 100 µg/ml denatured carrier DNA followed by washing two times in 2X SSC and 0.5% SDS at room temperature and two additional times in 0.1X SSC and 0.5% SDS at 42°C. And very high stringent hybridization conditions may be hybridization at 68°C and 0.1X SSC. Those of skill in the art know how to adjust the temperature, ionic strength, etc. as necessary to accommodate factors such as probe length and the like.

A nucleic acid encoding a variant beta-glucosidase may have a Tₘ reduced by 1°C-3°C or more compared to a duplex formed between the nucleotide of SEQ ID NO:1 and its identical complement.

The phrase "substantially similar" or "substantially identical," in the context of at least two nucleic acids or polypeptides, means that a polynucleotide or polypeptide comprises a sequence that has at least about 90%, at least about 91%, at least about 92%, at least about 93%, at least about 94%, at least about 95%, at least about 96%, at least about 97%, at least about 98%, or even at least about 99% identical to a parent or reference sequence, or does not include amino acid substitutions, insertions, deletions, or modifications made only to circumvent the present description without adding functionality.

As used herein, an "expression vector" refers to a DNA construct containing a DNA sequence that encodes a specified polypeptide and is operably linked to a suitable control sequence capable of effecting the expression of the polypeptides in a suitable host. Such control sequences may include a promoter to effect transcription, an optional operator sequence to control such transcription, a sequence encoding suitable mRNA ribosome binding sites and/or sequences that control termination of transcription and translation. The vector may be a plasmid, a phage particle, or a potential genomic insert. Once transformed into a suitable host, the vector may replicate and function independently of the host genome, or may, in some instances, integrate into the host genome.

The term "recombinant," refers to genetic material (*i.e.,* nucleic acids, the polypeptides they encode, and vectors and cells comprising such polynucleotides) that has been modified to alter its sequence or expression characteristics, such as by mutating the coding sequence to produce an altered polypeptide, fusing the coding sequence to that of another gene, placing a gene under the control of a different promoter, expressing a gene in a heterologous organism, expressing a gene at a decreased or elevated levels, expressing a gene conditionally or constitutively in a manner different from its natural expression profile, and the like. Generally recombinant nucleic acids, polypeptides, and cells based thereon, have been manipulated by man such that they are not identical to related nucleic acids, polypeptides, and cells found in nature.

A "signal sequence" refers to a sequence of amino acids bound to the N-terminal portion of a polypeptide, and which facilitates the secretion of the mature form of the polypeptide from the cell. The mature form of the extracellular polypeptide lacks the signal sequence which is cleaved off during the secretion process.

The term "selective marker" or "selectable marker," refers to a gene capable of expression in a host cell that allows for ease of selection of those hosts containing an introduced nucleic acid or vector. Examples of selectable markers include but are not limited to antimicrobial substances (e.g., hygromycin, bleomycin, or chloramphenicol) and/or genes that confer a metabolic advantage, such as a nutritional advantage, on the host cell.

The term "regulatory element," refers to a genetic element that controls some aspect of the expression of nucleic acid sequences. For example, a promoter is a regulatory element which facilitates the initiation of transcription of an operably linked coding region. Additional regulatory elements include splicing signals, polyadenylation signals and termination signals.

As used herein, "host cells" are generally cells of prokaryotic or eukaryotic hosts that are transformed or transfected with vectors constructed using recombinant DNA techniques known in the art. Transformed host cells are capable of either replicating vectors encoding the polypeptide variants or expressing the desired polypeptide variant. In the case of vectors, which encode the pre- or pro-form of the polypeptide variant, such variants, when expressed, are typically secreted from the host cell into the host cell medium.

The term "introduced," in the context of inserting a nucleic acid sequence into a cell, means transformation, transduction, or transfection. Means of transformation include protoplast transformation, calcium chloride precipitation, electroporation, naked DNA, and the like as known in the art. (*See,* Chang and Cohen Mol. Gen. Genet. 168:111-115, 1979; Smith et al. Appl. Env. Microbiol. 51:634, 1986; and the review article by Ferrari et al., in Harwood, Bacillus, Plenum Publishing Corporation, pp. 57-72, 1989).

"Fused" polypeptide sequences are connected, *i.e.,* operably linked, via a peptide bond between two subject polypeptide sequences.

The term "filamentous fungi" refers to all filamentous forms of the subdivision Eumycotina, particulary Pezizomycotina species.

An "ethanologenic microorganism" refers to a microorganism with the ability to convert a sugar or oligosaccharide to ethanol.

Other technical and scientific terms have the same meaning as commonly understood by one of ordinary skill in the art to which this disclosure pertains (*See, e.g.,* Singleton and Sainsbury, Dictionary of Microbiology and Molecular Biology, 2d Ed., John Wiley and Sons, NY 1994; and Hale and Marham, The Harper Collins Dictionary of Biology, Harper Perennial, NY 1991).

### Beta-glucosidase Polypeptides, Polynucleotides, Vectors, and Host Cells

### Ggr3A Polypeptides

In one aspect, the present compositions and methods provide a recombinant Ggr3A beta-glucosidase polypeptide, fragments thereof, or variants thereof having beta-glucosidase activity. An example of a recombinant beta-glucosidase polypeptide was isolated from *Glomerella graminicola.* The mature Ggr3A polypeptide has the amino acid sequence set forth as SEQ ID NO:3. (The predicted signal sequence is set forth as SEQ ID NO: 45.) Similar, substantially similar Ggr3A polypeptides may occur in nature, *e.g.,* in other strains or isolates of *Glomerella graminicola.* These and other recombinant Ggr3A polypeptides are encompassed by the present compositions and methods.

In some embodiments, the recombinant Ggr3A polypeptide is a variant Ggr3A polypeptide having a specified degree of amino acid sequence identity to the exemplified Ggr3A polypeptide, e.g., at least 90%, 91%, 92%, 93%, 94%, 95%, 96%, 97%, 98%, or even at least 99% sequence identity to the amino acid sequence of SEQ ID NO:2 or to the mature sequence SEQ ID NO:3. Sequence identity can be determined by amino acid sequence alignment, e.g., using a program such as BLAST, ALIGN, or CLUSTAL, as described herein.

In certain embodiments, the recombinant Ggr3A polypeptides are produced recombinantly, in a microorganism, for example, in a bacterial or fungal host organism, while in others the Ggr3A polypeptides are produced synthetically, or are purified from a native source *(e.g., Glomerella graminicola).*

In certain embodiments, the recombinant Ggr3A polypeptide includes substitutions that do not substantially affect the structure and/or function of the polypeptide. Examples of these substitutions are conservative mutations, as summarized in Table I.

**Table 1. Amino Acid Substitutions**

| **Original Residue** | **Code** | **Acceptable Substitutions** |
|---|---|---|
| Alanine | A | D-Ala, Gly, beta-Ala, L-Cys, D-Cys |
| Arginine | R | D-Arg, Lys, D-Lys, homo-Arg, D-homo-Arg, Met, Ile, D-Met, D-Ile, Orn, D-Orn |
| Asparagine | N | D-Asn, Asp, D-Asp, Glu, D-Glu, Gln, D-Gln |
| Aspartic Acid | D | D-Asp, D-Asn, Asn, Glu, D-Glu, Gln, D-Gln |
| Cysteine | C | D-Cys, S-Me-Cys, Met, D-Met, Thr, D-Thr |
| Glutamine | Q | D-Gln, Asn, D-Asn, Glu, D-Glu, Asp, D-Asp |
| Glutamic Acid | E | D-Glu, D-Asp, Asp, Asn, D-Asn, Gln, D-Gln |
| Glycine | G | Ala, D-Ala, Pro, D-Pro, beta-Ala, Acp |
| Isoleucine | I | D-Ile, Val, D-Val, Leu, D-Leu, Met, D-Met |
| Leucine | L | D-Leu, Val, D-Val, Leu, D-Leu, Met, D-Met |
| Lysine | K | D-Lys, Arg, D-Arg, homo-Arg, D-homo-Arg, Met, D-Met, Ile, D-Ile, Orn, D-Orn |
| Methionine | M | D-Met, S-Me-Cys, Ile, D-Ile, Leu, D-Leu, Val, D-Val |
| Phenylalanine | F | D-Phe, Tyr, D-Thr, L-Dopa, His, D-His, Trp, D-Trp, Trans-3,4, or 5-phenylproline, cis-3,4, or 5-phenylproline |
| Proline | P | D-Pro, L-I-thioazolidine-4-carboxylic acid, D-or L-1-oxazolidine-4-carboxylic acid |
| Serine | S | D-Ser, Thr, D-Thr, allo-Thr, Met, D-Met, Met(O), D-Met(O), L-Cys, D-Cys |
| Threonine | T | D-Thr, Ser, D-Ser, allo-Thr, Met, D-Met, Met(O), D-Met(O), Val, D-Val |
| Tyrosine | Y | D-Tyr, Phe, D-Phe, L-Dopa, His, D-His |
| Valine | V | D-Val, Leu, D-Leu, Ile, D-Ile, Met, D-Met |

Substitutions involving naturally occurring amino acids are generally made by mutating a nucleic acid encoding a recombinant Ggr3A polypeptide, and then expressing the variant polypeptide in an organism. Substitutions involving non-naturally occurring amino acids or chemical modifications to amino acids are generally made by chemically modifying a Ggr3A polypeptide after it has been synthesized by an organism.

In some embodiments, variant recombinant Ggr3A polypeptides are substantially identical to SEQ ID NO:2 or SEQ ID NO:3, meaning that they do not include amino acid substitutions, insertions, or deletions that do not significantly affect the structure, function, or expression of the polypeptide. Such variant recombinant Ggr3A polypeptides will include those designed to circumvent the present description. In some embodiments, variants recombinant Ggr3A polypeptides, compositions and methods comprising these variants are not substantially identical to SEQ ID NO:2 or SEQ ID NO:3, but rather include amino acid substitutions, insertions, or deletions that affect, in certain circumstances, substantially, the structure, function, or expression of the polypeptide herein such that improved characteristics, including, e.g., improved specific activity to hydrolyze a lignocellulosic substrate, improved expression in a desirable host organism, improved thermostability, pH stability, etc, as compared to that of a polypeptide of SEQ ID NO:2 or SEQ ID NO:3 can be achieved.

In some embodiments, the recombinant Ggr3A polypeptide (including a variant thereof) has beta-glucosidase activity. Beta-glucosidase activity can be determined and measured using the assays described herein, for example, those described in Example 2, or by other assays known in the art.

Recombinant Ggr3A polypeptides include fragments of "full-length" Ggr3A polypeptides that retain beta-glucosidase activity. Preferably those functional fragments (i.e., fragments that retain beta-glucosidase activity) are at least 100 amino acid residues in length (e.g., at least 100 amino acid residues, at least 120 amino acid residues, at least 140 amino acid residues, at least 160 amino acid residues, at least 180 amino acid residues, at least 200 amino acid residues, at least 250 amino acid residues, at least 3000 amino acid residues, at least 350 amino acid residues, at least 400 amino acid residues, at least 450 amino acid residues, at least 500 amino acid residues, or at least 600 amino acid residues in length or longer). Such fragments suitably retain the active site of the full-length precursor polypeptides or full length mature polypeptides but may have deletions of non-critical amino acid residues. The activity of fragments can be readily determined using the assays described herein, for example those described in Example 2, or by other assays known in the art.

In some embodiments, the Ggr3A amino acid sequences and derivatives are produced as an N- and/or C-terminal fusion protein, for example, to aid in extraction, detection and/or purification and/or to add functional properties to the Ggr3A polypeptides. Examples of fusion protein partners include, but are not limited to, glutathione-S-transferase (GST), 6XHis, GAL4 (DNA binding and/or transcriptional activation domains), FLAG-, MYC-tags or other tags known to those skilled in the art. In some embodiments, a proteolytic cleavage site is provided between the fusion protein partner and the polypeptide sequence of interest to allow removal of fusion sequences. Suitably, the fusion protein does not hinder the activity of the recombinant Ggr3A polypeptide. In some embodiments, the recombinant Ggr3A polypeptide is fused to a functional domain including a leader peptide, propeptide, binding domain and/or catalytic domain. Fusion proteins are optionally linked to the recombinant Ggr3A polypeptide through a linker sequence that joins the Ggr3A polypeptide and the fusion domain without significantly affecting the properties of either component. The linker optionally contributes functionally to the intended application.

The present disclosure provides host cells that are engineered to express one or more Ggr3A polypeptides of the disclosure. Suitable host cells include cells of any microorganism *(e.g.,* cells of a bacterium, a protist, an alga, a fungus *(e.g.,* a yeast or filamentous fungus), or other microbe), and are preferably cells of a bacterium, a yeast, or a filamentous fungus.

Suitable host cells of the bacterial genera include, but are not limited to, cells of *Escherichia, Bacillus, Lactobacillus, Pseudomonas,* and *Streptomyces.* Suitable cells of bacterial species include, but are not limited to, cells of *Escherichia coli, Bacillus subtilis, Bacillus licheniformis, Lactobacillus brevis, Pseudomonas aeruginosa,* and *Streptomyces lividans.*

Suitable host cells of the genera of yeast include, but are not limited to, cells of *Saccharomyces, Schizosaccharomyces, Candida, Hansenula, Pichia, Kluyveromyces,* and *Ph*affia. Suitable cells of yeast species include, but are not limited to, cells of *Saccharomyces cerevisiae, Schizosaccharomyces pombe, Candida albicans, Hansenula polymorpha, Pichia pastoris, P. canadensis, Kluyveromyces marxianus,* and *Phaffia rhodozyma.*

Suitable host cells of filamentous fungi include all filamentous forms of the subdivision *Eumycotina.* Suitable cells of filamentous fungal genera include, but are not limited to, cells of *Acremonium, Aspergillus, Aureobasidium, Bjerkandera, Ceriporiopsis, Chrysoporium, Coprinus, Coriolus, Corynascus, Chaertomium, Cryptococcus, Filobasidium, Fusarium, Gibberella, Humicola, Magnaporthe, Mucor, Myceliophthora, Mucor, Neocallimastix, Neurospora, Paecilomyces, Penicillium, Phanerochaete, Phlebia, Piromyces, Pleurotus,Scytaldium, Schizophyllum, Sporotrichum, Talaromyces, Thermoascus, Thielavia, Tolypocladium, Trametes,* and *Trichoderma.*

Suitable cells of filamentous fungal species include, but are not limited to, cells of *Aspergillus awamori, Aspergillus fumigatus, Aspergillus foetidus, Aspergillus japonicus, Aspergillus nidulans, Aspergillus niger, Aspergillus oryzae, Chrysosporium lucknowense, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Bjerkandera adusta, Ceriporiopsis aneirina, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Coprinus cinereus, Coriolus hirsutus, Humicola insolens, Humicola lanuginosa, Mucor miehei, Myceliophthora thermophila, Neurospora crassa, Neurospora intermedia, Penicillium purpurogenum, Penicillium canescens, Penicillium solitum, Penicillium funiculosum Phanerochaete chrysosporium, Phlebia radiate, Pleurotus eryngii, Talaromyces flavus, Thielavia terrestris, Trametes villosa, Trametes versicolor, Trichoderma harzianum, Trichoderma koningii, Trichoderma longibrachiatum, Trichoderma reesei,* and *Trichoderma viride.*

Methods of transforming nucleic acids into these organisms are known in the art. For example, a suitable procedure for transforming *Aspergillus* host cells is described in EP 238 023.

In some embodiments, the recombinant Ggr3A polypeptide is fused to a signal peptide to, for example, facilitate extracellular secretion of the recombinant Ggr3A polypeptide. For example, in certain embodiments, the signal peptide is encoded by a sequence selected from SEQ ID NOs: 11-40. In particular embodiments, the recombinant Ggr3A polypeptide is expressed in a heterologous organism as a secreted polypeptide. The compositions and methods herein thus encompass methods for expressing a *Ggr3A* polypeptide as a secreted polypeptide in a heterologous organism. In some embodiments the recombinant Ggr3A polypeptide is expressed in a heterologous organism intracellularly, for example, when the heterologous organism is an ethanologen microbe such as a *Saccharomyces cerevisiae* or a *Zymomonas mobilis.* In those cases, a cellibiose transporter gene can be introduced into the organism using genetic engineering tools, in order for the Ggr3A polypeptide to act on the cellobiose substrate inside the organism to convert cellobiose into D-glucose, which is then metabolized or converted by the organism into ethanol.

The disclosure also provides expression cassettes and/or vectors comprising the above-described nucleic acids. Suitably, the nucleic acid encoding a Ggr3A polypeptide of the disclosure is operably linked to a promoter. Promoters are well known in the art. Any promoter that functions in the host cell can be used for expression of a beta-glucosidase and/or any of the other nucleic acids of the present disclosure. Initiation control regions or promoters, which are useful to drive expression of a beta-glucosidase nucleic acids and/or any of the other nucleic acids of the present disclosure in various host cells are numerous and familiar to those skilled in the art *(see,* for example, WO 2004/033646 and references cited therein). Virtually any promoter capable of driving these nucleic acids can be used.

Specifically, where recombinant expression in a filamentous fungal host is desired, the promoter can be a filamentous fungal promoter. The nucleic acids can be, for example, under the control of heterologous promoters. The nucleic acids can also be expressed under the control of constitutive or inducible promoters. Examples of promoters that can be used include, but are not limited to, a cellulase promoter, a xylanase promoter, the 1818 promoter (previously identified as a highly expressed protein by EST mapping *Trichoderma*). For example, the promoter can suitably be a cellobiohydrolase, endoglucanase, or beta-glucosidase promoter. A particulary suitable promoter can be, for example, a *T. reesei* cellobiohydrolase, endoglucanase, or beta-glucosidase promoter. For example, the promoter is a cellobiohydrolase I (*cbh*1) promoter. Non-limiting examples of promoters include a *cbh1, cbh2, egl1, egl2, egl3, egl4, egl5, pki1, gpd1, xyn1, or xyn2* promoter. Additional non-limiting examples of promoters include a *T. reesei cbh1, cbh2, egll, egl2, egl3, egl4, egl5, pki1, gpd1, xyn1,* or *xyn2* promoter.

The nucleic acid sequence encoding a Ggr3A polypeptide herein can be included in a vector. In some aspects, the vector contains the nucleic acid sequence encoding the Ggr3A polypeptide under the control of an expression control sequence. In some aspects, the expression control sequence is a native expression control sequence. In some aspects, the expression control sequence is a non-native expression control sequence. In some aspects, the vector contains a selective marker or selectable marker. In some aspects, the nucleic acid sequence encoding the Ggr3A polypeptide is integrated into a chromosome of a host cell without a selectable marker.

Suitable vectors are those which are compatible with the host cell employed. Suitable vectors can be derived, for example, from a bacterium, a virus (such as bacteriophage T7 or a M-13 derived phage), a cosmid, a yeast, or a plant. Suitable vectors can be maintained in low, medium, or high copy number in the host cell. Protocols for obtaining and using such vectors are known to those in the art (*see,* for example, Sambrook et al., Molecular Cloning: A Laboratory Manual, 2nd ed., Cold Spring Harbor, 1989).

In some aspects, the expression vector also includes a termination sequence. Termination control regions may also be derived from various genes native to the host cell. In some aspects, the termination sequence and the promoter sequence are derived from the same source.

An nucleic acid sequence encoding a Ggr3A polypeptide can be incorporated into a vector, such as an expression vector, using standard techniques (Sambrook et al., Molecular Cloning: A Laboratory Manual, Cold Spring Harbor, 1982).

In some aspects, it may be desirable to over-express a Ggr3A polypeptide and/or one or more of any other nucleic acid described in the present disclosure at levels far higher than currently found in naturally-occurring cells. In some embodiments, it may be desirable to under-express (e.g., mutate, inactivate, or delete) an endogenous beta-glucosidase and/or one or more of any other nucleic acid described in the present disclosure at levels far below that those currently found in naturally-occurring cells.

### ggr3a Polynucleotides

Another aspect of the compositions and methods described herein is a polynucleotide or a nucleic acid sequence that encodes a recombinant Ggr3A polypeptide (including variants and fragments thereof) having beta-glucosidase activity. In some embodiments the polynucleotide is provided in the context of an expression vector for directing the expression of a Ggr3A polypeptide in a heterologous organism, such as one identified herein. The polynucleotide that encodes a recombinant Ggr3A polypeptide may be operably-linked to regulatory elements (*e.g*., a promoter, terminator, enhancer, and the like) to assist in expressing the encoded polypeptides.

An example of a polynucleotide sequence encoding a recombinant Ggr3A polypeptide has the nucleotide sequence of SEQ ID NO:1. Similar, including substantially identical, polynucleotides encoding recombinant Ggr3A polypeptides and variants may occur in nature, *e.g.,* in other strains or isolates of *Glomerella graminicola,* or *Glomerella sp..* In view of the degeneracy of the genetic code, it will be appreciated that polynucleotides having different nucleotide sequences may encode the same Ggr3A polypeptides, variants, or fragments.

In some embodiments, polynucleotides encoding recombinant Ggr3A polypeptides have a specified degree of amino acid sequence identity to the exemplified polynucleotide encoding a Ggr3A polypeptide, *e.g*., at least 90%, at least 91%, at least 92%, at least 93%, at least 94%, at least 95%, at least 96%, at least 97%, at least 98%, or even at least 99% sequence identity to the amino acid sequence of SEQ ID NO:2. Homology can be determined by amino acid sequence alignment, e.g., using a program such as BLAST, ALIGN, or CLUSTAL, as described herein.

In some embodiments, the polynucleotide that encodes a recombinant Ggr3A polypeptide is fused in frame behind (*i.e.,* downstream of) a coding sequence for a signal peptide for directing the extracellular secretion of a recombinant Ggr3A polypeptide. As described herein, the term "heterologous" when used to refer to a signal sequence used to express a polypeptide of interest, it is meant that the signal sequence and the polypeptide of interest are from different organisms. Heterologous signal sequences include, for example, those from other fungal cellulase genes, such as, e.g., the signal sequence of *Trichoderma reesei* Bgll, of SEQ ID NO:11. Expression vectors may be provided in a heterologous host cell suitable for expressing a recombinant Ggr3A polypeptide, or suitable for propagating the expression vector prior to introducing it into a suitable host cell.

In some embodiments, polynucleotides encoding recombinant Ggr3A polypeptides hybridize to the polynucleotide of SEQ ID NO:1 (or to the complement thereof) under specified hybridization conditions. Examples of conditions are intermediate stringency, high stringency and extremely high stringency conditions, which are described herein.

*Ggr3a* polynucleotides may be naturally occurring or synthetic (*i.e.,* man-made), and may be codon-optimized for expression in a different host, mutated to introduce cloning sites, or otherwise altered to add functionality.

### Ggr3A Vectors and Host Cells

In order to produce a disclosed recombinant Ggr3A polypeptide, the DNA encoding the polypeptide can be chemically synthesized from published sequences or can be obtained directly from host cells harboring the gene (*e.g.,* by cDNA library screening or PCR amplification). In some embodiments, the *ggr3a* polynucleotide is included in an expression cassette and/or cloned into a suitable expression vector by standard molecular cloning techniques. Such expression cassettes or vectors contain sequences that assist initiation and termination of transcription (*e.g*., promoters and terminators), and typically can also contain one or more selectable markers.

The expression cassette or vector is introduced into a suitable expression host cell, which then expresses the corresponding *ggr3a* polynucleotide. Suitable expression hosts may be bacterial or fungal microbes. Bacterial expression host may be, for example, *Escherichia* (*e.g., Escherichia coli), Pseudomonas (e.g., P.fluorescens or P. stutzerei), Proteus (e.g., Proteus mirabilis*), *Ralstonia (e.g., Ralstonia eutropha), Streptomyces, Staphylococcus (e.g., S. carnosus), Lactococcus (e.g., L. lactis),* or *Bacillus (e.g., Bacillus subtilis, Bacillus megaterium, Bacillus licheniformis,* etc.). Fungal expression hosts may be, for example, yeasts, which can also serve as ethanologens. Yeast expression hosts may be, for example, *Saccharomyces cerevisiae, Schizosaccharomyces pombe, Yarrowia lipolytica, Hansenula polymorpha, Kluyveromyces lactis* or *Pichia pastoris.* Fungal expression hosts may also be, for example, filamentous fungal hosts including *Aspergillus niger, Chrysosporium lucknowense, Myceliophthora thermophila, Aspergillus (e.g., A. oryzae, A. niger, A. nidulans,* etc.) or *Trichoderma reesei.* Also suited are mammalian expression hosts such as mouse (*e.g.,* NS0), Chinese Hamster Ovary (CHO) or Baby Hamster Kidney (BHK) cell lines. Other eukaryotic hosts such as insect cells or viral expression systems (*e.g*., bacteriophages such as M13, T7 phage or Lambda, or viruses such as Baculovirus) are also suitable for producing the Ggr3A polypeptide.

Promoters and/or signal sequences associated with secreted proteins in a particular host of interest are candidates for use in the heterologous production and secretion of Ggr3A polypeptides in that host or in other hosts. As an example, in filamentous fungal systems, the promoters that drive the genes for cellobiohydrolase I (cbh1), glucoamylase A (glaA), TAKA-amylase (amyA), xylanase (exlA), the gpd-promoter cbh1, cbhll, endoglucanase genes eg1-eg5, Cel61B, Cel74A, gpd promoter, Pgk1, pki1, EF-1alpha, tef1, cDNA1 and hex1 are suitable and can be derived from a number of different organisms (*e.g., A. niger, T. reesei, A. oryzae, A. awamori, A. nidulans).*

In some embodiments, the *ggr3a* polynucleotide is recombinantly associated with a polynucleotide encoding a suitable homologous or heterologous signal sequence that leads to secretion of the recombinant Ggr3A polypeptide into the extracellular (or periplasmic) space, thereby allowing direct detection of enzyme activity in the cell supernatant (or periplasmic space or lysate). Suitable signal sequences for *Escherichia coli,* other Gram negative bacteria and other organisms known in the art include those that drive expression of the HlyA, DsbA, Pbp, PhoA, PelB, OmpA, OmpT or M13 phage Gill genes. For *Bacillus subtilis,* Gram-positive organisms and other organisms known in the art, suitable signal sequences further include those that drive expression of the AprE, NprB, Mpr, AmyA, AmyE, Blac, SacB, and for *S. cerevisiae* or other yeast, including the killer toxin, Bar1, Suc2, Mating factor alpha, InulA or Ggplp signal sequence. Signal sequences can be cleaved by a number of signal peptidases, thus removing them from the rest of the expressed protein. Fungal expression signal sequences may be one that is selected from, for example, SEQ ID NOs: 13-37, herein. Yeast expression signal sequences may be one that is selected from, for example, SEQ ID NOs:36-38. Signal sequences that might be suitable for use to express Ggr3A polypeptides of the invention in *Zymomonas mobilis* may include, for example, one selected from SEQ ID NOs:39-40 (encoded by SEQ ID NOs: 43-44, respectively; Linger J.G. et al., Appl. Environ. Microbiol. 76(19):6360-6369 (2010)).

In some embodiments, the recombinant Ggr3A polypeptide is expressed alone or as a fusion with other peptides, tags or proteins located at the N- or C-terminus (*e.g*., 6XHis, HA or FLAG tags). Suitable fusions include tags, peptides or proteins that facilitate affinity purification or detection (*e.g*., 6XHis, HA, chitin binding protein, thioredoxin or FLAG tags), as well as those that facilitate expression, secretion or processing of the target beta-glucosidases Suitable processing sites include enterokinase, STE13, Kex2 or other protease cleavage sites for cleavage in vivo or in vitro.

*Ggr3a* polynucleotides are introduced into expression host cells by a number of transformation methods including, but not limited to, electroporation, lipid-assisted transformation or transfection ("lipofection"), chemically mediated transfection (e.g., CaCl and/or CaP), lithium acetate-mediated transformation (*e.g*., of host-cell protoplasts), biolistic "gene gun" transformation, PEG-mediated transformation (*e.g.*, of host-cell protoplasts), protoplast fusion (*e.g*., using bacterial or eukaryotic protoplasts), liposome-mediated transformation, *Agrobacterium tumefaciens,* adenovirus or other viral or phage transformation or transduction.

### Cell culture media

Generally, the microorganism is cultivated in a cell culture medium suitable for production of the Ggr3A polypeptides described herein. The cultivation takes place in a suitable nutrient medium comprising carbon and nitrogen sources and inorganic salts, using procedures and variations known in the art. Suitable culture media, temperature ranges and other conditions for growth and cellulase production are known in the art. As a non-limiting example, a typical temperature range for the production of cellulases by *Trichoderma reesei* is 24°C to 37°C, for example, between 25°C and 30°C.

### Cell culture conditions

Materials and methods suitable for the maintenance and growth of fungal cultures are well known in the art. In some aspects, the cells are cultured in a culture medium under conditions permitting the expression of one or more beta-glucosidase polypeptides encoded by a nucleic acid inserted into the host cells. Standard cell culture conditions can be used to culture the cells. In some aspects, cells are grown and maintained at an appropriate temperature, gas mixture, and pH. In some aspects, cells are grown at in an appropriate cell medium.

### Activities of Ggr3A

Recombinant Ggr3A polypeptides disclosed herein have beta-glucosidase activity or a capacity to hydrolyze cellobiose and liberate D-glucose therefrom. Ggr3A polypeptides disclosed herein may have higher beta-glucosidase activity and improved or increased capacity to liberate D-glucose from cellobiose than the benchmark high fidelity beta-glucosidase Bgl1 of *Trichoderma reesei,* under the same saccharification conditions. In some embodiments, the Ggr3A polypeptides herein may have higher beta-glucosidase activity and/or improved or increased capacity to liberate D-glucose from cellobiose than another benchmark beta-glucosidase B-glu Y of *Aspergillus niger.*

Recombinant Ggr3A polypeptides disclosed herein, as compared to the *Trichoderma reesei* Bgl1, may have dramatically improved or increased, for example, at least about 20% higher, more preferably at least about 25% higher, preferably at least about 30% higher, more preferably at least 35% higher, preferably at least 40%, even more preferably at least about 45% higher, preferably at least about 50% higher, more preferably at least about 55% higher, and most preferably at least about 60% higher cellobiase activity, which measures the enzymes' capability to catalyze the hydrolysis of cellobiose, liberating D-glucose. In some embodiments, the recombinant Ggr3A polypeptide has about 1/2, about 1/3, about 1/4, about 1/5, or even about 1/6 of the capacity to catalyze the hydrolysis of cellobiose, liberating D-glucose.

As shown in Example 6, the recombinant Ggr3A polypeptide, as compared to the *Trichoderma reesei* Bgl1, produced more glucose while achieving a lower level of residual cellobiose (but similar amount of total soluble sugars) from a phosphoric acid swollen cellulose substrate, resulting in an overall lower protein dose on a background cellulase enzyme mixture of Spezyme® CP (Genencor) required for comparable extent of biomass hydrolysis/conversion of the same substrate to soluble sugars.

As shown in Example 7, the recombinant Ggr3A polypeptide, as compared to the *Trichoderma reesei* Bgl1, had a lower apparent *T*ₘ as measured in buffer.

As shown in Example 8, the recombinant Ggr3A polypeptide was compared to the *Trichoderma reesei* Bgl1 for cellobiose hydrolysis kinetics. The recombinant Ggr3A polypeptide was found to be apparently significantly less tolerant of stress of high temperature saccharification than *Trichoderma reesei* Bgl1.

As shown in Example 9, the recombinant Ggr3A polypeptide, as compared to the *Trichoderma reesei* Bgl1, also produced more glucose while achieving a lower level of residual cellobiose (but similar amount of total soluble sugars) from a dilute acid preatreated corn stover substrate, resulting in an overall lower protein dose on a background cellulase enzyme mixture of Spezyme® CP (Genencor) required for comparable extent of biomass hydrolysis/conversion of the same substrate to soluble sugars.

### Compositions Comprising a Recombinant Beta-Glucosidase Ggr3A Polypeptide

The present disclosure provides engineered enzyme compositions (e.g., cellulase compositions) or fermentation broths enriched with a recombinant Ggr3A polypeptide. In some aspects, the composition is a cellulase composition. The cellulase composition can be, *e.g.,* a filamentous fungal cellulase composition, such as a *Trichoderma* cellulase composition. In some aspects, the composition is a cell comprising one or more nucleic acids encoding one or more cellulase polypeptides. In some aspects, the composition is a fermentation broth comprising cellulase activity, wherein the broth is capable of converting greater than about 50% by weight of the cellulose present in a biomass sample into sugars. The term "fermentation broth" and "whole broth" as used herein refers to an enzyme preparation produced by fermentation of an engineered microorganism that undergoes no or minimal recovery and/or purification subsequent to fermentation. The fermentation broth can be a fermentation broth of a filamentous fungus, for example, a *Trichoderma, Humicola, Fusarium, Aspergillus, Neurospora, Penicillium, Cephalosporium, Achlya, Podospora, Endothia, Mucor, Cochliobolus, Pyricularia, Myceliophthora* or *Chrysosporium* fermentation broth. In particular, the fermentation broth can be, for example, one of *Trichoderma spp.* such as a *Trichoderma reesei,* or *Penicillium spp.,* such as a *Penicillium funiculosum.* The fermentation broth can also suitably be a cell-free fermentation broth. In one aspect, any of the cellulase, cell, or fermentation broth compositions described herein can further comprise one or more hemicellulases.

In some aspects, the whole broth composition is expressed in *T. reesei* or an engineered strain thereof In some aspects the whole broth is expressed in an integrated strain of *T. reesei* wherein a number of cellulases including a Ggr3A polypeptide has been integrated into the genome of the *T. reesei* host cell. In some aspects, one or more components of the polypeptides expressed in the integrated *T. reesei* strain have been deleted.

In some aspects, the whole broth composition is expressed in A. *niger* or an engineered strain thereof.

Alternatively, the recombinant Ggr3A polypeptides can be expressed intracellularly. Optionally, after intracellular expression of the enzyme variants, or secretion into the periplasmic space using signal sequences such as those mentioned above, a permeabilisation or lysis step can be used to release the recombinant Ggr3A polypeptide into the supernatant. The disruption of the membrane barrier is effected by the use of mechanical means such as ultrasonic waves, pressure treatment (French press), cavitation, or by the use of membrane-digesting enzymes such as lysozyme or enzyme mixtures. A variation of this embodiment includes the expression of a recombinant Ggr3A polypeptide in an ethanologen microbe intracellularly. For example, a cellobiose transporter can be introduced through genetic engineering into the same ethanologen microbe such that cellobiose resulting from the hydrolysis of a lignocellulosic biomass can be transported into the ethanologen organism, and can therein be hydrolyzed and turned into D-glucose, which can in turn be metabolized by the ethanologen.

In some aspects, the polynucleotides encoding the recombinant Ggr3A polypeptide are expressed using a suitable cell-free expression system. In cell-free systems, the polynucleotide of interest is typically transcribed with the assistance of a promoter, but ligation to form a circular expression vector is optional. In some embodiments, RNA is exogenously added or generated without transcription and translated in cell-free systems.

### Uses of Ggr3A Polypeptides to Hydrolyze a Lignocellulosic Biomass Substrate

In some aspects, provided herein are methods for converting lignocelluloses biomass to sugars, the method comprising contacting the biomass substrate with a composition disclosed herein comprising a Ggr3A polypeptide in an amount effective to convert the biomass substrate to fermentable sugars. In some aspects, the method further comprises pretreating the biomass with acid and/or base and/or mechanical or other physical means In some aspects the acid comprises phosphoric acid. In some aspects, the base comprises sodium hydroxide or ammonia. In some aspects, the mechanical means may include, for example, pulling, pressing, crushing, grinding, and other means of physically breaking down the lignocellulosic biomass into smaller physical forms. Other physical means may also include, for example, using steam or other pressurized fume or vapor to "loosen" the lignocellulosic biomass in order to increase accessibility by the enzymes to the cellulose and hemicellulose. In certain embodiments, the method of pretreatment may also involve enzymes that are capable of breaking down the lignin of the lignocellulosic biomass substrate, such that the accessibility of the enzymes of the biomass hydrolyzing enzyme composition to the cellulose and the hemicelluloses of the biomass is increased.

**Biomass:** The disclosure provides methods and processes for biomass saccharification, using the enzyme compositions of the disclosure, comprising a Ggr3A polypeptide. The term "biomass," as used herein, refers to any composition comprising cellulose and/or hemicellulose (optionally also lignin in lignocellulosic biomass materials). As used herein, biomass includes, without limitation, seeds, grains, tubers, plant waste (such as, for example, empty fruit bunches of the palm trees, or palm fibre wastes) or byproducts of food processing or industrial processing (*e.g.,* stalks), corn (including, *e.g.,* cobs, stover, and the like), grasses (including, *e.g.,* Indian grass, such as *Sorghastrum nutans;* or, switchgrass, *e.g., Panicum* species, such as *Panicum virgatum),* perennial canes (*e.g.,* giant reeds), wood (including, *e.g.,* wood chips, processing waste), paper, pulp, and recycled paper (including, e.g., newspaper, printer paper, and the like). Other biomass materials include, without limitation, potatoes, soybean (*e.g*., rapeseed), barley, rye, oats, wheat, beets, and sugar cane bagasse.

The disclosure therefore provides methods of saccharification comprising contacting a composition comprising a biomass material, for example, a material comprising xylan, hemicellulose, cellulose, and/or a fermentable sugar, with a Ggr3A polypeptide of the disclosure, or a Ggr3A polypeptide encoded by a nucleic acid or polynucleotide of the disclosure, or any one of the cellulase or non-naturally occurring hemicellulase compositions comprising a Ggr3A polypeptide, or products of manufacture of the disclosure.

The saccharified biomass (*e.g.*, cellulosic material processed by enzymes of the disclosure) can be made into a number of bio-based products, *via* processes such as, *e.g.,* microbial fermentation and/or chemical synthesis. As used herein, "microbial fermentation" refers to a process of growing and harvesting fermenting microorganisms under suitable conditions. The fermenting microorganism can be any microorganism suitable for use in a desired fermentation process for the production of bio-based products. Suitable fermenting microorganisms include, without limitation, filamentous fungi, yeast, and bacteria. The saccharified biomass can, for example, be made it into a fuel (*e.g.,* a biofuel such as a bioethanol, biobutanol, biomethanol, a biopropanol, a biodiesel, a jet fuel, or the like) *via* fermentation and/or chemical synthesis. The saccharified biomass can, for example, also be made into a commodity chemical (*e.g*., ascorbic acid, isoprene, 1,3-propanediol), lipids, amino acids, polypeptides, and enzymes, *via* fermentation and/or chemical synthesis.

**Pretreatment:** Prior to saccharification or enzymatic hydrolysis and/or fermentation of the fermentable sugars resulting from the saccharifiction, biomass (*e.g*., lignocellulosic material) is preferably subject to one or more pretreatment step(s) in order to render xylan, hemicellulose, cellulose and/or lignin material more accessible or susceptible to the enzymes in the enzymatic composition (for example, the enzymatic composition of the present invention comprising a Ggr3A polypeptide) and thus more amenable to hydrolysis by the enzyme(s) and/or the enzyme compositions.

In some aspects, a suitable pretreatment method may involve subjecting biomass material to a catalyst comprising a dilute solution of a strong acid and a metal salt in a reactor. The biomass material can, *e.g.,* be a raw material or a dried material. This pretreatment can lower the activation energy, or the temperature, of cellulose hydrolysis, ultimately allowing higher yields of fermentable sugars. See, *e.g.,* U.S. Patent Nos. 6,660,506; 6,423,145.

In some aspects, a suitable pretreatment method may involve subjecting the biomass material to a first hydrolysis step in an aqueous medium at a temperature and a pressure chosen to effectuate primarily depolymerization of hemicellulose without achieving significant depolymerization of cellulose into glucose. This step yields a slurry in which the liquid aqueous phase contains dissolved monosaccharides resulting from depolymerization of hemicellulose, and a solid phase containing cellulose and lignin. The slurry is then subject to a second hydrolysis step under conditions that allow a major portion of the cellulose to be depolymerized, yielding a liquid aqueous phase containing dissolved/soluble depolymerization products of cellulose. See, *e.g.,* U.S. Patent No. 5,536,325.

In further aspects, a suitable pretreatment method may involve processing a biomass material by one or more stages of dilute acid hydrolysis using about 0.4% to about 2% of a strong acid; followed by treating the unreacted solid lignocellulosic component of the acid hydrolyzed material with alkaline delignification. See, *e.g.,* U.S. Patent No. 6,409,841.

In yet further aspects, a suitable pretreatment method may involve pre-hydrolyzing biomass (*e.g*., lignocellulosic materials) in a pre-hydrolysis reactor; adding an acidic liquid to the solid lignocellulosic material to make a mixture; heating the mixture to reaction temperature; maintaining reaction temperature for a period of time sufficient to fractionate the lignocellulosic material into a solubilized portion containing at least about 20% of the lignin from the lignocellulosic material, and a solid fraction containing cellulose; separating the solubilized portion from the solid fraction, and removing the solubilized portion while at or near reaction temperature; and recovering the solubilized portion. The cellulose in the solid fraction is rendered more amenable to enzymatic digestion. See, *e.g.,* U.S. Patent No. 5,705,369. In a variation of this aspect, the pre-hydrolyzing can alternatively or further involves pre-hydrolysis using enzymes that are, for example, capable of breaking down the lignin of the lignocellulosic biomass material.

In yet further aspects, suitable pretreatments may involve the use of hydrogen peroxide H₂O₂. See Gould, 1984, Biotech, and Bioengr. 26:46-52.

In other aspects, pretreatment can also comprise contacting a biomass material with stoichiometric amounts of sodium hydroxide and ammonium hydroxide at a very low concentration. See Teixeira et al., 1999, Appl. Biochem.and Biotech. 77-79:19-34.

In some embodiments, pretreatment can comprise contacting a lignocellulose with a chemical (*e.g.,* a base, such as sodium carbonate or potassium hydroxide) at a pH of about 9 to about 14 at moderate temperature, pressure, and pH. *See,* Published International Application WO2004/081185. Ammonia is used, for example, in a preferred pretreatment method. Such a pretreatment method comprises subjecting a biomass material to low ammonia concentration under conditions of high solids. See, *e.g.,* U.S. Patent Publication No. 20070031918 and Published international Application WO 06110901.

### The Saccharification Process

In some aspects, provided herein is a saccharification process comprising treating biomass with an enzyme composition comprising a polypeptide, wherein the polypeptide has beta-glucosidase activity and wherein the process results in at least about 50 wt.% (e.g., at least about 55 wt.%, 60 wt.%, 65 wt.%, 70 wt.%, 75 wt.%, or 80 wt.%) conversion of biomass to fermentable sugars. In some aspects, the biomass comprises lignin. In some aspects the biomass comprises cellulose. In some aspects the biomass comprises hemicellulose. In some aspects, the biomass comprising cellulose further comprises one or more of xylan, galactan, or arabinan. In some aspects, the biomass may be, without limitation, seeds, grains, tubers, plant waste (e.g., empty fruit bunch from palm trees, or palm fibre waste) or byproducts of food processing or industrial processing (*e.g.,* stalks), corn (including, *e.g.,* cobs, stover, and the like), grasses (including, *e.g.,* Indian grass, such as *Sorghastrum nutans;* or, switchgrass, *e.g., Panicum* species, such as *Panicum virgatum),* perennial canes (*e.g.,* giant reeds), wood (including, *e.g.,* wood chips, processing waste), paper, pulp, and recycled paper (including, *e.g.*, newspaper, printer paper, and the like), potatoes, soybean (*e.g.*, rapeseed), barley, rye, oats, wheat, beets, and sugar cane bagasse. In some aspects, the material comprising biomass is subject to one or more pretreatment methods/steps prior to treatment with the polypeptide. In some aspects, the saccharification or enzymatic hydrolysis further comprises treating the biomass with an enzyme composition comprising a Ggr3A polypeptide of the invention. The enzyme composition may, for example, comprise one or more other cellulases, in addition to the Ggr3A polypeptide. Alternatively, the enzyme composition may comprise one or more other hemicellulases. In certain embodiments, the enzyme composition comprises a Ggr3A polypeptide of the invention, one or more other cellulases, one or more hemicellulases. In some embodiments, the enzyme composition is a whole broth composition.

In some aspects, provided is a saccharification process comprising treating a lignocellulosic biomass material with a composition comprising a polypeptide, wherein the polypeptide has at least about 75% (e.g., at least about 75%, 80%, 85%, 90%, 91%, 92%, 93%, 94%, 95%, 96%, 97%, 98%, 99%) sequence identity to SEQ ID NO:2, and wherein the process results in at least about 50% (e.g., at least about 55%, 60%, 65%, 70%, 75%, 80%, 85%, or 90%) by weight conversion of biomass to fermentable sugars. In some aspects, lignocellulosic biomass material has been subject to one or more pretreatment methods/steps as described herein.

Other aspects and embodiments of the present compositions and methods will be apparent from the foregoing description and following examples.

### EXAMPLES

The following examples are provided to demonstrate and illustrate certain preferred embodiments and aspects of the present disclosure and should not be construed as limiting.

### EXAMPLE 1

### Cloning & Expression of Gene Expression of Benchmark T. reesei Bgll and G. graminicola Ggr3A

### A. Construction of the T. reesei bgl1 expression vector

The N-terminal portion of the native *T. reesei* β-glucosidase gene *bgl1* was codon optimized (DNA 2.0, Menlo Park, CA). This synthesized portion comprised the first 447 bases of the coding region of this enzyme. This fragment was then amplified by PCR using primers SK943 and SK941 (below). The remaining region of the native *bgll* gene was PCR amplified from a genomic DNA sample extracted from *T. reesei* strain RL-P37 (Sheir-Neiss, G et al. Appl. Microbiol. Biotechnol. 1984, 20:46-53), using the primers SK940 and SK942 (below). These two PCR fragments of the *bgll* gene were fused together in a fusion PCR reaction, using primers SK943 and SK942:
Forward Primer SK943: (5'-CACCATGAGATATAGAACAGCTGCCGCT-3') (SEQ ID NO:5)
Reverse Primer SK941: (5'-CGACCGCCCTGCGGAGTCTTGCCCAGTGGTCCCGCGACAG-3') (SEQ ID NO:6)
Forward Primer (SK940): (5'-CTGTCGCGGGACCACTGGGCAAGACTCCGCAGGGCGGTCG-3') (SEQ ID NO:7)
Reverse Primer (SK942): (5'-CCTACGCTACCGACAGAGTG-3') (SEQ ID NO:8)

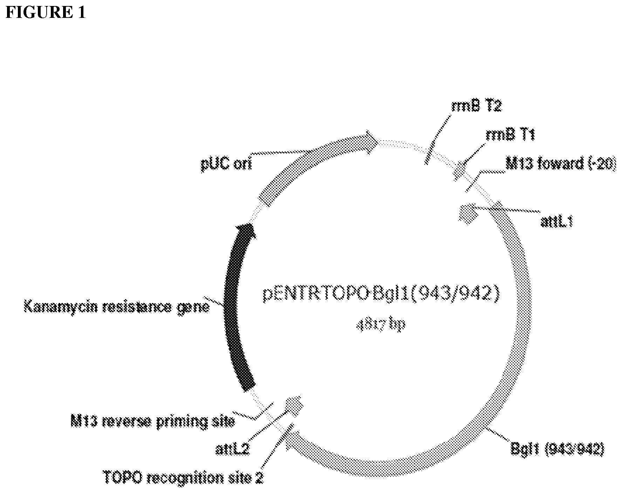

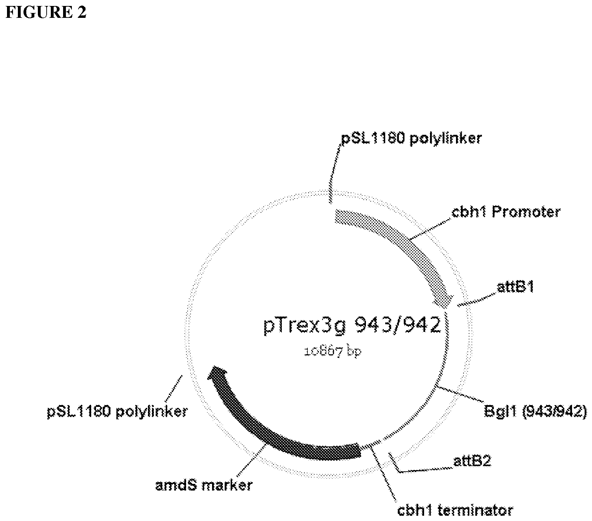

The resulting fusion PCR fragments were cloned into the Gateway ® Entry vector pENTR™/D-TOPO®, and transformed into *E. coli One Shot*® *TOP10* Chemically Competent cells (Invitrogen) resulting in the intermediate vector, pENTR TOPO-Bgl1(943/942) (Figure 1). The nucleotide sequence of the inserted DNA was determined. The pENTR-943/942 vector with the correct *bgll* sequence was recombined with pTrex3g using a LR clonase® reaction *(see,* protocols outlined by Invitrogen). The LR clonase reaction mixture was transformed into *E. coli One Shot*®*TOP10* Chemically Competent cells (Invitrogen), resulting in the expression vector, pTrex3g 943/942 (Figure 2). The vector also contained the *Aspergillus nidulans amdS* gene, encoding acetamidase, as a selectable marker for transformation of *T. reesei.* The expression cassette was PCR amplified with primers SK745 and SK771 to generate the product for transformation.
Forward Primer SK771: (5' - GTCTAGACTGGAAACGCAAC -3') (SEQ ID NO:9)
Reverse Primer SK745: (5' - GAGTTGTGAAGTCGGTAATCC -3') (SEQ ID NO:10)

### B. Construction of the ggr3α expression vector

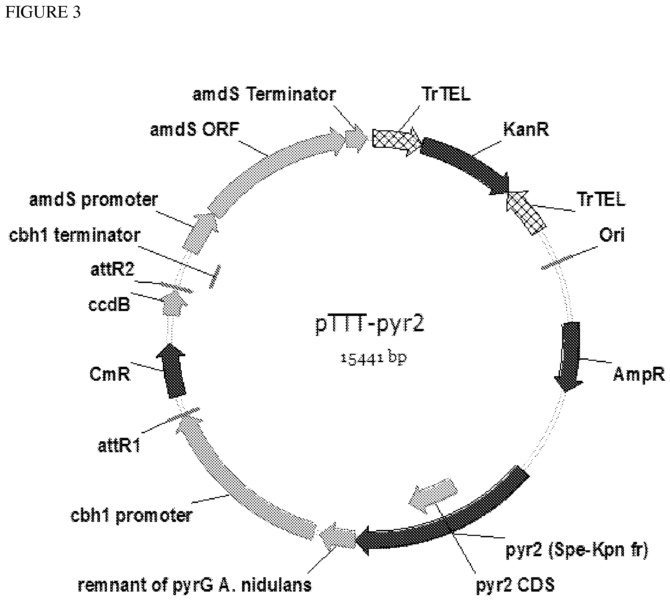

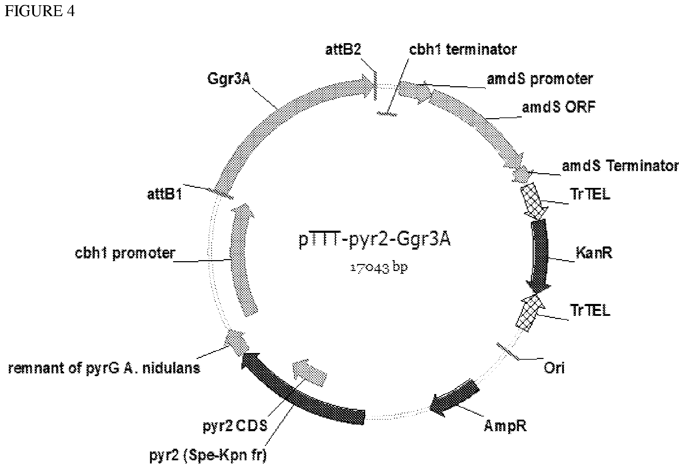

The open reading frame of the beta-glucosidase gene *ggr3a* was synthesized by Bionexus and cloned into the pTTT-pyr2 vector (Figure 3, SEQ ID NO:41) using Gateway Technology. The Ggr3A coding sequence was flanked by the CBHI promoter and CBHI terminator, with acetamidase (*amdS*) as the selection marker. The resulting vector, pTTT-pyr2-Ggr3A (Figure 4, SEQ ID NO:42) was used for transformation of *Trichoderma.*

A *T*. *reesei* strain deleted for 10 background activities (Δ(*cbh1, cbh2, egl1, egl2, egl3, egl4, egl5, egl6, manl, bgl1*)*,* based on WO 2010/141779, was transformed with the expression vector or a PCR product of the expression cassette.

Transformation was performed by the PEG-mediated protoplast method with slight modifications as described below. For protoplast preparation, spores were grown for 16-24 hours at 24°C in *Trichoderma* Minimal Medium MM, which contains 20 g/L glucose, 15 g/L KH₂PO₄, pH 4.5, 5 g/L (NH₄)₂SO₄, 0.6 g/L MgSO₄x7H₂O, 0.6 g/L CaCl₂ x 2H₂O, 1 mL of 1000 X *T*. *reesei* Trace elements solution (containing 5 g/L FeSO₄ x 7H₂O, 1.4 g/L ZnSO₄ x 7H₂O, 1.6 g/L MnSO₄ x H₂O, 3.7 g/L CoCl₂ x 6H₂O) with shaking at 150 rpm. Germinating spores were harvested by centrifugation and treated with 50 mg/mL of Glucanex G200 (Novozymes AG) solution to lyse the fungal cell walls. Further preparation of the protoplasts was performed in accordance with a method described by Penttilä et al. Gene 61(1987) 155-164. The transformation mixtures, which contained about 1 µg of DNA and 1-5x 10⁷ protoplasts in a total volume of 200 µL, were each treated with 2 mL of 25% PEG solution, diluted with 2 volumes of 1.2 M sorbitol/10 mM Tris, pH7.5, 10 mM CaCl₂, mixed with 3% selective top agarose MM containing 5 mM uridine and 20 mM acetamide. The resulting mixtures were poured onto 2% selective agarose plate containing uridine and acetamide. Plates were incubated further for 7 days at 28°C before single transformants were picked onto fresh MM plates containing uridine and acetamide. Spores from independent clones were used to inoculate a fermentation medium in shake flasks.

The fermentation media was 36 mL of defined broth containing glucose/sophorose and 2 g/L uridine, such as Glycine Minimal media (6.0 g/L glycine; 4.7 g/L (NH₄)₂SO₄; 5.0 g/L KH₂PO₄; 1.0 g/L MgSO₄•7H₂O; 33.0 g/L PIPPS; pH 5.5) with post sterile addition of ∼2% glucose/sophorose mixture as the carbon source, 10 ml/L of 100g/L of CaCl₂, 2.5 ml/L of *T. reesei* trace elements (400X) : 175g/L Citric acid anhydrous; 200g/L FeSO₄•7H₂O; 16g/L ZnSO₄•7H₂O; 3.2 g/L CuSO₄•5H₂O; 1.4 g/L MnSO₄•H₂O; 0.8 g/L H₃BO₃, in 250 mL Thomson Ultra Yield Flasks (Thomson Instrument Co., Oceanside, CA).

### C. Construction of a yeast shuttle vector pSC11 (Prophetic)

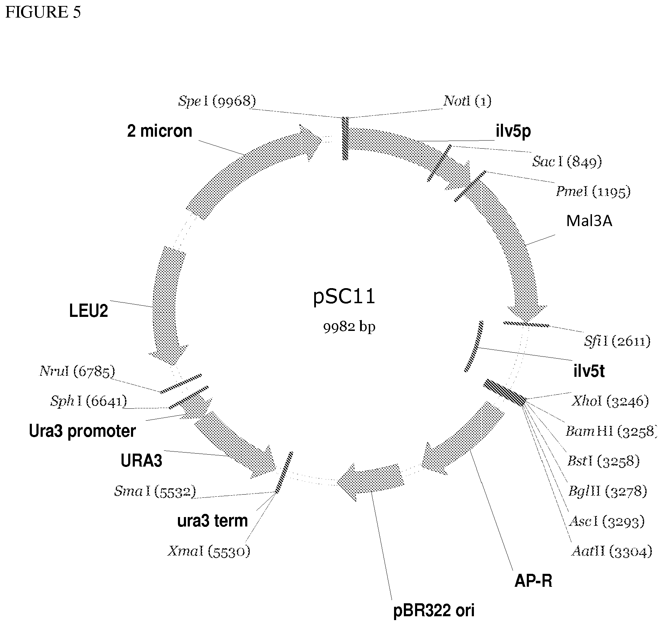

A yeast shuttle vector can be constructed in accordance with the vector map of Figure 5. This vector can be used to express a Ggr3A polypeptide in *Saccharomyces cerevisiae* intracellularly. A cellobiose transporter can be introduced into the *Saccharomyces cerevisiae* in the same shuttle vector or in a separate vector using known methods, such as, for example, those described by Ha et al., in PNAS, 108(2): 504-509 (2011).

Transformation of expression cassettes can be performed using the yeast EZ-Transformation kit. Transformants can be selected using YSC medium, which contains 20 g/L cellobiose. The successful introduction of the expression cassettes into yeast can be confirmed by colony PCR with specific primers.

Yeast strains can be cultivated in accordance with known methods and protocols. For example, they can be cultivated at 30°C in a YP medium (10 g/L yeast extract, 20 g/L Bacto peptone) with 20 g/L glucose. To select transformants using an amino acid auxotrophic marker, yeast synthetic complete (YSC) medium may be used, which contains 6.7 g/L yeast nitrogen base plus 20 g/L glucose, 20 g/L agar, and CSM-Leu-Trp-Ura to supply nucleotides and amino acids.

### D. Construction of a Zymomonas mobilis integration vector pZC11 (Prophetic)

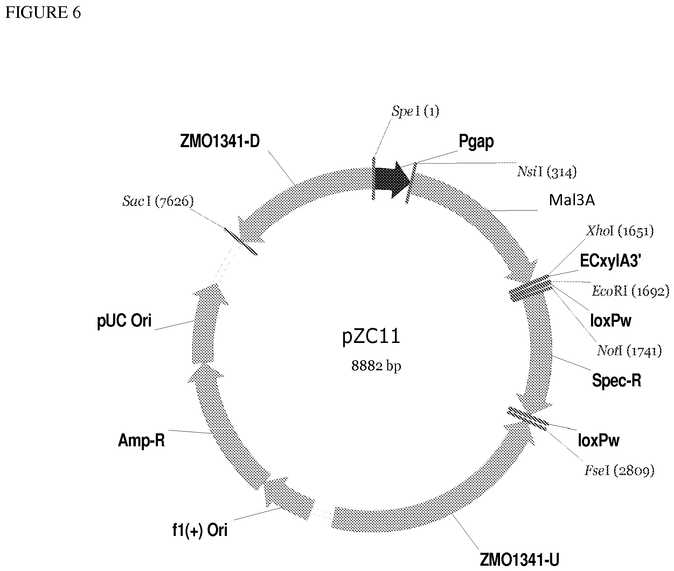

A *Zymomonas mobilis* integration vector pZC11 can be constructed in accordance with the vector map of Figure 6. This vector can be used to express a Ggr3A polypeptide in *Zymomonas mobilis* intracellularly. A cellobiose transporter can be introduced into the *Zymomonas mobilis* in the same integration vector or in a separate vector using known methods of introducing those transporters into a bacterial cell, such as, for example, those described by Sekar et al., Appl. Environ. Microbiol. (22 December 2011).

Successful introduction of the integration vector as well as the cellobiose transporter gene can be confirmed using various known approaches, for example by PCR using confirmatory primers specifically designed for this purpose.

*Zymomonas mobilis* strains can be cultivated and fermented according to known methods, such as, for example, those described in U.S. Patent No. 7,741,119.

### EXAMPLE 2

### Methods

### A. Protein concentration measurement by UPLC

An Agilent HPLC 1290 Infinity system was used for protein quantitation with a Waters ACQUITY UPLC C4BEH 300 Column (1.7 µm, 1 x 50 mm). A six minute program with an initial gradient from 5% to 33% acetonitrile (Sigma-Aldrich) in 0.5 min, followed by a gradient from 33% to 48% in 4.5 min, and then a step gradient to 90% acetonitrile was used. A protein standard curve based on the purified *T. reesei* Bgl1 was used to quantify the Ggr3A polypeptides.

### B. Purification of T. reesei Bgll & Ggr3A

*T. reesei* Bgl1 was over-expressed in, and purified from, the fermentation broth of a six-gene-deleted *Trichoderma reesei* host strain (*see, e.g.,* the description in Published International Patent Application Publication No. WO 2010/141779). A concentrated broth was loaded onto a G25 SEC column (GE Healthcase Bio-Sciences) and was buffer-exchanged against 50 mM sodium acetate, pH 5.0. The buffer exchanged *T. reesei* Bgll was then loaded onto a 25 mL column packed with amino benzyl-S-glucopyranosyl sepharose affinity matrix. After extensive washing with 250 mM sodium chloride in 50 mM sodium acetate, pH 5.0, the bound fraction was eluted with 100 mM glucose in 50 mM sodium acetate and 250 mM sodium chloride, pH 5.0. The eluted fractions that tested positive for chloro-nitro-phenyl glucoside (CNPG) activity were pooled and concentrated. A single band corresponding to the MW of the *T. reesei* Bgll on SDS-PAGE and confirmed by mass spectrometry verified the purity of the eluted Bgll. The final stock concentration was determined to be 2.2 mg/mL by absorbance at 280 nm.

A Ggr3A was expressed by *Trichoderma reesei* as described above, and purified from a fermentation broth. The broth of Ggr3A was diluted 10x with 50 mM Sodium Acetate pH 5.5, then mixed with Ammonium sulfate to achieve a concentration of 1 M ammonium sulfate. This sample was applied to an equilibrated (with 50mM sodium acetate pH 5.5, 1M Ammonium Sulfate buffer) Phenyl Sepharose Column (GE Healthcare PN 17-1086-01), and washed with this same buffer. The target protein was eluted using a gradient from 50 mM sodium acetate pH 5.5, 1M Ammonium Sulfate buffer, to 50mM sodium acetate, pH 5.5 buffer. The protein was then pooled and desalted into 10mM Tris pH 8.0 buffer, which was then applied to an equilibrated (with 10 mM Tris, pH 8.0 buffer) Resource Q column (GE Healthcare PN 17-0947-01). Once applied and washed with 10 mM Tris pH8.0 buffer, the target protein was then eluted with a gradient from 10 mM Tris pH 8.0 to 50 mM sodium acetate pH 5 and 1M sodium chloride buffer. The Ggr3A containing fractions were then pooled, concentrated and buffer exchanged into 50 mM MES pH 6.0, 100mM sodium chloride buffer for testing. Total protein concentration was determined using an absorbance measurement at 280 nanometers.

### C. ABTS assay for glucose determination

A series of glucose standard solutions was prepared in the same buffer as the enzyme samples. Glucose standards were added to an equal volume of glycine quench buffer prior to addition into the ABTS assay. An ABTS stock solution containing 2,2'-Azino-bis(3-ethylbenzothiazoline-6-sulfonic acid) di-ammonium salt (ABTS) at 2.88 mg/mL, horseradish peroxidise (HRP) at 0.11 U/mL and a glucose oxidase (OxyGO HP 5000L, Danisco US, Inc) at 1.05 U/mL was prepared in water. Ninety five (95) µL of the ABTS stock solution was pipetted into the wells of three separate Costar flat-bottom microtiter plates. Five (5) µL from the quenched cellobiose activity plate was pipetted into the reaction plate containing the ABTS solution. Plates were loaded in a spectrophotometer set for a 3 minute kinetic read at an absorbance of 405 nm with a 9-second read interval and a 60-second lag. A quick 5-second shaking step was added prior to the kinetic incubation for mixing and to eliminate any air bubbles in the wells. All ABTS reactions were analyzed using the included glucose standard curve on each plate to calculate glucose production.

### D. Preparation of Phosphoric Acid Swollen Cellulose (PASC)

Phosphoric acid swollen cellulose (PASC) was prepared from Avicel using an adapted protocol of Walseth, TAPPI 1971, 35:228 and Wood, Biochem. J. 1971, 121:353-362. In short, Avicel PH-101 was solubilized in concentrated phosphoric acid then precipitated using cold deionized water. The cellulose was collected and washed with more water to neutralize the pH. It was diluted to 1% solids in 50 mM sodium acetate pH5.

### E. Preparation of dilute acid pretreated corn stover substrate (whPCS)

Dilute acid pretreated corn stover was prepared by the National Renewable Energy Laboratory (NREL, Golden, CO) according to the method described in Schell et al., J Appl Biochem Biotechnol, 105:69-86, 2003.

The substrate was diluted by combining 20 g whPCS (32.7% solids) with 40 mL of 50 mM sodium acetate buffer, pH 5.0. The liquid was added small aliquots and stirred by hand to fully incorporate. Sixty (60) µL of 5% sodium azide was added as an anti-microbial. The substrate was covered and allowed to gently stir at room temperature overnight to equilibrate. The pH of the substrate was then adjusted from to pH 5 with 1 M sodium hydroxide solution. The final solids were 10.2%.

### EXAMPLE 3

### Cellobiose (or Cellotriose or Sophorose) Hydrolysis Assay

Cellobiose (or cellotriose or sophorose) hydrolysis activity was determined at 50°C based on the method of Ghose, T.K. Pure & Applied Chemistry, 1987, 59 (2), 257-268. 8.33 mM cellobiose (or cellotriose or sophorose) stock solution was prepared in assay buffer (50mM Nacitrate buffer, pH 5.3, + 0.005% Tween-80). The assay was performed in 96-well microtiter plate format. Cellobiose stock (90 µL) was pipetted into a Costar flat-bottom microtiter plate.

An enzyme dilution series was prepared and pipetted (10 µL) into triplicate reaction plates containing the cellobiose solution. The plates were sealed with aluminium seals (Nunc) and incubated for 30 minutes at 50°C, with shaking (1150 rpm, iEMS incubator). Reaction plates were quenched with 100 uL of 100 mM glycine buffer, at pH 10.0. Glucose concentrations were determined using the ABTS assay. Cellobiose units (derived as described in Ghose) are defined as 0.0926 divided by the amount of enzyme required to release 0.1 mg glucose under the assay conditions. Standard error for the cellobiase assay was determined to be 10%. The cellobiose activity assay was performed in triplicate at five enzyme dilution levels to generate a dose curve.

Establishing cutoffs of less than 15% and greater than 85% glucose yield in a double-reciprocal plot of the data resulted in a linear relationship with an R² value greater than 0.99 for all the enzymes tested. The performance index (PI) of a given sample was calculated by dividing the slope of the *T*. *reesei* Bgl1 (control) line by that of the sample.

The cellotriose and sophorose PIs were based on a single concentration of enzyme, which was obtained by using a common dilution which put all of the enzymes in the linear portion of the non-reciprocal cellobiose dose/response curve. These PIs were calculated as (specific activity for the variant)/(specific activity for *T. reesei* Bgl1) where (specific activity) is (molar % conversion of substrate to product)/(the UPLC-determined concentration of the variant).

The PI of Ggr3A relative to Bgl1 was > 1 on cellobiose, cellotriose and sophorose, indicating higher activity on all 3 substrates (Table 2).

**TABLE 2**

| | *T.reesei* Bgl1 | Ggr3A |
|---|---|---|
| Cellobiose PI, relative to Bgl1 | 1 | 5.5 |
| Cellotriose PI, relative to Bgl1 | 1 | 2.9 |
| Sophorose PI, relative to Bgl1 | 1 | 1.7 |

### EXAMPLE 4

### Cellobiose Thermal Activity Assay

The cellobiose assay (as in EXAMPLE 3, above) was performed at 40, 50, 60, and 69°C and glucose was determined using the ABTS assay.

Percent yield was determined by converting Vmax of the ABTS reaction to mM glucose, divided by the theoretical yield of glucose. Specific yield was determined by dividing the percent yield by the ppm of enzyme in the reaction. Cellobiose hydrolysis by Ggr3A was greater than Tr Bgl1 at all temperatures tested. The activity difference between the two beta-glucosidases was greatest at 50°C and least at 69°C (Table 3).

**TABLE 3**

| | *T. reesei* Bgl1 | Ggr3A |
|---|---|---|
| Cellobiose activity at 40°C, relative to Bgl1 | 1 | 3.5 |
| Cellobiose activity at 50°C, relative to Bgl1 | 1 | 3.6 |
| Cellobiose activity at 60°C, relative to Bgl1 | 1 | 2.8 |
| Cellobiose activity at 69°C, relative to Bgl1 | 1 | 1.8 |

### EXAMPLE 5

### Cellobiose Residual Activity (Thermal Stress)

To prepare the stress plate, an appropriate amount of enzyme was dispensed into a BioRad hard shell 96-well PCR plate and sealed with a silicone mat. The plate was placed in a thermocycler programmed for 65°C for 3 minutes, followed by a ramp down to 25°C.

The stressed enzyme samples were assayed in parallel with unstressed enzyme samples in the cellobiose activity assay (described in EXAMPLE 3 herein) to determine residual activity after thermal stress. The results in showed Ggr3A to have 42.8% residual activity, compared to 57.4% residual activity for *T. reesei* Bgll, under these conditions, and consistent with the lower apparent *T*ₘ. (as in EXAMPLE 7). This apparent lower tolerance of high temperature stress made it rather surprising that the hydrolysis of lignocellulosic biomass substrates as presented herein required a lower dose of Ggr3A.

### EXAMPLE 6

### PASC Activity Dose Response

Purified enzymes were loaded based on mg protein/g glucan in the substrate. Enzyme dilutions were made into 50 mM sodium acetate buffer, pH 5.0. One hundred and fifty (150) µL of cold PASC at 0.5% solids was added to 20 µL of enzyme solution per well in the assay plate. The final solids level in the assay was 0.44%.

The plates were covered with an aluminum plate sealer, mixed vigorously for 1 min. and placed in the Innova incubator/shaker at 50 °C, 200 rpm for 2 hours. The reaction was quenched with 100 µL 100 mM Glycine, pH 10 and transferred to a Millipore filter plate. The filter plate was sandwiched on top of an Agilent HPLC plate and centrifuged for 5 minutes to collect the centrate. Twenty (20) µL centrate was then added to 100 µL Millipore water in an Agilent HPLC plate. Soluble sugars were measured via HPLC.

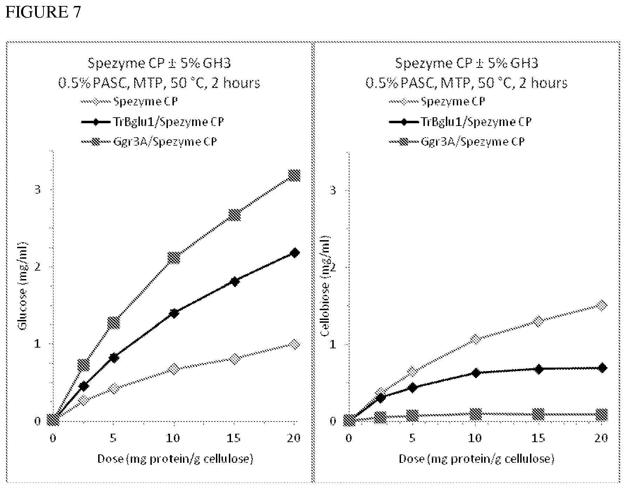

Percent glucan conversion was defined as (mg glucose + mg cellobiose) / mg cellulose in substrate. Results are shown in Figure 7.

### EXAMPLE 7

### Apparent Tm Measurement

Purified enzyme samples were diluted to 100 ppm in 50 mM sodium acetate buffer, pH 5.0. SYPRO Orange dye was diluted 1000-fold into the same buffer. Twenty five (25) µL of 100 ppm enzyme and 8 µL of diluted SYPRO Orange (Invitrogen Molecular Probes, #S6650) were added to the LightCycler 480 96-well plate. The plate was spun briefly to bring all the liquid to the bottom of the wells and then mixed on a bench top shaker. The plate was run in the LightCycler 480 Roche).

The program began with a 5 min. pre-incubation at 37 °C and a ramp rate of 4.4 °C/s. The melting curve target was 97 °C with a ramp rate of 0.2 °C/s. Detection format "Bodipy" was selected (fluorescence 498 - 580 nm), and the acquisition mode was continuous.

Results are indicated in Table 4 below.

**TABLE 4**

| GH3 | Purified | Apparent *T*ₘ |
|---|---|---|
| Tr Bgl1 | Y | 77.87 °C |
| Ggr3A | Y | 68.62 °C |

### EXAMPLE 8

### Kinetics of Cellobiose Hydrolysis

Purified *T. reesei* Bgl1 and Ggr3A were diluted to the same starting cellobiase activity and then serially diluted 8 times.

A 100 mM cellobiose solution was prepared and added to the top row of three non-binding assay plates (Costar #3641). The substrate was then serially diluted down the microtiter plate, resulting in 8 different substrate concentrations. Fifty (50) µL of enzyme solution was then added to 50 µL of substrate solution in the assay plate. The assay plates were covered and placed in the Innova44 incubator/shaker at 50 °C and 200 rpm for (1) 30 minutes (2) 60 minutes, or (3) 90 minutes. Each plate was quenched with 100 µL 100 mM Glycine, pH 10 buffer. Glucose concentrations were measured using a slightly modified ABTS assay (100 µL ABTS + 10 µL sample in a 96-well MTP, mixed, and placed in plate reader for 5 minutes. The kinetic readout was conducted at 420 nm).

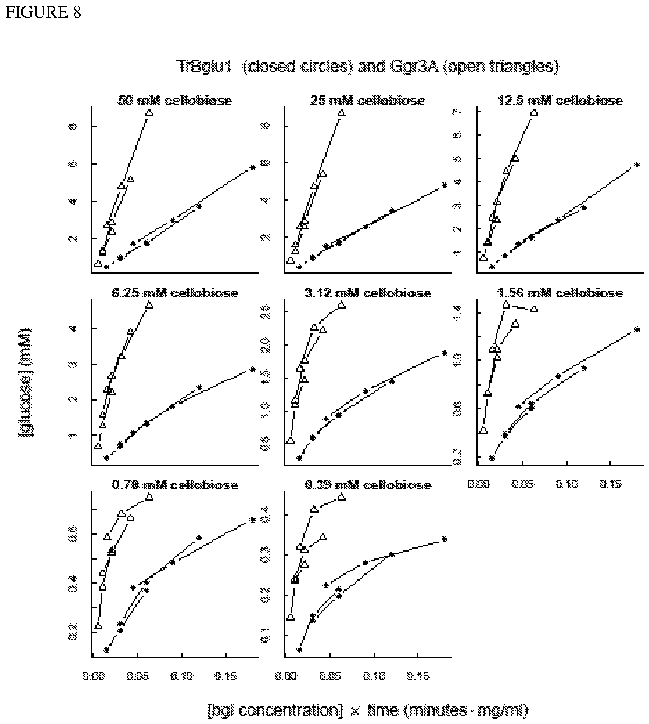

The results are plotted in Figure 8. The ordinate of these plots is the glucose released and is scaled to the maximum detected glucose. The abscissa is the product of the enzyme concentration and incubation time. The use of this abscissa allows for the direct comparison of all time points and dose values for both enzymes on the same plot. Separate plots are shown for each cellobiose concentration used in the reactions. Reactions containing beta-glucosidase T. *reesei* Bgl1 are indicated with closed circles, and reactions containing Ggr3A are

### EXAMPLE 9

### Saccharification of dilute acid pretreated corn stover (whPCS)

Purified beta-glucosidases *T. reesei* Bgl1 and Ggr3A were blended at 1% of the total protein with Spezyme® CP cellulase mixture (Genencor).

The enzyme mixtures were dosed from 0 - 20 mg protein/g glucan and used to saccharify whPCS in 50 mM acetate buffer, pH 5.0 and 7% solids in 96-well MTP (Nunc, #269787) at 50 °C for 2 days. Each enzyme blend had 4 assay replicates. The substrate was added to the assay plate using a repeater pipette with a 1 mL Eppendorf tip. Thirty (30) µL of enzyme solution was added to 70 mg substrate at 10% solids per well, for a final total solids of 7%. The plates were covered with aluminum plate seals (E&K Scientific #EK-46909), mixed for 1 minute, and placed in the Innova 44 incubator/shaker (New Brunswick Scientific) at 50 °C, 200 rpm for 2 days.

The reaction was quenched with 100 µL 100 mM glycine, pH 10 and transferred to a filter plate (Millipore, #MAHVN4550) using a multichannel pipette. The filter plate was sandwiched on top of an HPLC plate (Agilent, #5042-1385) and spun in the centrifuge for 5 min. to filter. Ten (10) µL supernatant was added to 100 µL Millipore water in an Agilent HPLC plate. Soluble sugars were measured via HPLC.

Percent glucan conversion is defined as (mg glucose + mg cellobiose) / mg cellulose in substrate.

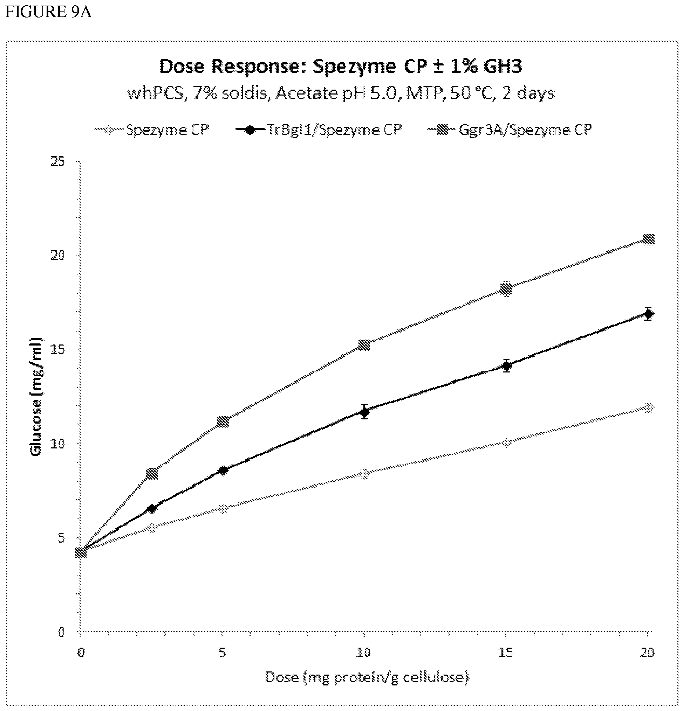

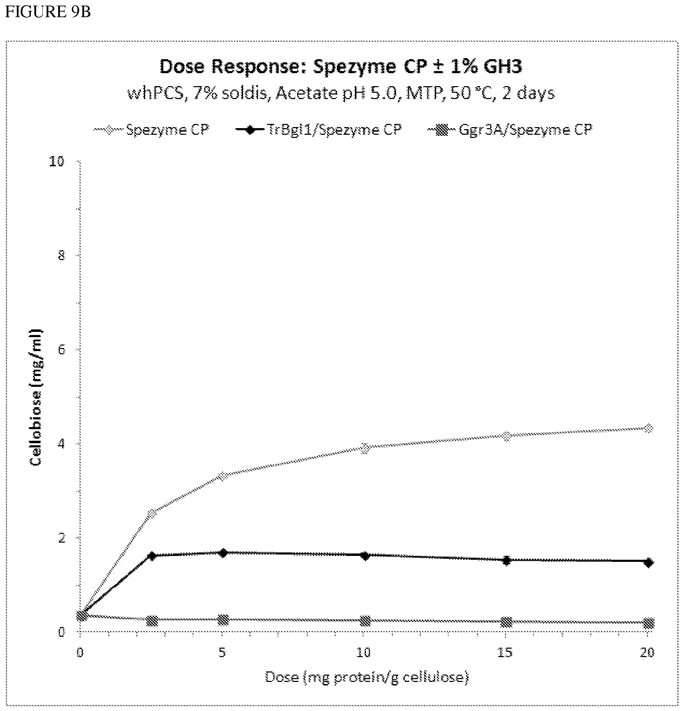

Results are depicted in Figures 9A-9B.

Ggr3A produced the more glucose and had less residual cellobiose than *T. reesei* Bgl1, resulting in a 1.4x reduction of overall protein dose required to hydrolyze the same substrate to approximately the same extent.

## Claims

1. A method for hydrolyzing a lignocellulosic biomass substrate, comprising:
contacting the lignocellulosic biomass substrate with a recombinant polypeptide to yield glucose and other sugars,
wherein the polypeptide comprises an amino acid sequence that is at least 90% identical to the amino acid sequence of SEQ ID NO 2: or SEQ ID NO:3 and wherein the polypeptide has improved beta-glucosidase activity as compared to *Trichoderma reesei* Bgll when the recombinant polypeptide and the *Trichoderma reesei* Bgll are used to hydrolyze lignocellulosic biomass substrates under the same saccharification conditions.

2. The method of claim 1, wherein the improved betaglucosidase activity is an increased cellobiose activity or an increased yield of glucose from a lignocellulosic biomass under the same saccharification conditions.

3. The method of claim 2, wherein the improved betaglucosidase activity is an increased cellobiose activity that is measured using cellobiose as a substrate at a temperature of about 30°C to about 65°C.

4. The method of any one of claims 1-3, wherein the recombinant protein forms part of a composition, the composition further comprising one or more other beta-glucosidases, one or more cellobiohydrolases, and one or more endoglucanases.

5. The method of any one of claims 1-4, wherein the recombinant protein forms part of a composition, the composition further comprising one or more hemicellulases selected from one or more xylanases, one or more beta-xylosidases, and one or more L-arabinofuranosidases.

6. A saccharification process comprising treating a lignocellulosic biomass material with an enzyme compositions comprising a polypeptide,
wherein the polypeptide comprises an amino acid sequence that is at least 90% identical to the amino acid sequence of SEQ ID NO 2: or SEQ ID NO:3, and
wherein the process results in at least about 50 wt% conversion to fermentable sugars, and
wherein the polypeptide has improved beta-glucosidase activity as compared to *Trichoderma reesei* Bgll when the recombinant polypeptide and the *Trichoderma reesei* Bgll are used to hydrolyze lignocellulosic biomass substrates under the same saccharification conditions..

7. The method or saccharification process according to any one of the preceding claims, further comprising a fermentation step during which the monomeric or fermentable sugars that resulted from the saccharification or hydrolysis step are metabolized.

8. The method according to claim 7 wherein ethanol is produced during the fermentation step.

## Patentansprüche

1. Verfahren zum Hydrolysieren eines Lignocellulose-Biomassesubstrats, umfassend:
Inkontaktbringen des Lignocellulose-Biomassesubstrats mit einem rekombinanten Polypeptid, um Glucose und andere Zucker zu ergeben,
wobei das Polypeptid eine Aminosäuresequenz umfasst, die zu mindestens 90 Prozent identisch ist mit der Aminosäuresequenz von SEQ ID NO: 2: oder SEQ ID NO: 3 und wobei das Polypeptid im Vergleich zu *Trichoderma reesei* Bgll eine verbesserte Beta-Glucosidase-Aktivität aufweist, wenn das rekombinante Polypeptid und das *Trichoderma reesei* Bgll verwendet werden, um Lignocellulose-Biomassesubstrate unter den gleichen Bedingungen der Verzuckerung zu hydrolysieren.

2. Verfahren nach Anspruch 1, wobei die verbesserte Beta-Glucosidase-Aktivität eine erhöhte Cellobiose-Aktivität oder eine erhöhte Ausbeute an Glucose aus einer Lignocellulose-Biomasse unter den gleichen Bedingungen der Verzuckerung ist.

3. Verfahren nach Anspruch 2, wobei die verbesserte Beta-Glucosidase-Aktivität eine erhöhte Cellobiose-Aktivität ist, die unter Verwendung von Cellobiose als ein Substrat bei einer Temperatur von etwa 30°C bis etwa 65°C gemessen wird.

4. Verfahren nach einem der Ansprüche 1 bis 3, wobei das rekombinante Protein Teil einer Zusammensetzung bildet, wobei die Zusammensetzung ferner eine oder mehrere andere Beta-Glucosidasen, eine oder mehrere Cellobiohydrolase und eine oder mehrere Endoglucanase umfasst.

5. Verfahren nach einem der Ansprüche 1 bis 4, wobei das rekombinante Protein Teil einer Zusammensetzung bildet, wobei die Zusammensetzung ferner eine oder mehrere Hemicollulasen umfasst, die ausgewählt sind aus einer oder mehreren Xylanasen, einer oder mehreren Beta-Xylosidasen und einer oder mehreren L-Arabinofuranosidasen.

6. Verfahren zur Verzuckerung, umfassend Behandeln einer Lignocellulose enthaltenden Biomasse mit einer Enzymzusammensetzung, die ein Polypeptid umfasst,
wobei das Polypeptid eine Aminosäuresequenz umfasst, die mindestens zu 90 Prozent identisch ist mit der Aminosäuresequenz von SEQ ID NO: 2: oder SEQ ID NO: 3, und
wobei das Verfahren zu einer Umwandlung von mindestens 50 Gew.-% zu fermentierbaren Zuckern resultiert, und
wobei das Polypeptid im Vergleich zu *Trichoderma reesei* Bgl1 eine verbesserte Beta-Glucosidase-Aktivität aufweist, wenn das rekombinante Polypeptid und das *Trichoderma reesei* Bgll verwendet werden, um Lignocellulose-Biomassesubstrate unter den gleichen Bedingungen der Verzuckerung zu verwenden.

7. Verfahren oder Verzuckerungsprozess nach einem der vorhergehenden Ansprüche, ferner umfassend einen Fermentationsschritt, während dessen die monomeren oder fermentierbaren Zucker metabolisiert werden, die aus dem Schritt der Verzuckerung oder Hydrolyse resultieren.

8. Verfahren nach Anspruch 7, wobei während des Fermentationsschrittes Ethanol erzeugt wird.

## Revendications

1. Méthode pour hydrolyser un substrat de biomasse lignocellulosique, comprenant:
la mise en contact du substrat de biomasse lignocellulosique avec un polypeptide recombinant pour donner du glucose et d'autres sucres,
dans laquelle le polypeptide comprend une séquence d'acides aminés qui est au moins 90% identique à la séquence d'acides aminés de SEQ ID NO: 2 ou SEQ ID NO: 3 et dans laquelle le polypeptide a une activité de bêta-glucosidase améliorée par rapport à Bgl1 de *Trichoderma reesei,* lorsque le polypeptide recombinant et le Bgl1 de *Trichoderma reesei* sont utilisés pour hydrolyser des substrats de biomasses lignocellulosiques dans les mêmes conditions de saccharification.

2. Méthode selon la revendication 1, dans laquelle l'activité de bêta-glucosidase améliorée est une activité de cellobiose accrue ou un rendement accru en glucose à partir d'une biomasse lignocellulosique dans les mêmes conditions de saccharification.

3. Méthode selon la revendication 2, dans laquelle l'activité de bêta-glucosidase améliorée est une activité de cellobiose accrue qui est mesurée en utilisant de la cellobiose en tant que substrat à une température d'environ 30°C à environ 65°C.

4. Méthode selon l'une quelconque des revendications 1 à 3, dans laquelle la protéine recombinante fait partie d'une composition, la composition comprenant en outre une ou plusieurs autres bêta-glucosidases, une ou plusieurs cellobiohydrolases et une ou plusieurs endoglucanases.

5. Méthode selon l'une quelconque des revendications 1 à 4, dans laquelle la protéine recombinante fait partie d'une composition, la composition comprenant en outre une ou plusieurs hémicellulases choisies parmi une ou plusieurs xylanases, une ou plusieurs bêta-xylosidases et une ou plusieurs L-arabinofuranosidases.

6. Méthode de saccharification comprenant le traitement d'une matière de biomasse cellulosique avec une composition d'enzyme comprenant un polypeptide,
dans laquelle le polypeptide comprend une séquence d'acides aminés qui est au moins 90% identique à la séquence d'acides aminés de SEQ ID NO: 2 ou SEQ ID NO: 3, et
laquelle méthode conduit à au moins environ 50% en poids de conversion en sucres fermentables, et
dans laquelle le polypeptide a une activité de bêta-glucosidase améliorée par rapport à Bgl1 de *Trichoderma reesei,* lorsque le polypeptide recombinant et le Bgl1 de *Trichoderma reesei* sont utilisés pour hydrolyser des substrats de biomasses lignocellulosiques dans les mêmes conditions de saccharification.

7. Méthode ou procédé de saccharification selon l'une quelconque des revendications précédentes, comprenant en outre une étape de fermentation pendant laquelle les sucres monomères ou fermentables qui résultent de l'étape de saccharification ou d'hydrolyse sont métabolisés.

8. Méthode selon la revendication 7, dans laquelle de l'éthanol est produit pendant l'étape de fermentation.
